# EUROPEAN PATENT APPLICATION

(11) **EP 1 956 009 A1**
(43) Date of publication of application: **13.08.2008**
(21) Application number: 06833714.6
(22) Date of filing: 30.11.2006
(51) Int. Cl.: C07D 239/94, A61P 11/06, A61P 17/00, A61P 29/00, A61P 37/02, A61P 37/06, A61P 43/00, C07D 487/04, C07D 495/04, A61K 31/517, A61K 31/519, A61K 31/5377, A61K 31/55

(54) **AROMATIC COMPOUND**

(30) Priority: 02.12.2005 JP 2005348598; 14.12.2005 US 750041 P
(71) Applicant: Mitsubishi Tanabe Pharma Corporation, Chuo-ku Osaka-shi Osaka 541-8505 (JP)
(72) Inventor: FURUKUBO, Shigeru, Osaka-shi, Osaka; 541-8505 (JP); MIYAZAKI, Hiroshi, Osaka-shi, Osaka; 541-8505 (JP)
(74) Representative: Srinivasan, Ravi Chandran
(86) International application number: PCT/JP2006/323911
(87) International publication number: WO 2007/063935

(57) **Abstract**

An aromatic compound represented by the following formula or a pharmaceutically acceptable salt thereof: 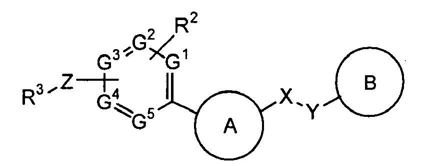 , wherein ring A is a heterocyclic ring, ring B is a carbocyclic ring, a heterocyclic ring etc., G¹, G², G³, G⁴ and G⁵ are CH or N, X is -NH-, -O-, -CH₂-, etc., Y is - CH₂-,-CO-,-SO₂- etc., Z is a single bond, -CO-, -SO₂-, -NH-, -O-, -S-, -CONH-,-SO₂NH-, etc., R² is hydrogen, alkyl, alkoxy, halogen, etc., and R³ is carbocyclic group, heterocyclic group, alkyl, etc.,
is useful as a controlling agent of the function of CCR4 useful for the treatment or therapy for bronchial asthma, atopic dermatitis, etc.

## Description

### TECHNICAL FIELD

The present invention relates to the compounds having an activity for controlling the function of CCR4, or TARC and/or MDC useful for the prophylaxis or treatment for allergic diseases such as bronchial asthma or atopic dermatitis, inflammatory diseases and autoimmune diseases.

### BACKGROUND ART

Allergic diseases such as bronchial asthma and atopic dermatitis are chronic inflammatory diseases associated with infiltration or activation of inflammatory cells (non patent documents 1 and 2). Bronchial asthma is a disease associated with reversible airway obstruction with airway inflammation and increased airway hypersensitivity. As a symptom thereof, stridor, shortness of breath, cough, etc. are observed. Chronic inflammation such as infiltration of eosinophils, lymphocytes and mast cells to airway, edema under mucosa, deposit of eosinopil-derived tissue damaged granular protein, or damage of airway epithelium are histologically observed. Atopic dermatitis is cutaneous chronic inflammatory disease with strong pruritus consisting of eczema which repeats exacerbation and remission as a main symptom. It is said that the pathema participates in both impairment of the epidermal barrier function consisting mainly of dermal dryness and the abnormal production of cytokines by immune cells. Therefore, to control such chronic inflammation is considered as one of approaches in the therapy of allergic diseases.

Recently, it has been revealed that helper T (Th) cells and cytokines produced by Th cells play very important roles in the process of pathogenesis of allergic inflammation (non patent documents 1 and 3). Th cells are classified to two sub-classes according to cytokine producing pattern, namely to Th 1 cells producing interferon γ (IFN-γ) or interleukin 2 (IL-2), and to Th 2 cells producing interleukin 4 (IL-4) or interleukin 5 (IL-5) (non patent document 4). IFN-γ and IL-2 control cellular immunity such as defense to infection and so on by activating macrophages or natural killer (NK) cells. On the other hand, since IL-4 and IL-5 participate in production of immunoglobulin(Ig) E and activation of eosinophils, respectively, Th 2 cells are considered to play large roles in the development of allergic inflammation (non patent documents 1, 5, 6 and 7).

Chemokines are classified to an endogenic leucocyte chemotactic factor and play an important role to tissue accumulation of leukocytes. The majority of chemokines is produced at inflammatory regions by the inflammatory stimulation, etc., and act on leukocytes to induce the chemotactic response. Up to now more than 40 chemokines have been identified, and they are classified to sub-classes, namely CXC, CC, C and CX3C according to structural features thereof. On the other hand, chemokine receptors are seven-transmembrane receptors which are conjugated with G protein, and consist of CXC chemokine receptor, CC chemokine receptor, CX3C chemokine receptor and C chemokine receptor. It is known that the majority of chemokine receptors is combined with plural chemokines, and the majority of chemokines are combined with plural chemokine receptors.

The gene coding for CC chemokine receptor 4 (CCR4) was cloned from human basophil-like cell line KU-812 in 1995 (non patent document 8). Thereafter, TARC (thymus and activation-regulated chemokine)/CCL17 as a CC chemokine which specifically migrates T cells and then MDC (macrophage-derived chemokine)/CCL22 as CC chemokine which shows chemotactic activity to monocytes, dendritic cells and NK cells were cloned, respectively (non patent documents 9 and 10), and it was revealed that these chemokines are ligands of CCR4 (non patent documents 11 and 12). CCR4 is much expressed in thymus and peripheral blood lymphocytes (non patent document 8) and it is comparatively localized and expressed in Th cells in lymphocytes (non patent document 11). CCR4 is selectively expressed in Th 2 cells, and as it is revealed that the migration of Th 2 cells is induced by the stimulation of TARC/CCL17 or MDC/CCL22 (non patent documents 11∼15), the role of CCR4 in the process of pathogenesis of allergic diseases has been paid attention.

In regard to the relation of allergic diseases and CCR4, and the relation of its ligands, namely TARC/CCL17 and MDC/CCL22, it is reported that (1) T cells expressed mRNA of CCR4 are detected at bronchial mucosa of a patient suffering from chronic bronchial asthma and the number of CCR4 expressed T cells increases after antigen exposure (non patent document 16), (2) the expression of mRNA of CCR4 is promoted in peripheral blood T cells of a patient suffering from atopic dermatitis, and the expression level of CCR4 relates to the number of the blood eosinophils, the level of serum IgE and the severity of dermatitis (non patent documents 17 and 18), (3) the serum concentration of TARC/CCL17 and MDC/CCL22 in patients suffering from atopic dermatitis is higher than that of in healthy persons (non patent documents 19 and 20), (4) in experimental asthma model, by treating with anti TARC antibody or anti MDC antibody, increased airway reactivity and infiltration of inflammatory cell to airway or pulmonary interstitium are inhibited (non patent documents 21 and 22) and so on.

Furthermore, as evidences showing the relation of CCR4 and/or its ligands with allergic diseases, inflammatory diseases and autoimmune diseases, there are following reports:
Dermatitis (atopic dermatitis, contact dermatitis): non patent documents 23-25
Asthma: non patent documents 16 and 26
Rhinitis: non patent document 27
Conjunctivitis: non patent document 28
Psoriasis: non patent document 29
Rheumatoid arthritis: non patent document 30
Systemic lupus erythematosus: non patent documents 31-33
Insulin dependant diabetes mellitus (IDDM): non patent document 34
Rejection on organ transplantation: non patent document 35
Inflammatory bowel disease (ulcerative colitis, Crohn's disease): non patent document 36
Glomerulonephritis: non patent document 37
Sepsis: non patent document 38
Pain: non patent document 39
Adult T cell leukemia (ATL): non patent document 40
Fibroid lung: non patent documents 41 and 42
Eosinophilic pneumonia: non patent document 43
Pneumoeosinophil granuloma: non patent document 44
Dermal T cell lymphoma: non patent documents 20 and 45
Ankylosing spondylitis: non patent document 46
Coronary disease: non patent document 47
Pemphigoid: non patent document 48
Hodgkin's disease : non patent document 49

These reports do not only suggest that abnormal expression of CCR4 and its ligands, namely TARC/CCL17 and MDC/CCL22 great participates in pathogenesis of many kinds of pathological condition such as allergic diseases, but a possibility to treat or improve these pathological condition by controlling the function of CCR4 and its ligands are also suggested.

Now β2 stimulants, xanthine, steroids and antiallergic agents (especially leukotriene antagonist) are used in clinical field as a therapeutic agent for bronchial asthma. Among them, inhaled steroids are positioned as the first-line drug and it is widely used for therapy of asthma. However, when the steroids are administered for a long term, the side effects are anxious and therefore, it can not maintain drug compliance.

In the therapy of atopic dermatitis, tacrolimus having immunosuppressive activity is used as an external preparation in order to suppress inflammatory as well as the steroids. External steroids are anxious for side effects such as hairiness or atrophia cutis in skin diseases. On the other hand, external tacrolimus does not show such side effects as the steroids, but the relation of tacrolimus with occurrence of feeling of dermal irritation and pathogenesis of carcinoma cutaneum are indicated.

Therefore, there is desired therapeutic and prophylactic agents for allergic diseases, inflammatory diseases and autoimmune diseases, which have the same strong therapeutic activity as steroids based on new mechanism with few side effects. Furthermore, since compounds having CCR4 antagonistic activity or CCR4 function-controlling activity can selectively control the infiltration and the activation of Th 2 cells to inflammatory regions, it is expected that these compounds will become an orally-available drug with few side effects, unlike steroids or immunosuppressant.

As the compounds having CCR4 antagonism or CCR4 function-controlling activity, there are known following compounds: a 5-cyanopyrimidine derivative (patent document 1), a bicyclic pyrimidine derivative (patent document 2), a 5-arylpyrimidine derivative (patent document 3), a bicyclic compound (patent document 4), a tricyclic compound (patent documents 5 and 6), a fused bicyclic pyrimidine derivative (patent document 7), a substituted pyrimidine derivative (patent document 8), a sulfonamide compound (patent documents 9 to 15) and so on.
[Patent document 1] WO03/082855
[Patent document 2] WO03/104230
[Patent document 3] WO2004/074260
[Patent document 4] WO2004/020584
[Patent document 5] WO2004/007472
[Patent document 6] WO2005/023771
[Patent document 7] WO2005/082865
[Patent document 8] WO2005/085212
[Patent document 9] W02005/021513
[Patent document 10] WO2004/108692
[Patent document 11] WO2004/108717
[Patent document 12] WO2004/108690
[Patent document 13] WO03/059893
[Patent document 14] WO03/051870
[Patent document 15] WO02/30358
[Non Patent document 1] Immunol. Today, 13, 501 (1992)
[Non Patent document 2] Allergy Clin. Immunol., 94, 1310 (1994)
[Non Patent document 3] Am. Rev. Respir. Dis., 147, 540 (1993)
[Non Patent document 4] J. Immunol., 1986, 136, 2348-57
[Non Patent document 5] Immunol. Today, 12, 256 (1991)
[Non Patent document 6] N. Eng. J. Med., 326, 298 (1992)
[Non Patent document 7] Trends. Pharmacol. Sci., 15, 324 (1994)
[Non Patent document 8] J. Biol. Chem., 270, 19495 (1995)
[Non Patent document 9] J. Biol. Chem., 271, 21514 (1996)
[Non Patent document 10] J. Exp. Med., 185, 1595 (1997)
[Non Patent document 11] J. Biol. Chem., 272, 15036 (1997)
[Non Patent document 12] J. Biol. Chem., 273, 1764 (1998)
[Non Patent document 13] J. Exp. Med., 187, 129 (1998)
[Non Patent document 14] J. Exp. Med., 187, 875 (1998)
[Non Patent document 15] Int. Immunol., 11, 81 (1999)
[Non Patent document 16] J. Clin. Invest., 107, 1357 (2001)
[Non Patent document 17] J. Allergy Clin. Immunol., 107, 353 (2001)
[Non Patent document 18] J. Invest. Dermatol., 117, 188 (2001)
[Non Patent document 19] J. Allergy Clin. Immunol., 107, 535 (2001)
[Non Patent document 20] Eur. J. Immunol., 30, 204 (2000)
[Non Patent document 21] J. Immunol., 163, 403 (1999)
[Non Patent document 22] J. Immunol., 166, 2055 (2001)
[Non Patent document 23] The Journal of Clinical Investigation, 107, 535, 2001
[Non Patent document 24] Journal of Investigative Dermatology, 115, 640, 2000
[Non Patent document 25] Journal of Investigative Dermatology, 186, 1052, 2002
[Non Patent document 26] Allergy, 57, 2, 173, 2002
[Non Patent document 27] European Journal of Immunology, 32, 7, 1933, 2002
[Non Patent document 28] Br J Ophthalmol, 86, 10, 1175, 2002
[Non Patent document 29] Laboratory Investigation, 81, 335, 2001
[Non Patent document 30] Arthritis & Rheumatism, 44, 2750, 2001
[Non Patent document 31] Journal of Investigative Dermatology, 124, 1241, 2005
[Non Patent document 32] Clinical Experimental Immunology, 138, 342, 2004
[Non Patent document 33] Arthritis & Rheumatism, 46, 735, 2002
[Non Patent document 34] The Journal of Clinical Investigation, 110, 1675, 2002
[Non Patent document 35] European Journal of Immunology, 35, 128, 2005
[Non Patent document 36] Clinical & Experimental Immunology, 132, 332, 2003
[Non Patent document 37] American Journal of Pathology, 162, 1061, 2003
[Non Patent document 38] Journal of Experimental Medicine, 191, 1755, 2000
[Non Patent document 39] The Journal of Neuroscience, 21, 5027, 2001
[Non Patent document 40] Cancer Res. 2005 Mar 15; 11(6): 2427-35
[Non Patent document 41] American Journal of Respiratory and Critical Care Medicine, Vol. 173, pp. 310-317 (2006)
[Non Patent document 42] The Journal of Immunology, 173, 4692, 2004
[Non Patent document 43] Clinical Immunology, 116, 83, 2005
[Non Patent document 44] American Journal of Pathology, 165, 1211, 2004
[Non Patent document 45] British Journal of Dermatology, 152, 746, 2005
[Non Patent document 46] Clinical Experimental Immunology, 138, 342, 2004
[Non Patent document 47] Journal of the American College of Cardiology, 41, 1460, 2003
[Non Patent document 48] British Journal of Dermatology, 148, 203, 2003
[Non Patent document 49] International Journal of Cancer, 98, 567, 2002

### DISCLOSURE OF INVENTION

The present invention provides to the compounds having an excellent activity for controlling the function of CCR4, or TARC/CCL17 and/or MDC/CCL22 with few side effects, useful as the prophylactic or therapeutic agent for allergic diseases, inflammatory diseases, autoimmune diseases and so on.

To solve the above-mentioned problem, the present inventors have earnestly studied, and found that the compounds represented by the following formula have an excellent activity for controlling the function of CCR4 or TARC/CCL17 and/or MDC/CCL22. Thus the present invention was accomplished.

Namely, the present invention is as follows.
1. An aromatic compound represented by the following formula (1): 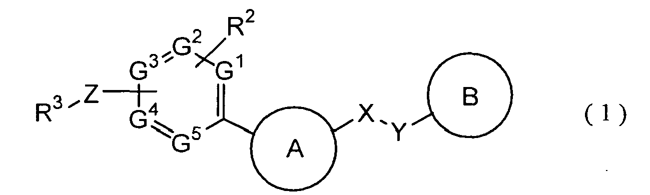 , wherein ring A is a group selected from the group consisting of the following formulas; 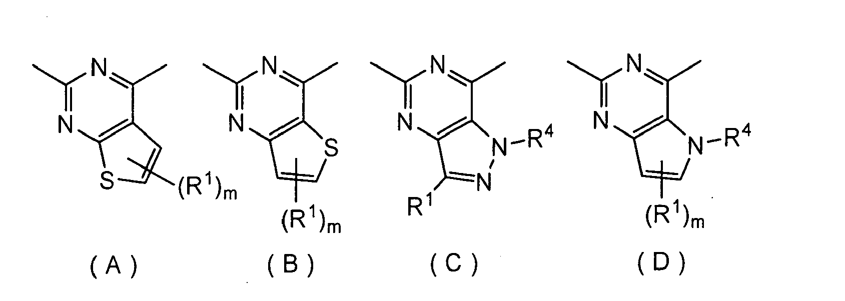 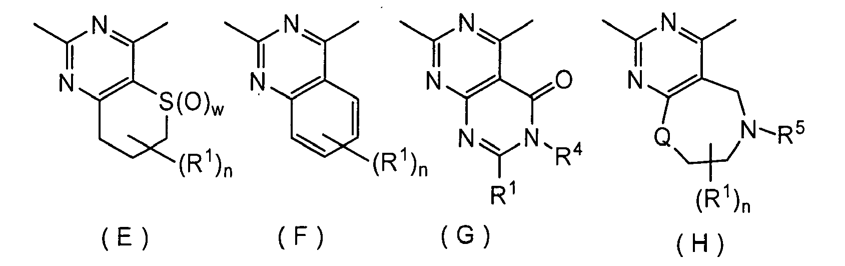 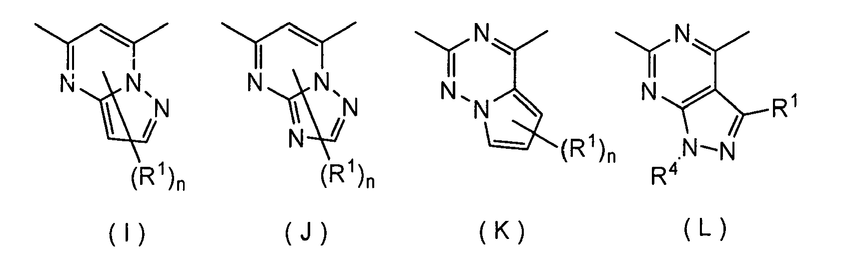 , ring B is an optionally substituted aromatic carbocyclic ring or an optionally substituted heterocyclic ring,
   G¹, G2, G3, G⁴ and G5 are each the same or different, and CH or N, provided that two or more among G¹, G², G3, G⁴ and G5 are CH,
   Q is oxygen atom, sulfur atom or -N(R⁶),
   m is an integer of 1 or 2, n is an integer of 1 to 3,
   w is an integer of 0, 1 or 2,
   X is -N(R⁷)-, -O- or -C(R⁸)(R⁹)-,
   Y is -C(R¹⁰)(R¹¹)-, -CO- or -SO₂-,
   Z is a single bond, -CO-, -SO₂-, -N(R¹²)-, -CON(R¹³)-, -SO₂N(R¹³)-, -N(R¹³)CO-, - N(R¹³)SO₂-, -N(R¹⁴)CON(R¹⁵)- or -N(R¹⁴)SO₂N(R¹⁵)-,
   R¹ is hydrogen atom, alkyl group, alkoxy group, halogen atom, carboxy group, alkoxycarbonyl group, optionally substituted carbamoyl group, optionally substituted amino group, nitro group or optionally substituted ureido group,
   R² is hydrogen atom, alkyl group, alkoxy group, halogen atom, haloalkyl group, carboxy group, alkoxycarbonyl group, optionally substituted carbamoyl group or optionally substituted amino group,
   R³ is optionally substituted carbocyclic group, optionally substituted heterocyclic group or optionally substituted alkyl group,
   R⁴ is hydrogen atom or alkyl group,
   R⁵ is hydrogen atom, alkyl group or optionally substituted alkanoyl group,
   R⁶ is hydrogen atom, alkyl group or optionally substituted alkanoyl group,
   R⁷ is hydrogen atom or alkyl group,
   R8 and R⁹, or R¹⁰ and R¹¹ are each the same or different, and hydrogen atom or alkyl group,
   R¹² is hydrogen atom, alkyl group, alkanoyl group or carboxyalkyl group,
   R¹³ is hydrogen atom or alkyl group, and
   R¹⁴ and R¹⁵ are each the same or different, and hydrogen atom or alkyl, group, or a pharmaceutically acceptable salt thereof.
2. The aromatic compound or a pharmaceutically acceptable salt thereof mentioned in above 1, wherein ring A is a group selected from the groups consisting of the following formulas: 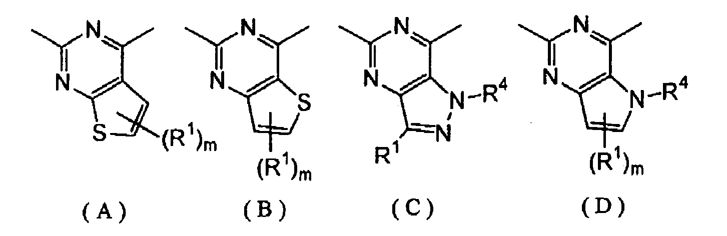 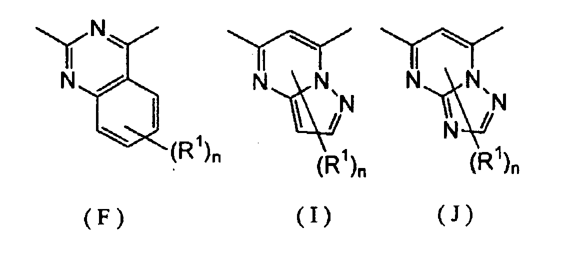 , wherein each signal is the same as defined above.
3. The aromatic compound or a pharmaceutically acceptable salt thereof mentioned in above 1 or 2, wherein ring A is a group selected from the group consisting of the following formulas: 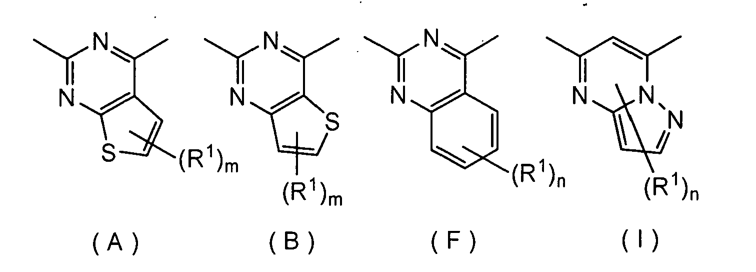 , wherein each signal is the same as defined above.
4. The aromatic compound or a pharmaceutically acceptable salt thereof mentioned in any one of above 1 to 3, wherein Z is a single bond, -CONH-, - NHCO- or -CO-.
5. The aromatic compound or a pharmaceutically acceptable salt thereof mentioned in any one of above 1 to 4, wherein R³ is
   (1) pyrrolidinyl group which is optionally substituted by (a) oxo group, (b) hydroxymethyl group, (c) alkyl group, (d) amino group which is optionally substituted by one or two alkyl group(s), or (e) carbamoyl group which is optionally substituted by one or two alkyl group(s),
   (2) piperidinyl group which is optionally substituted by alkyl group, alkanoyl group, cyano group, amino group which is optionally substituted by one or two alkyl group(s) or oxo group,
   (3) piperadinyl group which is optionally substituted by alkyl group,
   (4) morpholinyl group which is optionally substituted by alkyl group, or
   (5) tetrahydropyridyl group which is optionally substituted by alkyl group.
6. The aromatic compound or a pharmaceutically acceptable salt thereof mentioned in any one of above 1 to 5, wherein X is -NH-, Y is -CH₂-, -CH(CH₃)-or -C(CH₃)₂-, and ring B is benzene which is substituted by one or two substituents selected from the group consisting of halogen atom, alkyl group and haloalkyl group.
   In the compounds (I) of the present invention, other preferable embodiments include following compounds.
7. An aromatic compound represented by the following formula (1) 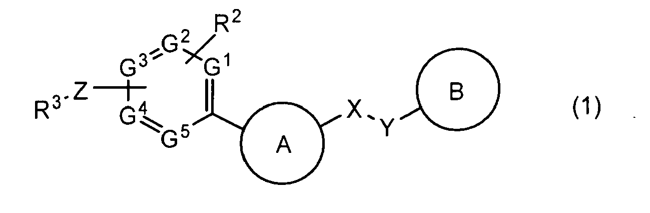 , wherein ring A is a group selected from the following formulas; 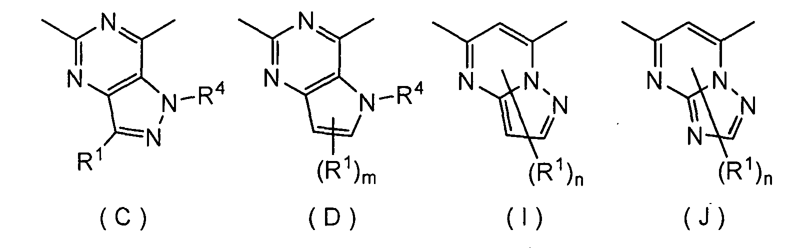 , ring B is an aromatic carbocyclic ring which is optionally substituted by one to three and the same or different substituents selected from halogen atom and cyano group,
   ring: 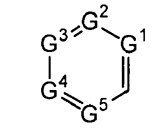 is benzene, pyridine or pyrimidine,
   m is an integer of 1 or 2, n is an integer of 1 to 3,
   X-Y is -NH-CH₂- or -NH-CH(CH₃)-,
   Z is a single bond, -CO- or -N(R¹²)-,
   R¹ is hydrogen atom, alkyl group, alkoxy group, halogen atom, carboxy group, alkoxycarbonyl group, carbamoyl group which is optionally substituted, amino group which is optionally substituted, nitro group, or ureide group which is optionally substituted,
   R² is hydrogen atom, alkyl group, alkoxy group, halogen atom, haloalkyl group, carboxy group, alkoxycarbonyl group, carbamoyl group which is optionally substituted, amino group which is optionally substituted,
   R³ is a heterocyclic group which is optionally substituted,
   R⁴ is hydrogen atom or alkyl group,
   or a pharmaceutically acceptable salt thereof.
8. The aromatic compound or a pharmaceutically acceptable salt thereof mentioned in above 7, whereinR¹ is hydrogen atom.
9. The aromatic compound or a pharmaceutically acceptable salt thereof mentioned in above 7 or 8, whereinR² is hydrogen atom, alkyl group or amino group which is optionally substituted.
10. The aromatic compound or a pharmaceutically acceptable salt thereof mentioned in above 7 or 8, wherein R² is hydrogen atom.
11. The aromatic compound or a pharmaceutically acceptable salt thereof mentioned in any one of above 7 to 10, whereinR³ is pyrrolidinyl group which is optionally substituted, piperidinyl group which is optionally substituted, piperazinyl group which is optionally substituted, morpholinyl group which is optionally substituted or tetrahydropyridinyl group which is optionally substituted.
12. The aromatic compound or a pharmaceutically acceptable salt thereof mentioned in any one of above 7 to 11, wherein R³ is
   (1) pyrrolidinyl group which is optionally substituted by (a) oxo group, (b) hydroxymethyl group, (c) alkyl group, (d) amino group which is optionally substituted by one or two alkyl group(s), or (e) carbamoyl group which is optionally substituted by one or two alkyl group(s),
   (2) piperidinyl group which is optionally substituted by alkyl group, alkanoyl group, cyano group, amino group which is optionally substituted by one or two alkyl group(s) or oxo group,
   (3) piperazinyl group which is optionally substituted by alkyl group,
   (4) morpholinyl group which is optionally substituted by alkyl group, or
   (5) tetrahydropyridyl group which is optionally substituted by alkyl group.
13. The aromatic compound or a pharmaceutically acceptable salt thereof mentioned in any one of above 7 to 12, wherein ring B is benzene which is optionally substituted by one to three and the same or different substituents selected from halogen atom and cyano group.
14. The aromatic compound or a pharmaceutically acceptable salt thereof mentioned in any one of above 7 to 13, wherein ring 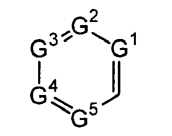 is benzene.
15. The aromatic compound or a pharmaceutically acceptable salt thereof mentioned in any one of above 7 to 14, wherein ring A is a group selected from the group consisting of the following formulas: 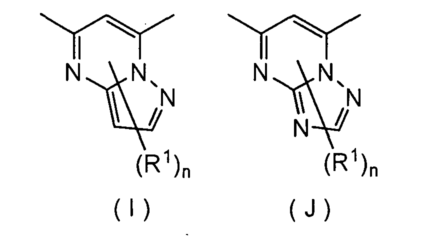 Furthermore, the following embodiments are the subject of the present invention.
16. A medicament comprising the compound or a pharmaceutically acceptable salt thereof mentioned in any one of above 1 to 15.
17. An agent for controlling the function of CCR4 or TARC/CCL17 and/or MDC/CCL22 comprising the compound or a pharmaceutically acceptable salt thereof mentioned in any one of above 1 to 15 as an active ingredient, and a method for controlling said function by administering said compound to a patient.
18. An therapeutic or prophylactic agent for allergic disease, inflammatory disease, autoimmune disease or cancer containing the compound or a pharmaceutically acceptable salt thereof mentioned in any one of above 1 to 15 as an active ingredient, and a method for treating said disease by administering said compound to a patient.
19. An therapeutic or prophylactic agent for asthma or dermatitis containing the compound or a pharmaceutically acceptable salt thereof mentioned in any one of above 1 to 15 as an active ingredient, and a method for treating said disease by administering said compound to a patient.
   Each signal used in the present specification is explained below. Furthermore, the abbreviations used in the present specification mean as follows.
   THF: tetrahydrofuran
   DMF: N,N-dimethylformamide
   DMSO: dimethyl sulfoxide
   DMA: dimethylacetamide
   DME: 1,2-dimethoxyethane
   LDA: lithium diisopropylamide
   DBU: 1,8-diazabicyclo[5.4.0]-7-undecene
   DBN: 1,5-diazabicyclo[4.3.0]nona-5-ene
   Ac: acetyl
   Me: methyl
   Et: ethyl
   Pr: n-propyl
   iPr: isopropyl
   t-Bu: tert-butyl
   Boc: tert-butoxycarbonyl
   Bn: benzyl
   Ph: phenyl

"Aromatic carbocyclic ring" includes for example, a 6 to 14 membered monocyclic, bicyclic and tricyclic unsaturated carbocyclic rings, such as benzene, naphthalene, phenanthrene, anthracene and so on.

"Heterocyclic ring" includes for example, a 3 to 15 membered monocyclic and bicyclic unsaturated, saturated or partially saturated heterocyclic rings which contain 1 to 4 hetero atoms selected from the group consisting of nitrogen atom, oxygen atom and sulfur atom, such as pyrrole, imidazole, triazole, tetrazole, pyrazole, pyridine, pyrazine, pyrimidine, pyridazine, azepine, diazepine, furan, pyran, oxepin, thiophene, thiapyran, thiepine, oxazole, isoxazole, thiazole, isothiazole, frazan, oxadiazole, oxazine, oxadiazine, oxazepine, oxadiazepine, thiadiazole, thiazine, thiadiazine, thiazepine, thiadiazepine, indole, isoindole, benzofuran, benzothiophene, indazole, quinoline, isoquinoline, quinoxaline, quinazoline, benzoxazole, benzothiazole, benzimidazole, pyrroline, pyrrolidine, imidazoline, imidazolidine, pyrazoline, pyrazolidine, dihydropyridine, tetrahydropyridine, dihydropyrazine, tetrahydropyrazine, dihydropyrimidine, tetrahydropyrimidine, dihydroazepine, tetrahydroazepine, dihydrodiazepine, tetrahydrodiazepine, dihydrofuran, tetrahydrofuran, dihydropyran, tetrahydropyran, dihydrothiophene, tetrahydrothiophene, dihydrothiapyran, tetrahydrothiapyran, piperidine, piperazine, morpholine, thiomorpholine, homopiperidine and so on.

"Aliphatic heterocyclic ring" includes for example, a 5 to 7 membered monocyclic and saturated hetero ring which contains 1 or 2 hetero atoms selected from the group consisting of nitrogen atom, oxygen atom and sulfur atom, such as piperidine, piperazine, morpholine, thiomorpholine, homopiperidine, tetrahydrooxazine and so on.

"Alkyl group" includes for example, straight or branched C₁ to C₆ alkyl, such as methyl, ethyl, propyl, isopropyl, butyl, isobutyl, tert-butyl, pentyl, hexyl and so on.

"Alkoxy group" includes for example, straight or branched C₁ to C₆ alkoxy group, such as methoxy, ethoxy, propoxy, isopropoxy, butoxy, isobutoxy, tert-butoxy, pentyloxy, hexyloxy and so on.

"Halogen atom" includes fluorine atom, chlorine atom, bromine atom and iodine atom.

"Haloalkyl group" includes for example, straight or branched C₁ to C₆ alkyl group which is substituted by 1 to 6 halogen atoms such as fluoromethyl, chloromethyl, trifluoromethyl, 2,2,2-trifluoroethyl and so on.

"Alkoxycarbonyl group" includes for example, straight or branched C₂ to C₇alkoxycarbonyl group, such as methoxycarbonyl, ethoxycarbonyl, propoxycarbonyl, isopropoxycarbonyl, butoxycarbonyl, isobutoxycarbonyl, tert-butoxycarbonyl and so on.

"Alkanoyl group" includes for example, straight or branched C₁ to C₆alkanoyl, such as formyl, acetyl, propionyl, butyryl, pentanoyl, hexanoyl and so on.

"Carboxyalkyl group" includes straight or branched C₁ to C₆ alkyl substituted by one or two carboxy groups, such as carboxymethyl, carboxyethyl, carboxypentyl and so on.

"Aralkyl group" includes for example, straight or branched C₁ to C₆ alkyl substituted by aromatic carbocyclic group (preferably benzene), such as benzyl, 2-phenylethyl, 1-phenylethyl, 3-phenylpropyl and so on.

"Cycloalkyl group" includes for example, C₃ to C₆ cycloalkyl group, such as cyclopropyl, cyclobutyl, cyclopentyl, cyclohexyl and so on.

"Alkylsulfonyl group" includes for example, straight or branched C₁ to C₆ alkylsulfonyl group, such as methylsulfonyl, ethylsulfonyl, propylsulfonyl, isopropylsulfonyl, butylsulfonyl and so on.

"Carbocyclic group" includes for example, a 3 to 15 membered monocyclic, bicyclic, or tricyclic and unsaturated, saturated or partially saturated carbocyclic group, such as phenyl, naphthyl, phenanthryl, anthryl, cyclopropyl, cyclobutyl, cyclopentyl, cyclohexyl, cycloheptyl, cyclooctyl, cyclopentenyl, cyclohexenyl, cycloheptenyl, cyclooctenyl, cyclopentadienyl, cyclohexadienyl, cycloheptadienyl, cyclooctadienyl, indenyl, indanyl, dihydronaphthyl, tetrahydronaphthyl and so on.

"Heterocyclic group" includes for example, a 3 to 15 membered monocyclic or bicyclic unsaturated, saturated or partially saturated heterocyclic groups which contain a 1 to 4 hetero atoms selected from the group consisting of nitrogen atom, oxygen atom and sulfur atom, such as pyrrolyl, imidazolyl, triazolyl, tetrazolyl, pyrazolyl, pyridyl, pyrazinyl, pyrimidinyl, pyridazinyl, azepinyl, diazepinyl, furyl, pyranyl, oxepinyl, thienyl, thiapyranyl, thiepinyl, oxazolyl, isoxazolyl, thiazolyl, isothiazolyl, furazanyl, oxadiazolyl, oxazinyl, oxadiazinyl, oxazepinyl, oxadiazepinyl, thiadiazolyl, thiazinyl, thiadiazinyl, thiazepinyl, thiadiazepinyl, indolyl, isoindolyl, benzofuranyl, benzothiophenyl, indazolyl, quinoliny, isoquinolinyl, quinoxalinyl, quinazolinyl, benzoxazolyl, benzothiazolyl, benzoimidazolyl, pyrrolinyl, pyrrolidinyl, imidazolinyl, imidazolidinyl, pyrazolinyl, pyrazolidinyl, dihydropyridyl, tetrahydropyridyl, dihydropyrazinyl, tetrahydropyrazinyl, dihydropyrimidinyl, tetrahydropyrimidinyl, dihydroazepinyl, tetrahydroazepinyl, dihydrodiazepinyl, tetrahydrodiazepinyl, dihydrofuryl, tetrahydrofuryl, dihydropyranyl, tetrahydropyranyl, dihydrothienyl, tetrahydrothienyl, dihydrothiapyranyl, tetrahydrothiapyranyl, piperidyl, piperazinyl, morpholinyl, thiomorpholinyl, homopiperidyl and so on.

"Aliphatic heterocyclic group" includes for example, 5 to 7 membered monocyclic and saturated heterocyclic groups which contain 1 or 2 hetero atoms selected from the group consisting of nitrogen atom, oxygen atom and sulfur atom, such as piperidyl, piperazinyl, morpholinyl, thiomorpholinyl, homopiperidyl, tetrahydrooxazinyl and so on.

Substituents on "optionally substituted aromatic carbocyclic group" and "optionally substituted heterocyclic ring" in ring B include for example, halogen atom, alkyl group, haloalkyl group, hydroxy group, alkoxy group, cyano group, carboxy group, alkoxycarbonyl group, nitro group and so on, preferably halogen atom, alkyl group and haloalkyl group, especially preferably halogen atom. Said ring may have 1 to 3 said substituents.

Substituents on "optionally substituted carbamoyl group" in R¹ and R² include for example, alkyl group which may be substituted by hydroxy group, alkoxy group or alkyl sulfonyl; aralkyl group and so on. The carbamoyl group may have the same or different, and 1 or 2 said substituents. R¹ and R² may be taken together and form heterocyclic ring with the adjacent nitrogen atom, such as pyrrolidine, pyperidine, morpholine, thiamorpholine, homopyperidine, which may have a substituent such as oxo group.

Substituents on "optionally substituted amino group" in R¹ and R² include for example, alkyl group, optionally substituted alkanoyl group, alkylsulfonyl group, optionally substituted alkoxycarbonyl group, cycloalkylcarbonyl group, hydroxy group and so on. The amino group may have the same or different, and 1 or 2 said substituents. Substituents on said optionally substituted alkanoyl group and said optionally substituted alkoxycarbonyl group herein include for example, alkoxy group, hydroxy group and so on.

Substituents on "optionally substituted ureido group" in R¹ include for example, alkyl group and so on. The ureido group may have the same or different, and 1 or 2 said substituents. Said two substituents may be taken together and form a 5 to 7 membered aliphatic heterocyclic ring with the adjacent nitrogen atom.

Substituents on "optionally substituted carbocyclic group" in R³ include for example, aliphatic heterocyclic ring which may be substituted by oxo group, optionally substituted alkyl group, cyano group, optionally substituted amino group, alkylenedioxy group and so on. Substituents on said optionally substituted alkyl group include for example, cyano group and so on. Substituents on said optionally substituted amino group include for example, alkylsulfonyl group and so on.

Substituents on "optionally substituted heterocyclic group" in R³ include for example, (a) oxo group, (b) carboxy group, (c) alkoxycarbonyl group, (d) amino group which is optionally mono- or di-substituted by sulfonyl group substituted by heterocyclic group which is optionally substituted by alkyl group, or alkyl group, (e) heterocyclic group which is optionally substituted by oxo group, alkyl group, alkylsulfonyl group or alkanoyl group, (f) alkyl group which is optionally substituted by phenyl, (g) carbamoyl group which is optionally mono-or di-substituted by alkyl group, (h) alkylsulfonyl group, (i) alkanoyl group, (j) phenyl group which is optionally substituted by alkoxy group, (k) halogen atom, (i) cyano group, (m) hydroxy group, (n) alkoxy group and so on.

Substituents on "optionally substituted alkyl group" in R³ include, for example, halogen atom, hydroxy group, alkoxy group, amino group which is optionally mono-or di-substituted by alkyl group, optionally substituted heterocyclic group and so on. The alkyl group may have the same or different, and 1 or 2 said substituents. Substituents on said optionally substituted heterocyclic group herein are the same as the substituents on heterocyclic group in R³.

Substituents on "optionally substituted alkanoyl group" in R⁵ and R⁶ include for example, cycloalkyl group and so on.

Preferable examples of the group represented by the following formula, 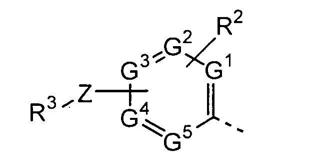 are as follows:

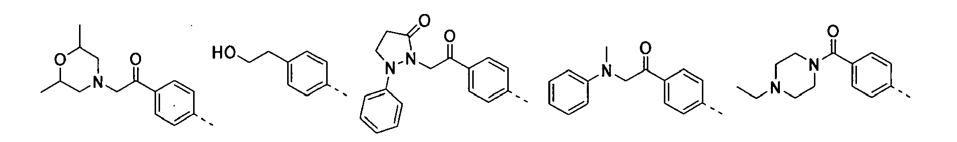

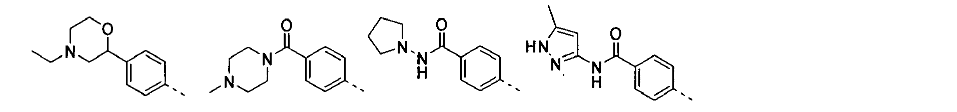

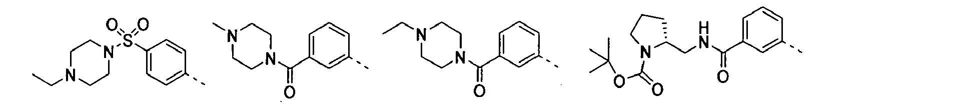

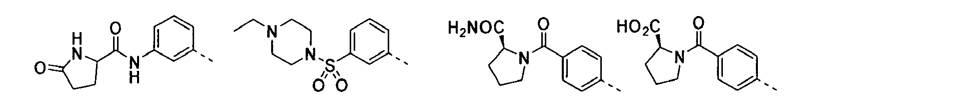

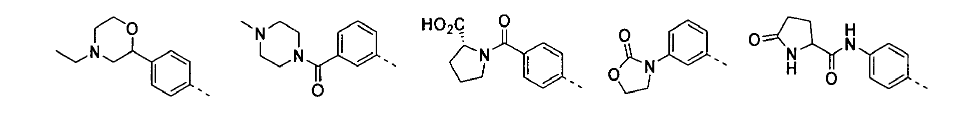

The pharmaceutically acceptable salt of the aromatic compound of the present invention includes an inorganic acid salt, such as hydrochloride, sulfate, phosphate or hydrobromide, an organic acid salt such as acetate, fumarate, oxalate, citrate, methanesulfonate, benzenesulfonate, tosylate or malate. Furthermore, when the compound has an acidic group such as carboxy group and so on, the salt with a base such as an alkali metal salt, e.g., sodium salt, potassium salt, an alkaline earth salt, e.g., calcium salt, an organic base salt, e.g., triethylamine salt, or an amino acid salt, e.g., lysine salt are also included therein.

The pharmaceutically acceptable salt of the aromatic compound of the present invention includes its inner salt, or its solvate such as hydrate.

The compound (1) of the present invention exists in optically active isomers based on its asymmetric carbon, and includes any of forms of its isomers and a mixture thereof. Furthermore, when the compound (1) has a double bond or cycloalkandiyl group, the compound exists in trans or cis configuration and includes any configurations and a mixture thereof. When the compound of the present invention has an amino group, its quaternary ammonium salt is included, too.

The compound (1) of the present invention can be prepared by the following methods.

Method 1: Compound (1) is prepared by the following method. 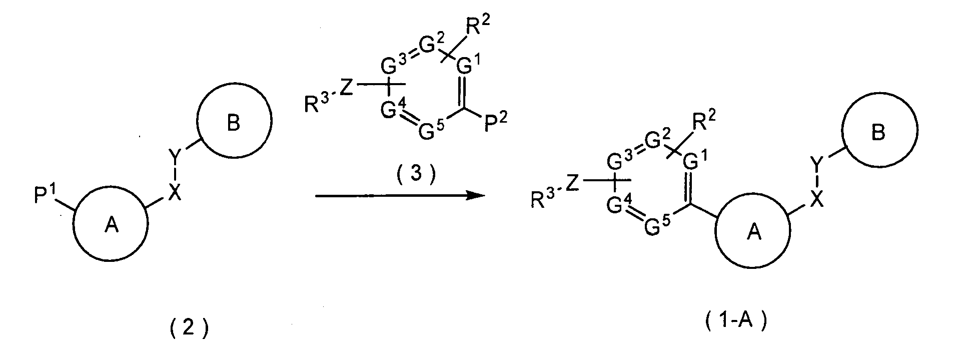 ,wherein, P¹ is a leaving group such as halogen atom or alkylsulfonyloxy group, P² is -B(OH)₂, -B(OR^{A})₂ or -Sn(R^{A})₃, R^{A} is alkyl, and the other signals are the same as defined above.

Compound (2) is reacted with compound (3) under the palladium catalyst, such as tetrakis(triphenylphosphine)palladium(O), bis(triphenylphosphine)palladium(II) chloride, palladium(II) acetate and so on, in a solvent such as DME, THF, dioxane, DMF, DMA, toluene, benzene, water or a mixture thereof, to give the compound (1-A).

When compound (3) wherein P² is -B(OH)₂ or -B(OR^{A})₂ is used, a base is preferably added in this reaction, such as an inorganic base, e.g., an alkali metal carbonate, an alkali metal hydroxide, an alkali metal phosphate or alkali metal fluoride, or an organic base, e.g., triethylamine.

The reaction is usually at room temperature to 150°C for 1 to 24 hours. Method 2: Compound (1) wherein R³ is heterocyclic ring containing nitrogen atom, and Z is -CO- or -N(R¹³)CO-, is prepared by the following method. 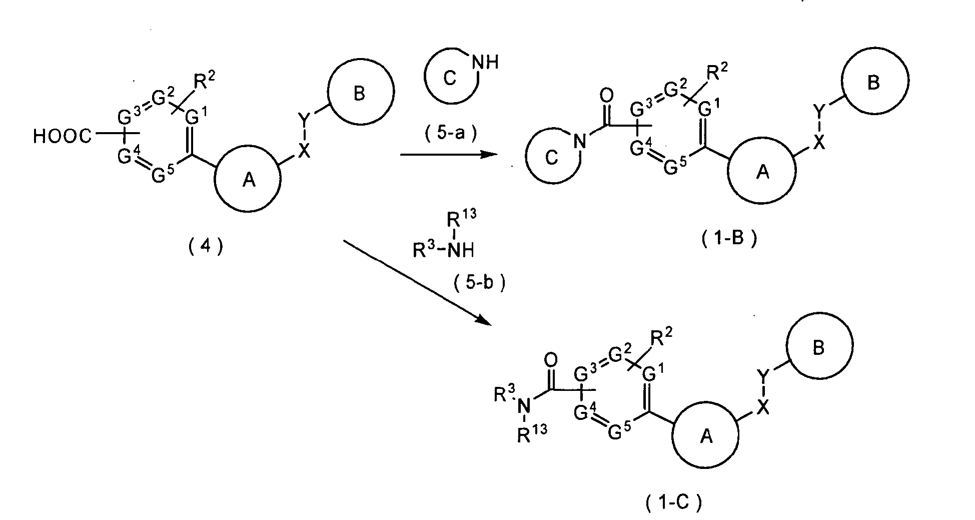 ,wherein, ring C is a saturated or partially saturated 5 to 7 membered heterocyclic ring containing a nitrogen atom such as optionally substituted piperazine, optionally substituted piperidine, optionally substituted pyrrolidine, and the other signals are the same as defined above.

This reaction is carried out by either of the following methods.
(1) Compound (4) is treated with a halogenating agent (thionyl chloride or oxalyl chloride, if necessary in DMF) to prepare the acid chloride, and then reacted with compound (5-a) or (5-b) in the presence of a base (sodium hydrogencarbonate, potassium hydrogencarbonate, potassium carbonate, sodium carbonate, triethylamine, pyridine, etc.) at -20 to 100°C for 30 minutes to 24 hours to give compound (1-B) or (1-C).
(2) Compound (4) is condensed with compound (5-a) or (5-b) in the presence of a condensing agent [1,3-dicyclohexylcarbodiimide, 1-ethyl-3-(3-dimethylaminopropyl)carbodiimide, carbonyldiimidazole, diethyl cyanophosphate or PyBOP (benzotriazole-1-yloxy)tripyrrolidinophosphonium hexafluorophosphate)], if necessary in a solvent (DMF, THF, dioxane, dichloromethane, etc.) to give compound (1-B) or (1-C). The reaction is carried out usually at 0°C to 100°C and usually for 30 minutes to 24 hours, and if necessary in the presence of 1-hydroxybenzotriazole, or N-hydroxysuccinimide and so on.
(3) Compound (4) is converted into a mixed acid anhydride (carbonate with methyl chloroformate or ethyl chloroformate, sulfonate with mesyl chloride, or tosyl chloride) and the mixed acid anhydride is condensed with compound (5) in a suitable solvent (THF, toluene, nitrobenzene or a mixture thereof), in the presence of a base (triethylamine, pyridine, 4-dimethylaminopyridine, diethylaniline, collidine, Hunig (diisopropylethylamine, etc.), at room temperature to refluxing temperature for 1 to 24 hours to give the compound (1-B) or (1-C).

Method 3: Compound (1) wherein Z is -CON(R¹³)- or -SO₂N(R¹³)-, is prepared by the following methods. 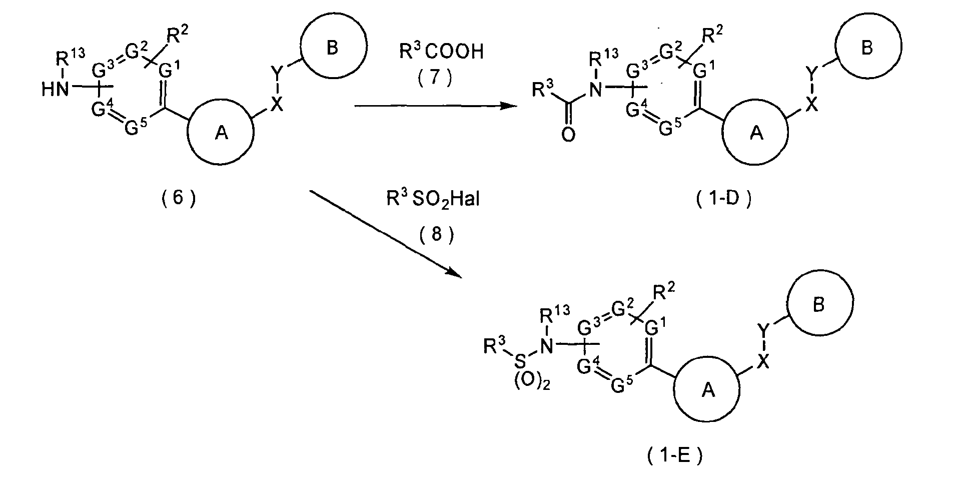 , wherein each signal is the same as defined above.

The reaction of compound (6) with compound (7) is carried out in the same manner as in method 2. The reaction of compound (6) with compound (8) is carried out in the same manner as the reaction of the acid halide in method 2 (1).

Method 4: Compound (1) wherein Z is -N(R¹⁴)CON(R¹⁵)- and R¹⁵ is hydrogen atom is prepared by the following method. 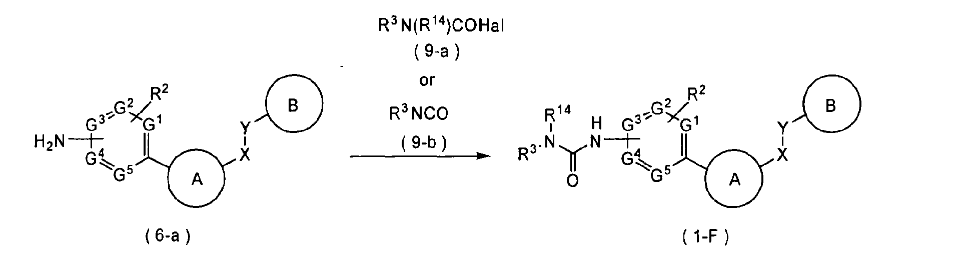 , wherein each signal is the same as defined above.

Compound (6-a) is reacted with compound (9-a) in the presence of a solvent (chloroform, dichloromethane, DMF, DMSO, dioxane, THF, etc.) or in the absence of the solvent in the presence of a base (triethylamine, diisopropylethylamine, 4-methylmorpholine, pyridine, etc.) to give compound (1-F). This reaction is preferably carried out at -40 to 100°C for 1 to 48 hours.

Otherwise, compound (6-a) is reacted with compound (9-b) in the presence of a solvent (chloroform, dichloromethane, DMF, DMSO, dioxane, THF, etc.) to give compound (1-F). This reaction is preferably carried out at -40 to 100°C for 1 to 48 hours.

Method 5: Compound (1) wherein Z is a single bond, R³ is a 5 to 7 membered saturated heterocyclic group containing nitrogen atom is prepared by the following method. 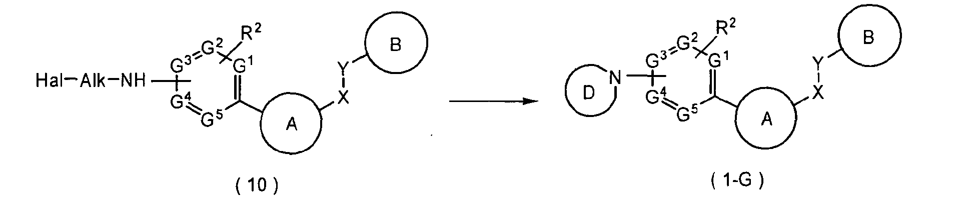 , wherein Alk is C₂ to C₆ alkylene group which is optionally substituted 1 to 3 oxo groups and interrupted by 1 to 3 nitrogen atoms, oxygen atoms or sulfur atoms, ring D is a 5 to 7 membered saturated heterocyclic ring containing nitrogen atom such as optionally substituted pyrrolidine, optionally substituted piperidine, optionally substituted piperazine, optionally substituted morpholine, optionally substituted thiomorpholine, or optionally substituted imidazolidine, optionally substituted thiazolidine, optionally substituted isothiazolidine, etc., and the other signals are the same as defined above.

Compound (10) is treated with a base (sodium hydride or potassium tert-butoxide, etc.) in a solvent (DMF, DMSO, N,N-dimethylacetamide, etc.) to give the compound (1-G). The reaction is preferably carried out at 0 to 100°C for 1 to 48 hours.

Method 6: Compound (1) wherein ring A is a group (I), X is -N(R⁵)- or -O- is prepared by the following method. 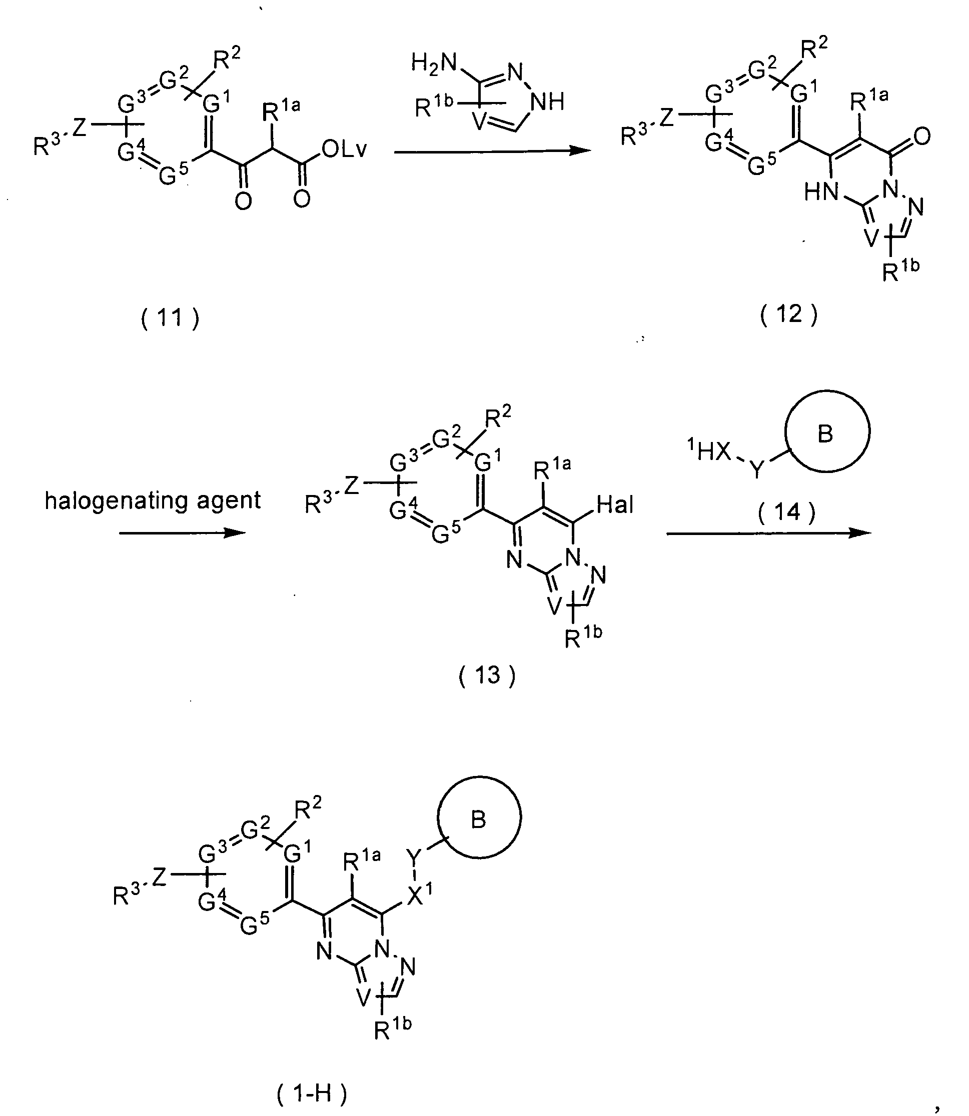 wherein, R^{1a} and R^{1b} are the same as R¹, Lv is alkyl group, optionally substituted phenyl group or optionally substituted benzyl group, V is CH or N, X¹ is -N(R⁵)- or -O-, and the other signals are the same defined above.

Compound (11) is reacted with 3-aminopyrazole in the presence of a solvent (methanol, ethanol, isopropyl alcohol, THF, dioxane) or in the absence of a solvent and in the presence of an acid (an inorganic acid such as hydrochloric acid or sulfuric acid, an organic acid such as acetic acid, etc.) to give compound (12).

Compound (12) is reacted with a halogenating agent (phosphorous oxychloride, phosphorous oxybromide, etc.) in the presence of a solvent (benzene, toluene, xylene, chloroform, methylene chloride, actonitrile, DMF, etc.) or in the absence of a solvent and if necessary in the presence of a base (dimethylaniline, diethylaniline, triethylamine, etc.) at room temperature to refluxing temperature for 1 to 12 hours to give compound (13).

Compound (13) is reacted with compound (14) in the presence of a solvent (THF, dioxane, diethyl ether, DMF, DMSO, methanol, ethanol, ethylene glycol, etc.) in the presence of a base (triethylamine, diisopropylethylamine, pyridine, sodium hydroxide, potassium hydroxide, sodium carbonate, potassium carbonate, etc.) at 0 to 150°C for 1 to 24 hours to give compound (1-H).

Method 7: Compound (2) wherein X is -N(R⁵)- or -O- is prepared by the following method. 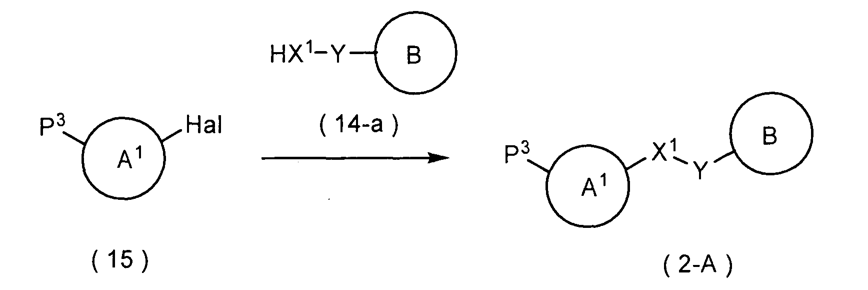 , wherein P³ is halogen atom and the other signals are the same as defined above.

The reaction with compound (15) and compound (14-a) is carried out in the same manner as in method 6.

### Method 8:

Method 8: Compound (15) wherein ring A¹ is group (C) is prepared by the following method. 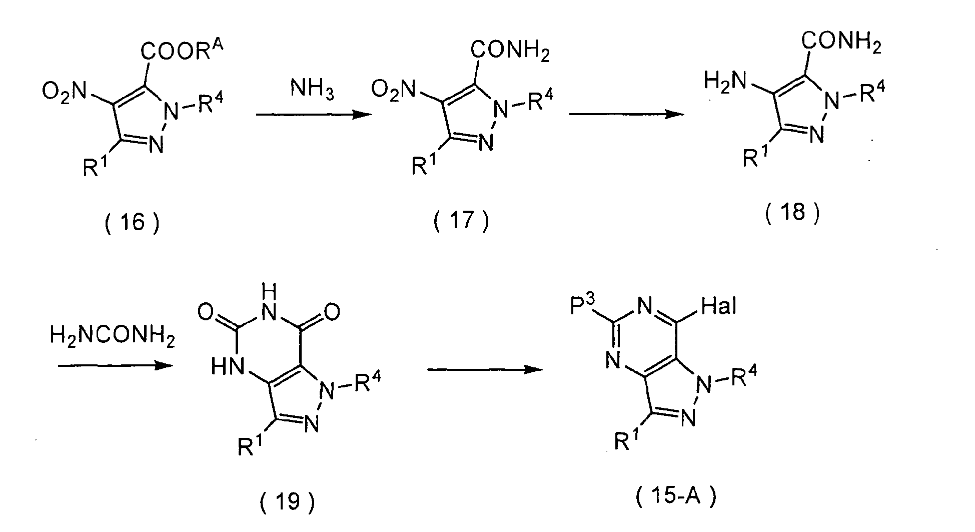 , wherein R^{A} is hydrogen atom or alkyl group, and the other signals are the same as defined above.

After compound (16) is converted to compound (17) by treating with aqueous ammonium in the conventional manner, compound (17) was reduced in a solvent such as water, methanol, tert-butyl alcohol, THF, dioxane, ethyl acetate, acetic acid, xylene, DMF, DMSO or a mixture thereof to give compound (18).

The reduction can be carried out using sodium borohydride, lithium borohydride, lithium alminium hydride, etc., or the catalytic reduction can be carried out using a metal such as Fe, Zn, Sn, etc., or a transition metal such as palladium-C, platinum oxide, Raney nickel, rhodium, ruthenium, etc. In case of the catalytic reduction, oxalic acid, ammonium oxalate, 1,4-cyclohexadiene, etc., may be used as hydrogen sauce. The reaction is usually completed at - 20∼150°C for 30 minutes to 48 hours.

Compound (19) is prepared by reacting compound (18) with urea at 100∼250°C for 1∼12 hours.

Compound (19) is reacted with a halogenating agent such as phosphorus oxychloride, phosphorus oxybromide, etc., in a solvent such as benzene, toluene, xylene, chloroform, methyl chloride, acetonitrile, DMF, etc., or without solvent, and if necessary in the presence of a base such as dimethylaniline, diethylaniline, triethylamine, colidine, pyridine, diisopropylethylamine, etc., at room temperature to refluxing temperature for 1 to 12 hours to give compound (15-A).

Method 9: Compound (15) wherein ring A¹ is group (D) is prepared by the following method. 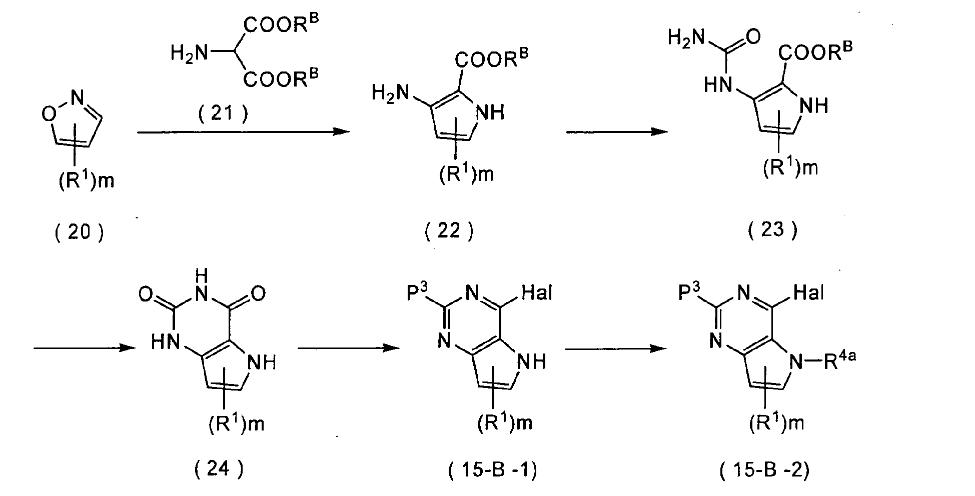 , wherein R^{B} and R^{4a} are alkyl group, and the other signals are the same as defined above.

An 3-oxopropionitrile derivative which is prepared by treating compound (20) in as solvent such as methanol, ethanol, etc., in the presence of a strong base such as sodium methoxide, potassium methoxide, sodium ethoxide, potassium ethoxide, etc., is reacted with compound (21) in the presence of a weak base such as sodium acetate, potassium acetate, sodium carbonate, et al., to give compound (22).

Compound (22) is reacted with a cyanate such as sodium cyanate, potassium cyanate, etc., in a solvent such as methanol, ethanol, acetic acid, water or a mixture thereof to give compound (23). This reaction is carried out at 0°C to 100°C, preferably at room temperature for 1∼12 hours.

Compound (23) is treated with a base such as sodium hydroxide, potassium hydroxide, sodium carbonate, potassium carbonate, etc., in a solvent such as water, methanol, ethanol, DMSO, DMF, etc., to give compound (24). This reaction is carried out at 0∼150°C, preferably at a refluxing temperature of the solvent for 1∼12 hours.

Compound (24) is reacted with a halogenating agent in the same manner as method 8 to give compound (15-B-1). Further, compound (15-B-1) is conventionally reacted with an alkyl halide to give compound (15-B-2). Method 10: Compound (15) wherein Ring A¹ is group (I) or (J) is prepared by the following method. 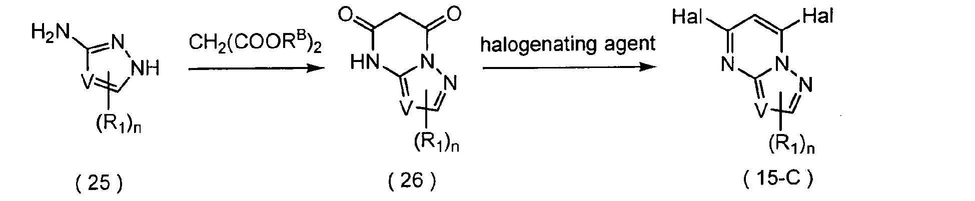 , wherein V is CH or N and the other signals are the same as defined above.

Compound (25) is reacted with dialkyl malonate in a solvent such as methanol, ethanol, isopropyl alcohol, etc., in the presence of a base such as sodium methoxide, sodium ethoxide, potassium methoxide, etc., at room temperature to refluxing temperature of the solvent for 1 to 48 hours to give compound (26).

Compound (26) is reacted with a halogenating agent in the same manner as method 8 to give compound (15-C).

Method 11: Compound (15) wherein ring A is group (A) or (B) is prepared by the following method. 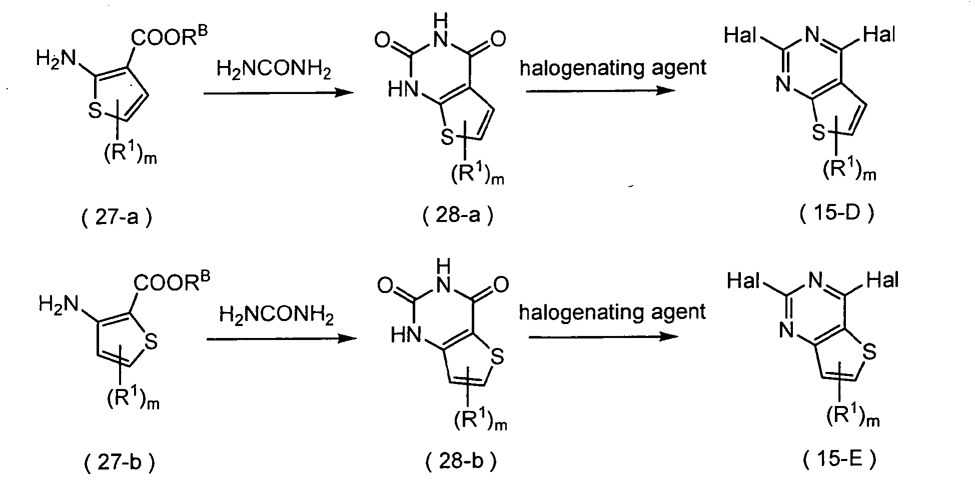 , wherein each signal is the same as define above.

Compound (27-a) or compound (27-b) is reacted with urea at 100∼250°C for 1∼12 hours to give compound (28-a) or compound (28-b).

Compound (28-a) or compound (28-b) is reacted with a halogenating agent in the same manner as method 8 to give compound (15-D) or compound (15-E).

Method 12: Compound (15) wherein ring A is group (F) is prepared by the following method. 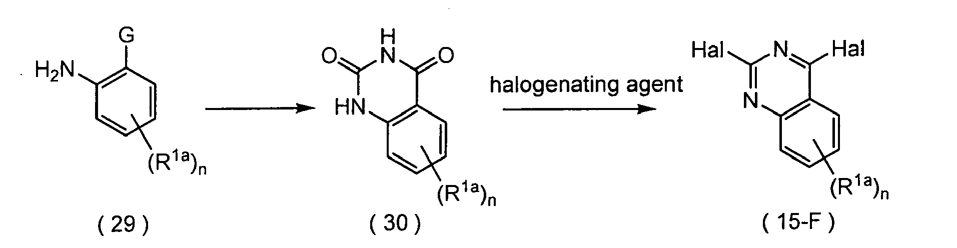 , wherein G is carboxy group or cyano group, and the other signals are the same as defined above.

Compound (29) is prepared in the same manner as the methods described in Tetrahedron, 58, (2002) 3155-3158 or WO95/32205. Namely (1) compound (29) wherein G is cyano group is reacted with carbon dioxide in a solvent such as DMF, DMSO, THF, etc., in the presence of excessive amount of a base such as DBU, DBN, etc., at room temperature of room temperature to 100°C for 1 to 48 hours, or (2) compound (29) wherein G is carboxy group is reacted with urea at 100∼250°C for 1∼12, to give compound (30).

Compound (30) is reacted with a halogenating agent in the same manner as method 8 to give compound (15-F).

Method 13: Compound (15) wherein ring A is group (E) is prepared by the following method. 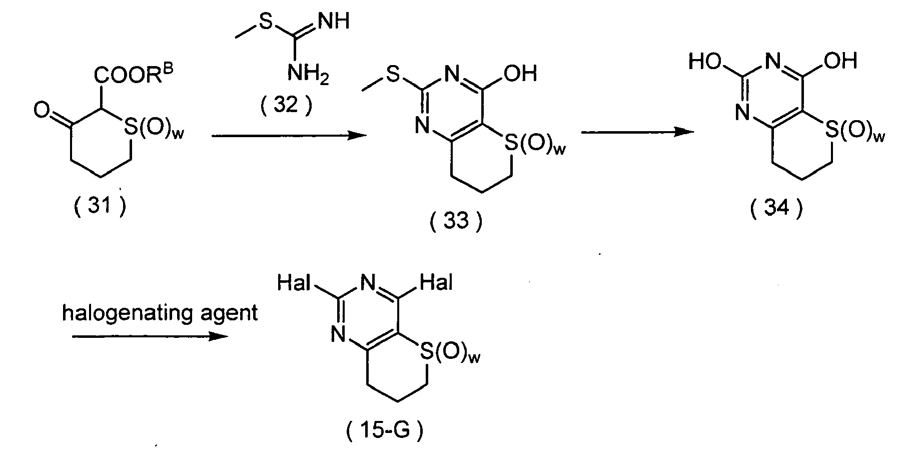 , wherein each signal is the same as defined above.

The reaction is carried out in the same manner as the method described in Japanese patent A58-146586. Namely compound (31) is reacted with compound (32) or its salt such as hydrochloride, sulfate, etc., in a solvent such as methanol, ethanol, isopropanol, DMF, DMSO, etc., in the presence of a base such as sodium hydroxide, potassium hydroxide, sodium carbonate, potassium carbonate, sodium hydride, sodium methoxide, sodium ethoxide, etc., at room temperature to 100°C for 1∼12 hours to give compound (33).

Compound (33) is reacted with an acid such as acetic acid, hydrochloric acid, sulfuric acid, etc., or an alkali such as sodium hydroxide, potassium hydroxide, etc., in a solvent such as methanol containing water, ethanol containing water, water, etc., at room temperature to refluxing temperature of the solvent for 1 hour to 3 days to give compound (34).

Compound (34) is reacted with a halogenating agent in the same manner as method 8 to give compound (15-G).

Method 14: Compound (29) wherein G is cyano group and R^{1a} is alkoxycarbonyl group is prepared by the following method. 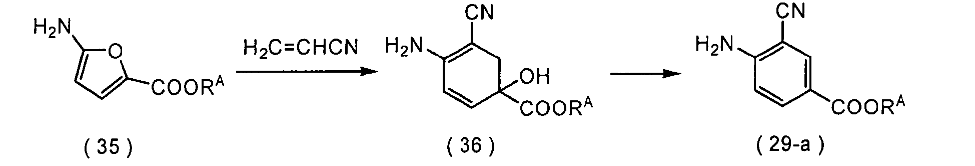 , wherein each signal is the same as defined above.

Compound (35) is reacted with acrylonitrile in a solvent such as benzene, toluene, xylene, chloroform, methyl chloride, etc., at room temperature to refluxing temperature of the solvent for 1 to 24 hours to give compound (36).

Compound (36) is reacted with boron trifluoride·diethyl ether complex preferably at refluxing temperature for 1 to 12 hours to give compound (29-a). Method 15: Compound (1) wherein ring A is group (G), X is -N(R⁷)- or -O- and Y is -C(R¹⁰)(R¹¹)-, is prepared by the following method. 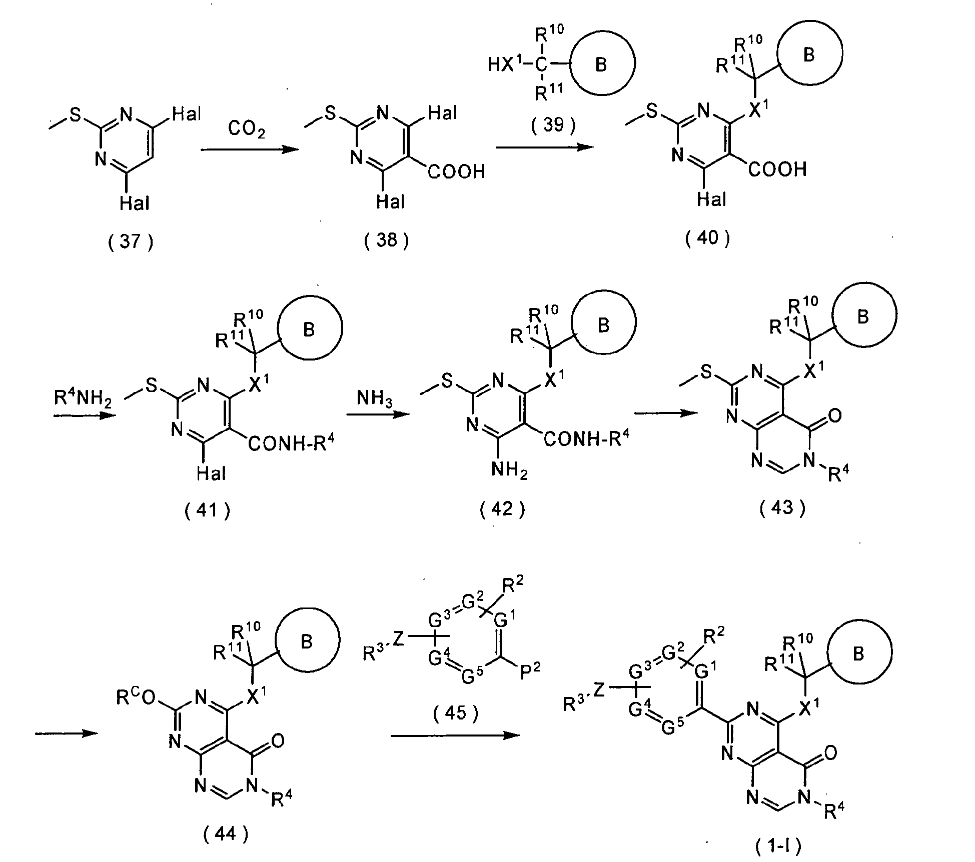 , wherein R^{C} is a leaving group (trifluoromethanesulfonyl group, etc.) and the other signals are the same as defined above.

Compound (37) is reacted with a base such as n-butyllithium, LDA, etc., in a solvent such as THF, diethyl ether, dioxane, etc., at -78°C to ice cooling, followed by reacting with carbon dioxide for 1 to 12 hours at the same temperature to give compound (38).

Compound (38) is reacted with compound (39) in the same manner as method 6 to give compound (40).

Compound (40) is reacted with R⁴NH₂ in the same manner as method 2.

Compound (40) is conventionally treated with a halogenating agent such as thionyl chloride etc., to give acid halide of compound (40).

Compound (41) is reacted with ammonia in as solvent such as THF, diethyl ether, dioxane, etc., at 0°C to refluxing temperature of the solvent for 1 hour to 10 days to give compound (42).

Compound (42) is reacted with trialkyl orthoformate such as trimethyl orthoformate, tripropyl orthoformate, tributyl orthoformate, etc., in the presence of an acid such as acetic acid, acetic anhydride, hydrochloric acid, sulfuric acid, etc., at room temperature to 150°C for 1∼12 hours to give compound (43).

After compound (43) is hydrolyzed in the same manner as method 13 to give compound (43) wherein R^{C} is hydrogen atom, the compound is treated with anhydrous trifluoromethanesulfonic acid to convert Rc into a leaving group to give compound (44).

Compound (44) is reacted with compound (45) in the same manner as method 1 o give compound (1-1).

Method 16: Compound (1) wherein Ring A is group (H), X is -N(R⁷)- or -O- and Y is -C(R¹⁰)(R¹¹)-, is prepared by the following method. 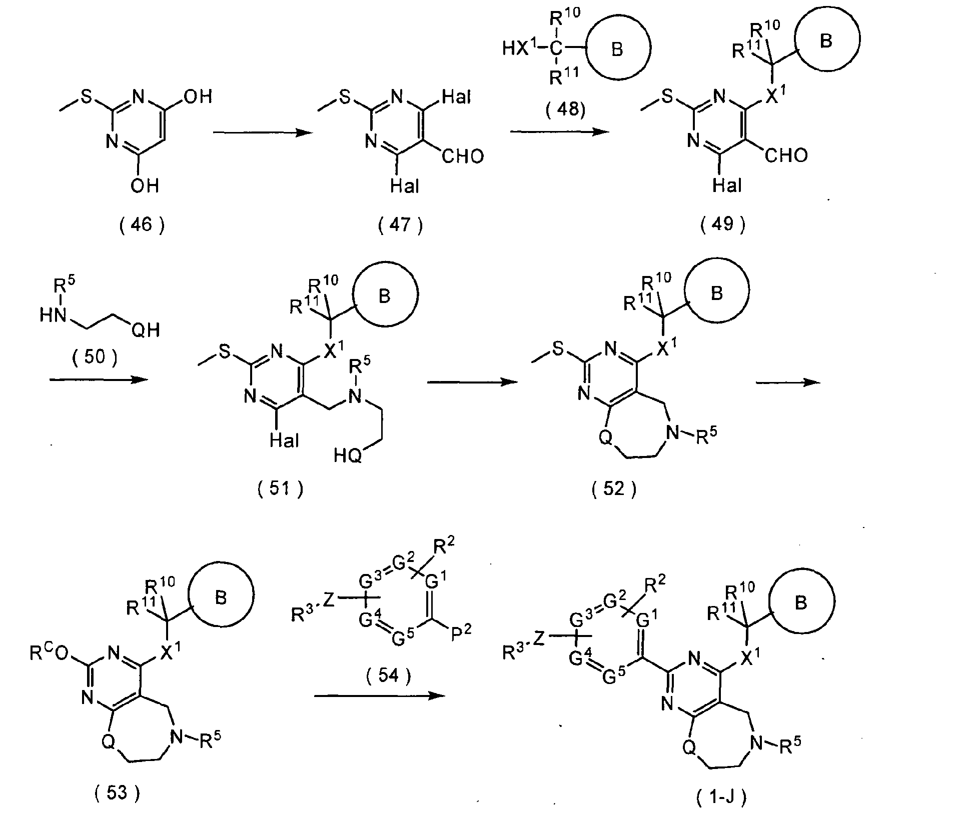 , wherein each symbol is the same as defined above.

Compound (46) is reacted with DMF and oxyphosphorus chloride or oxyphosphorus bromide at room temperature to refluxing temperature of the solvent for 1∼12 hours to give compound (47).

The reaction of compound (47) and compound (48) is carried out in the same manner as method 6.

Compound (49) is reacted with compound (50) in the presence of a reducing agent such as sodium borohydride, sodium cyanoborohydride, sodium triacetoxyborohydride, etc., and if necessary in the presence of an acid such as hydrochloric acid, sulfuric acid, acetic acid, trifluoroacetic acid in a solvent such as methanol, ethanol, isopropyl alcohol, methyl chloride, DMF, DMSO, THF, dioxane, etc., to give compound (51). This reaction is carried out at room temperature to refluxing temperature of the solvent for 30 minutes to 2 days.

Compound (51) is treated with a base such as triethylamine, pyridine, sodium hydride, potassium t-butoxide, sodium t-butoxide in a solvent such as THF, dioxane, diethyl ether, DMF, DMSO, etc., at room temperature to refluxing temperature of the solvent for 1 to 24 hours to give compound (52).

Compound (52) is reacted in the same manner as method 15 to give compound (53).

The compound (53) is reacted with compound (54) in the same manner as method 1 to give compound (1-J).

Method 17: Compound (1) is prepared by converting the functional groups of compound (55) in accordance of the conventional method in the field of the organic chemistry. 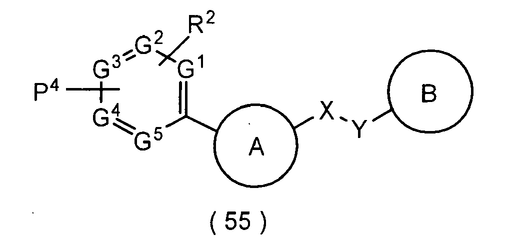 , wherein P⁴ is carboxy group, alkoxycarbonyl group, amino group, or a leaving group such as halogen atom, methanesulfonyl group, trifuruoromethanesulfonyl group, etc., and the other signals are the same as defined above.

Compound (55) can be prepared by using a corresponding starting material in the same manner as mentioned above.

Method 18: In the above methods, when the compound of the present invention, an intermediate thereof, or the starting compound has a functional group (hydroxy group, amino group, carboxy group, etc.), the functional group is protected with an ordinary protective group in the field of the organic synthetic chemistry in accordance with the method disclosed in "Protective Groups in Organic Synthesis" T. W. Greene, P. M. G. Wuts, John Wiley and Sons 1991, and then the reaction is carried out and is followed by cleavage of the protective group to give the object compound.

As the protective group, the protective groups described in the above text book and ordinarily used in the field of the organic synthetic chemistry are illustrated. For example, as protective groups of hydroxy group, tetrahydropyranyl, trimethylsilyl, tert-butyldimethylsilyl, benzyl, methoxymethyl, acetyl and so on, as protective groups of amino group, tert-butoxycarbonyl, benzyloxycarbonyl, 9-fluorenylmethoxycarbonyl, 2,2,2-trichloroethoxycarbonyl, tert-amyloxycarbonyl and so on, and as protective groups of carboxy group, alkyl group like methyl or ethyl, benzyl and so on are respectively illustrated.

Furthermore, after preparing the compound of the present invention or the intermediate thereof, the functional group is converted or modified in accordance with the conventional method. The following methods are illustrated.
(1) Conversion of amino into amide.
   The amino group can be converted into the corresponding amide group by reacting amino group with an acyl halide, or by condensing carboxy group with an amine in the presence of condensing agent.
(2) Conversion of carboxy or ester thereof into carbamoyl.
   The carboxy group can be converted into the corresponding carbamoyl group by converting carboxy group into acyl halide and then reacting it with an amine, by reacting carboxy group with an amine in the presence of a condensing agent, or by reacting the ester with an amine.
(3) Hydrolysis of ester.
   The ester can be converted into the corresponding carboxy by hydrolysis of ester in an alkali (sodium hydroxide, potassium hydroxide, etc.) or an acid (hydrochloric acid, sulfuric acid, etc.).
(4) Conversion of carbamoyl into nitrile.
   The carbamoyl can be converted into the corresponding nitrile by reacting carbamoyl with phosphorous oxychloride or trifluoroacetic anhydride.
(5) N-Alkylation or N-phenylation.
   The amino group can be converted into the corresponding mono- or di-alkylated amino group or phenylated amino group by reacting amino group with an alkyl halide or a phenyl halide.
(6) N-Sulfonylation.
   The amino group can be converted into the corresponding alkylsulfonylamino group or phenylsulfonylamino group by reacting amino group with an alkylsulfonyl halide or a phenylsulfonyl halide. The amino group can be converted into the corresponding mono- or di-alkylated amino by reductive amination.
(7) Conversion of amine into ureido.
   The amino group can be converted into an alkyl ureido by reacting amino group with alkyl isocyanate. The amino group can be converted into ureido by reacting amino group with carbamoyl halide or by reacting the isocyanate, which is converted from the amino, with an amine.
(8) Conversion of aromatic nitro compound into aromatic amine.
   The aromatic nitro compound can be converted into the corresponding aromatic amine by conventionally reducing it with a reducing agent such as a metal reducing agent (e.g., sodium borohydride, lithium borohydride, lithium aluminum hydride), metals (Fe, Zn, Sn, SnCl₂, Ti), or by catalytic reduction of it under transition metal catalyst (e.g., palladium-carbon, Pt, Raney-nickel). In case of catalytic reduction, ammonium formate, hydrazine and so on can be used as hydrogen source.
   Furthermore, the compound of the present invention or the intermediate thereof prepared by the above methods is purified by the conventional method such as column chromatography, or recrystallization, etc. As the solvent for recrystallization, an alcohol solvent such as methanol, ethanol or 2-propanol, an ether solvent such as diethyl ether, an ester solvent such as ethyl acetate, an aromatic solvent such as toluene, a ketone solvent such as acetone, a hydrocarbon solvent such as hexane, water and so on, or a mixture thereof are illustrated. Furthermore, the compound of the present invention can be converted into its pharmaceutically acceptable salt by the conventional method and thereafter, can be subjected to recrystallization.

### EFFECT OF INVENTION

Since the compound of the present invention or its pharmaceutically acceptable salt has an activity for controlling the function of CCR4, or TARC/CCL17 and/or MDC/CCL22, it is useful as a prophylactic or treatment agent for allergic diseases, inflammatory diseases, autoimmune diseases and cancer diseases such as asthma (e.g., bronchial asthma), allergic rhinitis, allergic conjunctivitis, pollen allergy, dermatitis (atopic dermatitis, contact dermatitis, etc.), psoriasis, rheumatoid arthritis, systemic lupus erythematosus, multiple sclerosis, insulin dependent diabetes mellitus (IDDM), rejection on organ transplantation, inflammatory bowel disease (ulcerative colitis, Crohn's disease), interstitial crystitis, glomerulonephritis, sepsis, pain, adult T cell leukemia (ATL), malignant tumor, pulmonary fibrosis, eosinophilic pneumonia, pulmonary eosinophilic granuloma, cutaneous T cell lymphoma, ankylosing spondylitis, coronary artery disease, pemphigoid, Hodgkin's disease, etc.

The compound of the present invention or its pharmaceutically acceptable salt can be formulated in a medicament consisting of a therapeutically effective amount of said compound and a pharmaceutically acceptable carrier(s). The pharmaceutically acceptable carrier(s) are a diluent, a binder (syrup, gum arabic, gelatin, solbit, tragacanth gum, polyvinyl pyrrolidone, etc.), an excipient (lactose, sucrose, corn starch, potassium phosphate, solbit, glycine, etc.), a lubricant (magnesium stearate, talc, polyethylene glycol, silica, etc.), a disintegrant (potato starch), a humectant (sodium lauryl sulfate), and so on.

The compound of the present invention or its pharmaceutically acceptable salt can be orally or parenterally administered in an appropriate preparation form. The preparation suitable for oral application includes, for example solid preparations such as tablets, granules, capsules, powders, etc., solutions, suspensions, emulsions and so on. The preparation suitable for parenteral administration includes suppositories, injections or solutions for infusion containing distilled water for injection, physiological saline or an aqueous sucrose solution, preparations for inhalation and so on.

The dose of the compound of the present invention or its pharmaceutically acceptable salt varies depending on application route, age, body weight or condition of the patient, but usually, about 0.003 to 100mg/kg/day, preferably about 0.01 to 30mg/kg/day, and especially preferably about 0.05 to 10mg/kg/day.

### THE BEST MODE FOR CARRYING OUT THE INVENTION

The present invention is explained by examples and reference examples below, but the invention should not be limited by them.

### Example

### Example 1

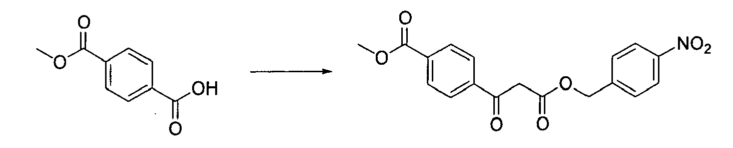

(1) To a solution of monomethyl terephthalate (10g) in THF (185ml) was added under ice cooling N,N'-carbonyldiimidazole (9.9g), and the mixture was stirred overnight at room temperature. To the reaction mixture was added under ice cooling magnesium mono (p-nitrobenzyl)malonate (30.6g) and the mixture was stirred for 4 hours at 50∼60°C. To the reaction mixture was added ethyl acetate (500ml). The mixture was washed with hydrochloric acid, water, saturated sodium bicarbonate solution and saturated brine, and dried over sodium sulfate. After removal of the solvent, the residue was solidified by adding diisopropyl ether and hexane. The solid was filtered, washed with hexane/ethyl acetate (4/1) and dried to give methyl 4-[2-(4-nitro-benzyloxycarbonyl)-acetyl]-benzoate (17.2g) as a colorless powder.
   APCI-MS (m/e): 358 (M+H)+ 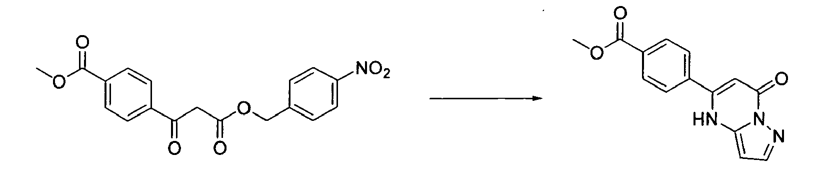
(2) A solution of methyl 4-[2-(4-nitro-benzyloxycarbonyl)-acetyl]-benzoate (17.2g) and 3-aminopyrazole (3.8g) in acetic acid (82ml) was refluxed with stirring for 2.5 hours. After being allowed to stand overnight at room temperature, the reaction mixture was again refluxed with stirring for 2.5 hours. The reaction mixture was concentrated in vacuo, and thereto was added ethyl acetate (200ml). The insoluble materials were filtered and dried in vacuo to give methyl 4-(7-oxo-4,7-dihydro-pyrazolo[1,5-a]pyrimidin-5-yl)-benzoate (12.3g) as a pale brown powder.
   APCI-MS (m/e): 270 (M+H)+ 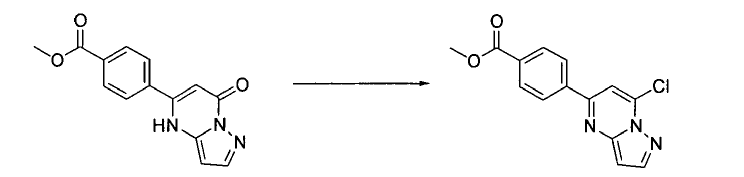
(3) To methyl 4-(7-oxo-4,7-dihydro-pyrazolo[1,5-a]pyrimidin-5-yl)-benzoate (7.1g) were added diethylaniline (8.5ml) and phosphorous oxychloride (20.6g), and the mixture was stirred at 80°C for 3 hours. The reaction mixture was allowed to cool and thereto was added diethyl ether to collect the insoluble materials. The insoluble materials were dissolved in chloroform, washed with saturated sodium bicarbonate solution and saturated brine, and dried over sodium sulfate. After removal of the solvent, the resulting crystals were washed with diethyl ether and dried in vacuo to give methyl 4-(7-chloropyrazolo[1,5-a]pyrimidin-5-yl)-benzoate (6.30g) as a yellow powder.
   APCI-MS (m/e): 288/290 (M+H)⁺ 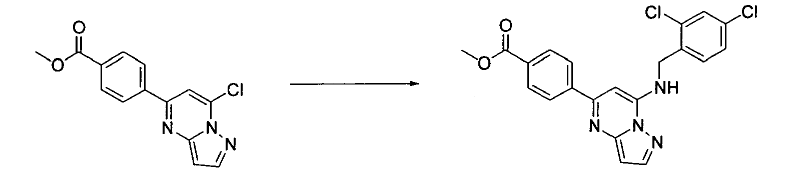
(4) To a solution of methyl 4-(7-chloropyrazolo[1,5-a]pyrimidin-5-yl)-benzoate (6.3g) in 1,4-dioxane (40ml) were added 2,4-dichlorobenzylamine (4.6g) and diisopropylethylamine (3.8ml), and the mixture was stirred at 60°C for 3 hours and then at 90°C for 4 hours. After the reaction mixture was allowed to cool, thereto was added chloroform, and the mixture was washed with saturated sodium bicarbonate solution and saturated brine. After the mixture was dried over sodium sulfate, the solvent was removed, and to the residue was added diisopropyl ether. The resulting crystals were filtered and dried in vacuo to give methyl 4-[7-(2,4-dichlorobenzylamino)-pyrazolo[1,5-a]pyrimidin-5-yl]-benzoate (9.1g) as a pale yellow powder.
   APCI-MS (m/e): 427/429 (M+H)⁺ 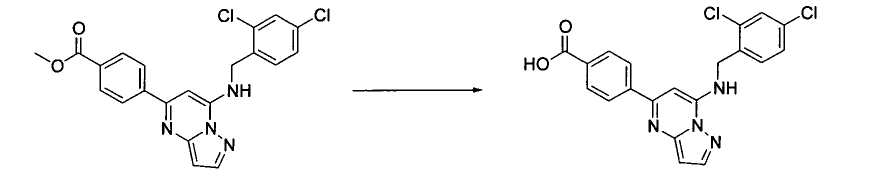
(5) To a solution of methyl 4-[7-(2,4-dichlorobenzylamino)-pyrazolo[1,5-a]pyrimidin-5-yl]-benzoate (9.1g) in methanol (250ml) was added an aqueous 2N sodium hydroxide solution (50ml) and the mixture was stirred overnight at 50∼60°C. After being allowed to cool, the reaction mixture was poured into citric acid (100g/water 1.5L), followed by stirring for 30 minutes. The insoluble materials were taken by filtration, washed with water and ethyl acetate/diisopropyl ether and dried to give 4-[7-(2,4-dichlorobenzylamino)-pyrazolo[1,5-a]pyrimidin-5-yl]-benzoic acid (8.4g) as a pale brown powder.
   APCI-MS (m/e): 413/415 (M+H)⁺ 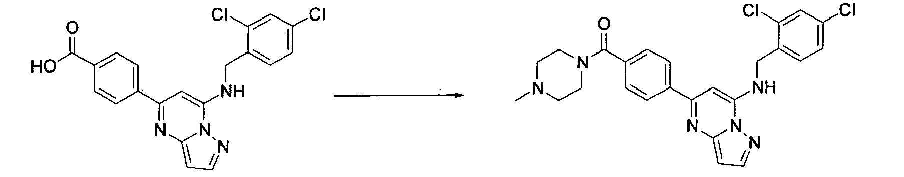
(6) To a solution of 4-[7-(2,4-dichlorobenzylamino)-pyrazolo[1,5-a]pyrimidin-5-yl]-benzoic acid (124mg) and methylpiperazine (50mg) in DMF (1.5ml) were dropped at room temperature diethylphosphorocyanidate (79mg) and triethylamine (51mg), and the mixture was stirred overnight at room temperature. Thereto was added ethyl acetate, and the mixture was washed with saturated sodium bicarbonate solution, water and saturated brine and dried over magnesium sulfate. After removal of the solvent, the residue was recrystallized from ethyl acetate/diisopropyl ether to give {4-[7-(2,4-dichlorobenzylamino)-pyrazolo[1,5-a]pyrimidin-5-yl]-phenyl}-(4-methylpiperazin-1-yl)-methanone (148mg) as pale yellow crystals.
   APCI-MS (m/e): 495/497 (M+H)⁺

### Examples 2 to 29

The following compounds were prepared by reacting and treating in the same manner as examples and reference examples as described above and below.

| | | | | |
|---|---|---|---|---|
| 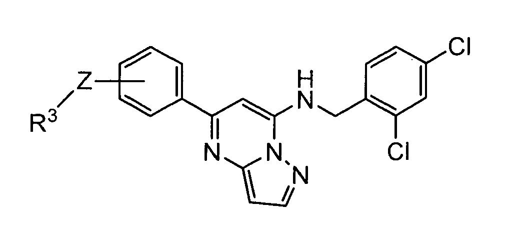 | | | | |

| Example | R³ | Z | Substituted position | MS([M+H]⁺) |
|---|---|---|---|---|
| 2 | 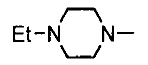 | -SO₂- | position 4 | 545/547, APCI |
| 3 | 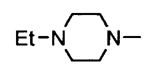 | -SO₂- | position 3 | 545/547, APCI |
| 4 | 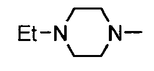 | -CO- | position 4 | 509/511, APCl |
| 5 | 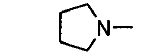 | -CONH- | position 4 | 481/483, APCI |
| 6 | 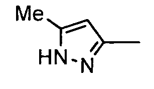 | -NHCO- | position 4 | 492/494, APCI |
| 7 | 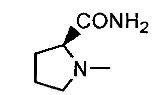 | -CO- | position 4 | 509/511, APCI |
| 8 | 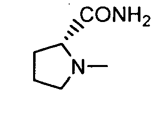 | -CO- | position 4 | 509/511, APCI |

| | | | |
|---|---|---|---|
| 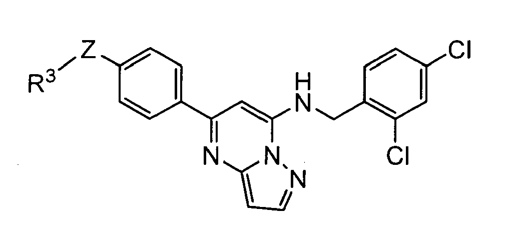 | | | |

| Example | R³ | Z | MS([M+H]⁺) |
|---|---|---|---|
| 9 | 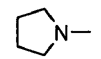 | -CO- | 466/468, APCI |
| 10 | 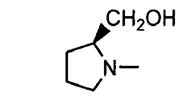 | -CO- | 496/498, APCI |
| 11 | 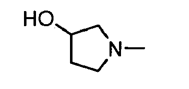 | -CO- | 482/484, APCI |
| 12 | 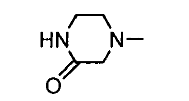 | -CO- | 495/497, APCI |
| 13 | 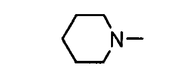 | -CO- | 480/482, APCI |
| 14 | 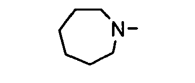 | -CO- | 494/496, APCI |
| 15 | Me | -N(Me)CO- | 490/492, APCI |

| | | | |
|---|---|---|---|
| 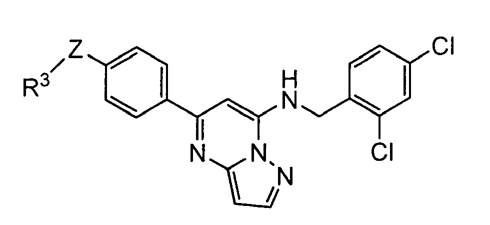 | | | |

| Example | R³ | Z | MS([M+H]⁺) |
|---|---|---|---|
| 16 | Et | -N(Et)CO- | 468/470, APCI |
| 17 | 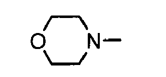 | -CO- | 482/484, APCI |
| 18 | Me | -NHCO- | 426/428, APCI |
| 19 | iPr | -NHCO- | 454/456, APCI |
| 20 | 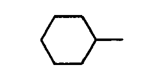 | -NHCO- | 494/496, APCI |
| 21 | 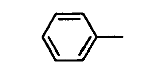 | -NHCO- | 488/490, APCI |
| 22 | 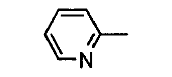 | -NHCO- | 489/491, APCI |
| 23 | 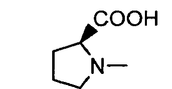 | -CO- | 510/512, APCI |
| 24 | 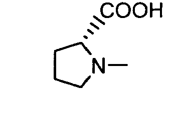 | -CO- | 510/512, APCI |

| | | | |
|---|---|---|---|
| 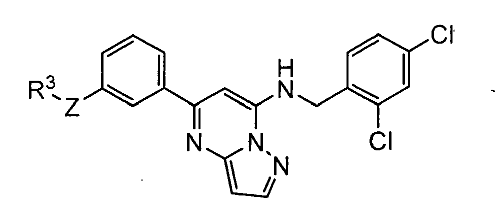 | | | |

| Example | R³ | Z | MS([M+H]⁺) |
|---|---|---|---|
| 25 | 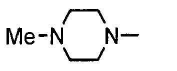 | -CO- | 495/497, APCI |
| 26 | 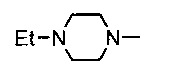 | -CO- | 509/511, APCI |
| 27 | 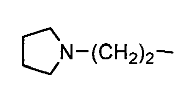 | -NHCO- | 509/511, APCI |
| 28 | 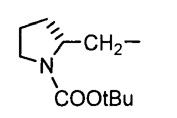 | -NHCO- | 595/597, APCI |
| 29 | 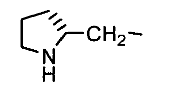 | -NHCO- | 495/497, APCI |

### Example 30

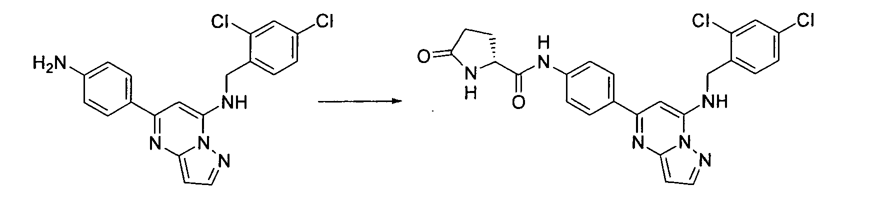

To a solution of [5-(4-aminophenyl)-pyrazolo[1,5-a]pyrimidin-7-yl]-(2,4-dichlorobenzyl)amine (100mg) and (R)-pyroglutamic acid (34mg) in DMF (1ml) were added under ice cooling 1-(3-dimethylaminopropyl)-3-ethylcarbodimide hydrochloride (65mg) and 1-hydroxybenzotriazole monohydrate (51mg), and the mixture was stirred overnight at room temperature. Thereto was added chloroform and the mixture was washed with saturated sodium bicarbonate solution and saturated brine and dried over sodium sulfate. After removal of the solvent, the residue was purified by silica gel column chromatography (chloroform/methanol = 100/0→95/5) to give 5-oxopyrrolidine-2-carboxylic acid {4-[7-(2,4-dichlorobenzylamino)-pyrazolo[1,5-a]pyrimidin-5-yl]-phenyl}amide (86mg) as a pale yellow solid.
APCI-MS (m/e): 495/497 (M+H)+

### Example 31

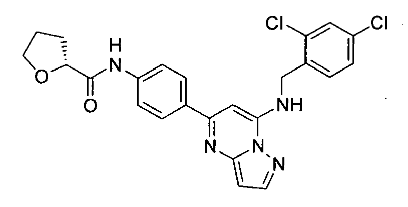

The above compound was prepared in the same manner as example 30. APCI-MS (m/e): 482/484 (M+H)⁺

### Example 32

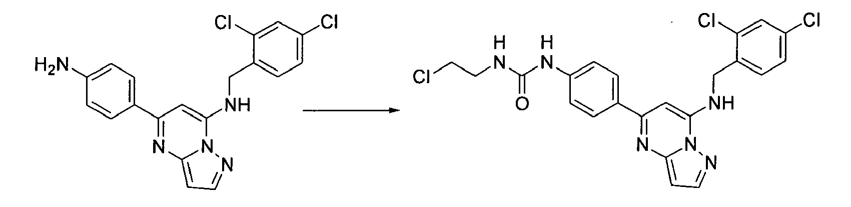

(1) To a solution of [5-(4-aminophenyl)-pyrazolo[1,5-a]pyrimidin-7-yl]-(2,4-dichlorobenzyl)amine (200mg) in DMF (1ml) was added 2-chloroethylisocyanate (88µl), and the mixture was stirred at room temperature for 4 days. Thereto was added chloroform, and the mixture was washed with saturated sodium bicarbonate solution and saturated brine and dried over sodium sulfate. After removal of the solvent, the residual solid was crushed by adding ethyl acetate and diethyl ether and dried to give 1-(2-chloroethyl)-3-{4-[7-(2,4-dichlorobenzylamino)-pyrazolo[1,5-a]pyrimidin-5-yl]-phenyl}urea (128mg) as a pale yellow solid.
   APCI-MS (m/e): 489/491 (M+H)⁺ 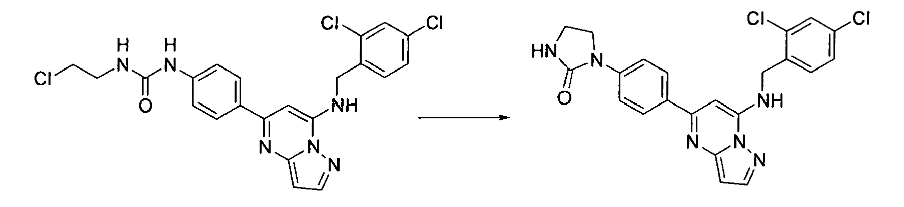
(2) To a solution of 1-(2-chloroethyl)-3-{4-[7-(2,4-dichlorobenzylamino)-pyrazolo[1,5-a]pyrimidin-5-yl]-phenyl}urea (120mg) in DMF (2ml) was added under ice cooling sodium hydride (60%, 50mg), and the mixture was stirred overnight at room temperature. Thereto was added saturated sodium bicarbonate solution, and the mixture was extracted with chloroform. The extract was washed with saturated brine and dried over sodium sulfate. After removal of the solvent, the residual solid was crushed by adding methanol and diethyl ether to give 1-{4-[7-(2,4-dichlorobenzylamino)-pyrazolo[1,5-a]pyrimidin-5-yl]-phenyl}-imidazolidin-2-one (64mg) as a pale yellow solid.
APCI-MS (m/e): 453/455 (M+H)+

### Example 33

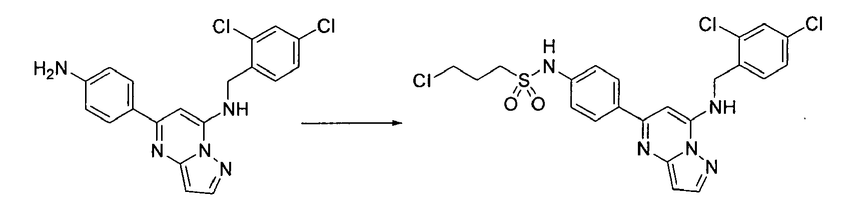

(1) To a solution of [5-(4-aminophenyl)-pyrazolo[1,5-a]pyrimidin-7-yl]-(2,4-dichlorobenzyl)amine (96mg) in THF (5ml) was added 3-chloropropylsulfonyl chloride (885mg), and the mixture was stirred at 40°C for one day. Thereto was added ethyl acetate and the mixture was washed with water and dried over magnesium sulfate. After removal of the solvent, the residue was purified by silica gel column chromatography (hexane/ethyl acetate = 100/0→60/40), crystallized from ethyl acetate/hexane and dried to give 3-chloropropane-1-sulfonic acid{4-[7-(2,4-dichlorobenzylamino)-pyrazolo[1,5-a]pyrimidin-5-yl]-phenyl}amide (23mg) as yellowish crystals.
   APCI-MS (m/e): 524/526 (M+H)⁺ 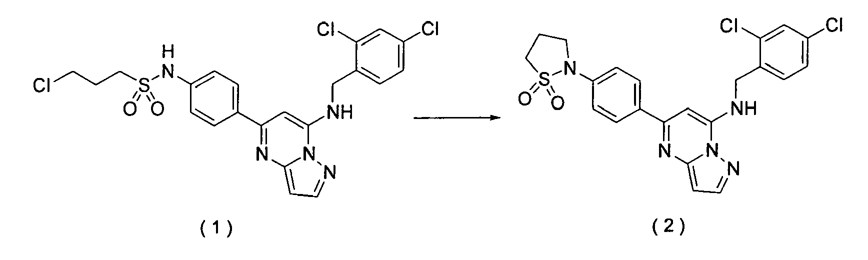
(2) The compound (2) was prepared by reacting and treating the compound (1) in the same manner as example 32 (2).
APCI-MS (m/e): 488/490 (M+H)+

### Example 34

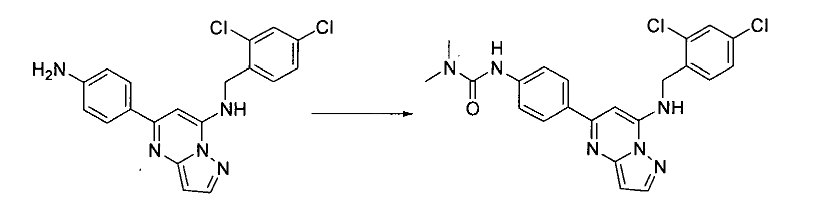

To a solution of [5-(4-aminophenyl)-pyrazolo[1,5-a]pyrimidin-7-yl]-(2,4-dichlorobenzyl) amine (115mg) in pyridine (1ml) was added dimethylaminocarbamoyl chloride (129mg), and the mixture was stirred at room temperature for one day. Thereto was added ethyl acetate, and the mixture was washed with an aqueous citric acid solution, water, saturated sodium bicarbonate solution and saturated brine, respectively and dried over magnesium sulfate. After removal of the solvent, the residue was purified by silica gel column chromatography (hexane/ethyl acetate =90/10→20/80), solidified by adding ethyl acetate/hexane and dried to give 3-{4-[7-(2,4-dichlorobenzylamino)-pyrazolo[1,5-a]pyrimidin-5-yl]-phenyl}-1,1-dimethylurea (62mg) as a pale brown solid.
APCI-MS (m/e): 455/457 (M+H)+

### Examples 35 to 43

The following compounds were prepared in the same manner as the above examples.

| | | | |
|---|---|---|---|
| 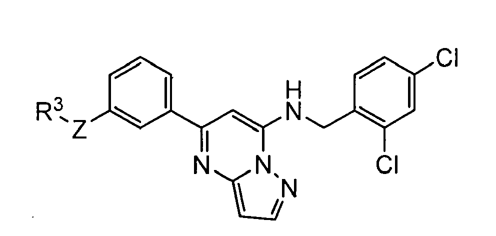 | | | |

| Example | R³ | Z | MS([M+H]⁺) |
|---|---|---|---|
| 35 | 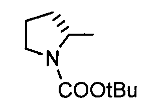 | -CONH- | 581/583, APCI |
| 36 (2 HCl) | 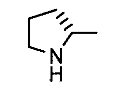 | -CONH- | 481/483, APCI |
| 37 | 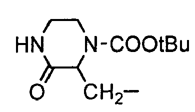 | -CONH- | 624/626, APCI |
| 38 | 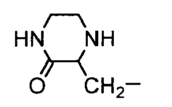 | -CONH- | 524/526, APCI |
| 39 | 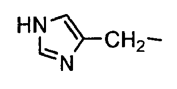 | -CONH- | 492/494, APCI |
| 40 | 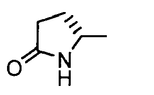 | -CONH- | 495/497, APCI |
| 41 | 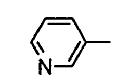 | -NHCONH- | 504/506, APCI |
| 42 | 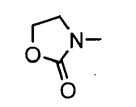 | single bond | 454/456, APCI |
| 43 | 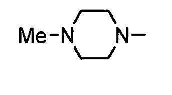 | -CONH- | 510/512, APCI |

### Example 44

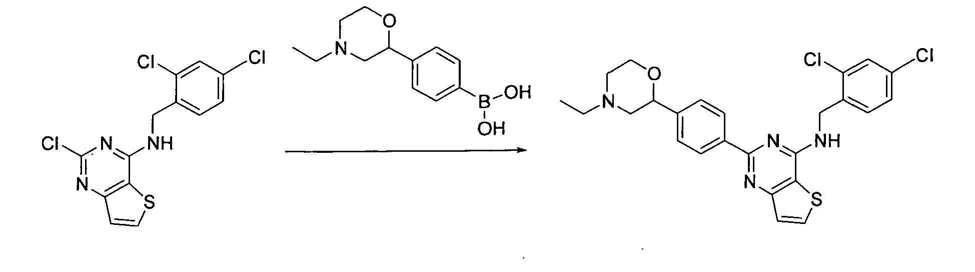

(2-Chlorothieno[3,2-d]pyrimidin-4-yl)-(2,4-dichlorobenzyl)amine (150mg) and 4-(4-ethylmorpholin-2-yl)boronic acid (135mg) were dissolved in a mixture of dimethoxyethane (7ml) and ethanol (1ml), and to the solution were added under nitrogen atmosphere tetrakistriphenylphosphine palladium (203mg) and 1M aqueous sodium carbonate solution (1ml). The mixture was stirred overnight at 80°C. After being allowed to cool, the reaction mixture was made weakly acidic with 2N hydrochloric acid and then the solution was made basic with potassium carbonate. The solution was extracted three times with ethyl acetate and the organic layers were combined and dried over magnesium sulfate. After removal of the solvent, the residue was purified twice by silica gel column chromatography (Si column: hexane/ethyl acetate =70/30→0/100, NH column:hexane/ethyl acetate =100/0→60/40) to give (2,4-dichlorobenzyl)-{2-[4-(4-ethylmorpholin-2-yl)-phenyl]-thieno[3,2-d]pyrimidin-4-yl}amine (94mg) as a white solid.
APCI-MS (m/e): 499/501 (M+H)+

### Example 45

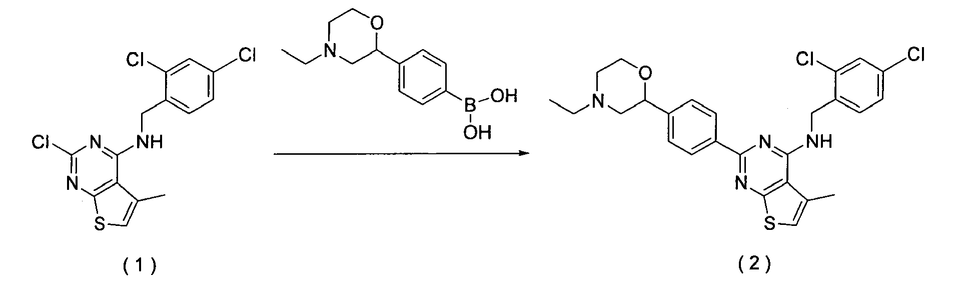

Compound (2) was prepared by reacting and treating compound (1) in the same manner as example 44. APCI-MS (m/e): 513/515 (M+H)⁺

### Example 46

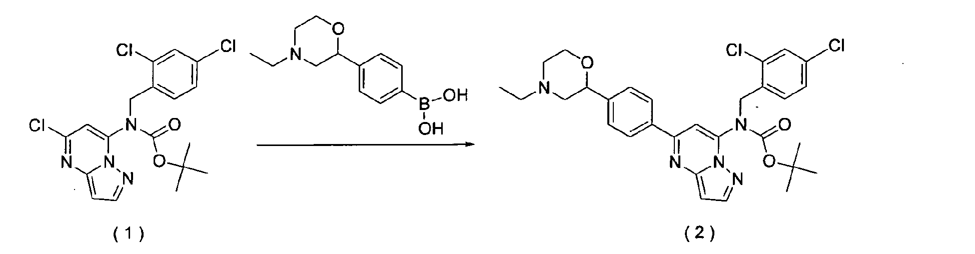

(1) Compound (2) was prepared by reacting and treating compound (1) in the same manner as example 44.
   APCI-MS (m/e): 582/584 (M+H)+ 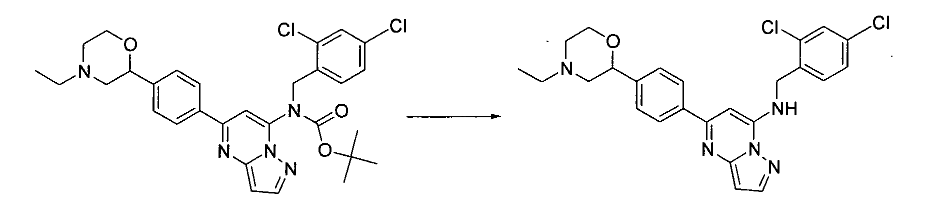
(2) tert-Butyl (2,4-dichlorobenzyl)-{5-[4-(4-ethylmorpholin-2-yl)-phenyl]-pyrazolo[1,5-a]pyrimidin-7-yl}carbamate (122mg) was dissolved in methylene chloride (2ml), and to the solution was added under ice cooling trifluoroacetic acid (1ml), followed by stirring for 2 hours. Additional trifluoroacetic acid (1ml) was added and the mixture was stirred for 3 hours. Thereto was added saturated sodium bicarbonate solution and the mixture was extracted three times with chloroform. The organic layers were combined and dried over magnesium sulfate. After removal of the solvent, the residue was purified with silica gel column chromatography (hexane/ethyl acetate 100/0→50/50) to give (2,4-dichlorobenzyl)-{5-[4-(4-ethylmorpholin-2-yl)-phenyl]pyrazolo[1,5-a]pyrimidin-7-yl}-amine (77mg) as a pale yellow oil.
APCI-MS (m/e): 482/484 (M+H)⁺

### Example 47

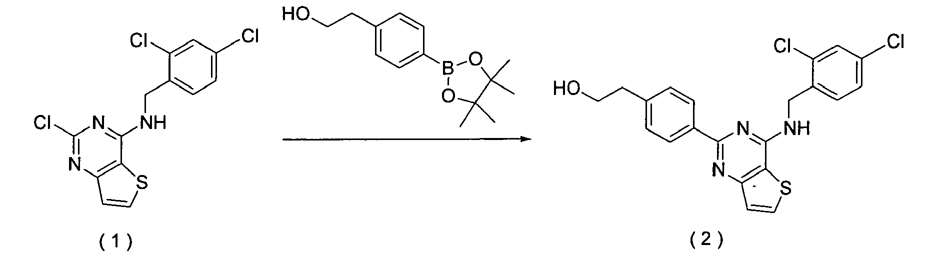

(1) Compound (2) was prepared by reacting and treating compound (1) in the same manner as example 44.
   APCI-MS (m/e): 430/432 (M+H)+ 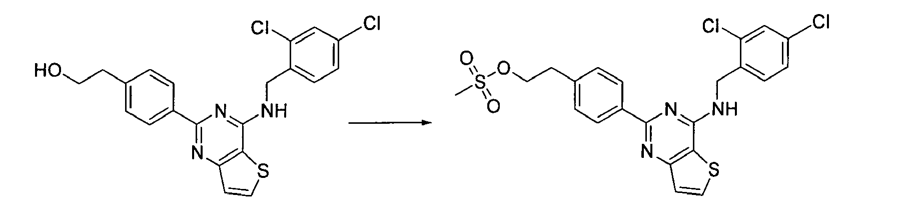
(2) To a solution of 2-{4-[4-(2,4-dichlorobenzylamino)-thieno[3,2-d]pyrimidin-2-yl]phenyl}-ethanol (76mg) in THF (1ml) were added at 0°C methanesulfonyl chloride (21µM) and triethylamine (49µl), and the mixture was stirred at room temperature for 1 hour. Additional methanesulfonyl chloride (10.5µM) and triethylamine (30µl) were added and the mixture was stirred at room temperature for 30 minutes. Thereto was added ethyl acetate, and the mixture was washed with water and saturated brine, and dried over magnesium sulfate. After removal of the solvent, the residual solid was crushed by adding ethyl acetate and dried to give methanesulfonic acid 2-{4-[4-(2,4-dichlorobenzylamino)-thieno[3,2-d]pyrimidin-2-yl]phenyl}-ethyl ester (65mg) as a colorless solid.
   APCI-MS (m/e): 508/510 (M+H)+ 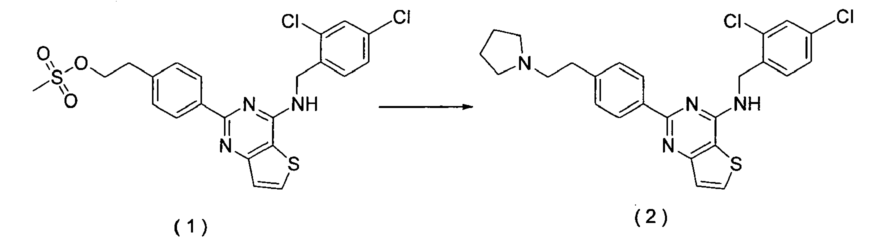
(3) Compound (2) was prepared by reacting and treating Compound (1) in the same manner as reference example 23 (1).
APCI-MS (m/e): 483/485 (M+H)+

### Examples 48 to 62

The following compounds were prepared in the same manners as the examples and reference examples as described above and below.

| | | | | |
|---|---|---|---|---|
| 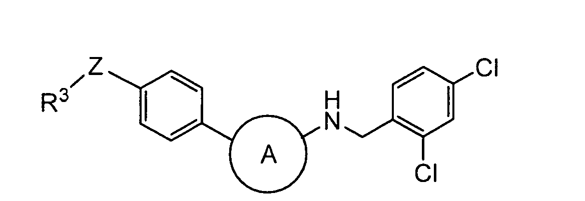 | | | | |

| Example | R³ | Z | Ring A | MS([M+H]⁺) |
|---|---|---|---|---|
| 48 | 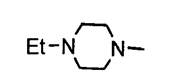 | -SO₂- | 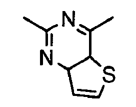 | 562/564, APCI |
| 49 | 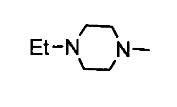 | -SO₂- | 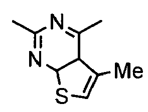 | 576/578, APCI |
| 50 | 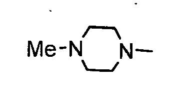 | -CO- | 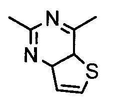 | 512/514, APCI |
| 51 | 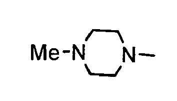 | -CO- | 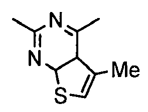 | 526/528, APCI |
| 52 | 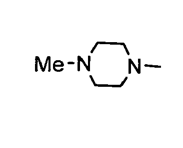 | -CO- | 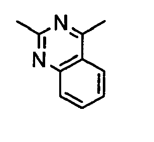 | 506/508, APCI |

| | | | | | | |
|---|---|---|---|---|---|---|
| 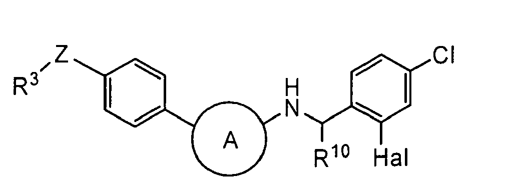 | | | | | | |

| Example | R³ | Z | Ring A | R¹⁰ | Hal | MS([M⁺H]⁺) |
|---|---|---|---|---|---|---|
| 53 | 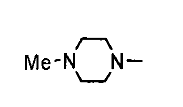 | -CO- | 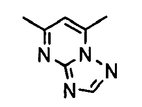 | H | F | 480/482, APCI |
| 54 | 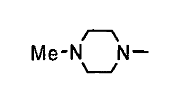 | -NHCO⁻ | 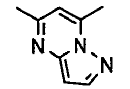 | H | Cl | 510/512, APCI |
| 55 | 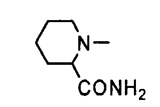 | -CO⁻ | 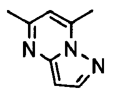 | H | Cl | 523/525, APCI |
| 56 | 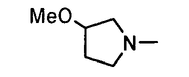 | -CO⁻ | 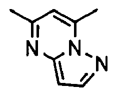 | H | Cl | 496/498, APCI |
| 57 | 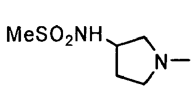 | -CO⁻ | 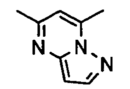 | H | Cl | 559/561, APCI |
| 58 | 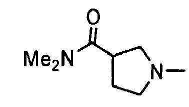 | -CO⁻ | 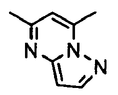 | H | Cl | 537/539, APCI |
| 59 | 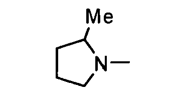 | -CO⁻ | 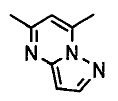 | H | Cl | 480/482, APCI |
| 60 | 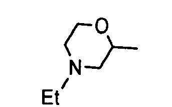 | single bond | 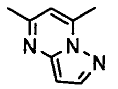 | Me (R form) | Cl | 496/498, APCI |
| 61 (2HCl) | 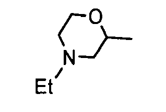 | single bond | 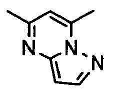 | H | F | 466/468, APCI |
| 62 | 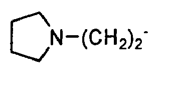 | -NH- | 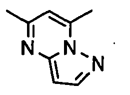 | H | Cl | 481/483, APCI |

### Example 63

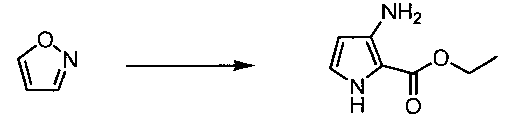

(1) To isoxazole (75g) in ethanol (300ml) was gradually added sodium ethoxide (21w% in ethanol) (369g) at 8°C or less in a period of 2 hours and the mixture was stirred for 45 minutes at the same temperature. To the reaction mixture were added acetic acid (22.5g), diethyl 2-aminomalonate hydrochloride (143g) and sodium acetate (61.4g), respectively at 4-6°C and the mixture was stirred at room temperature overnight. After concentrated in vacuo, to the residue was added chloroform and the mixture was washed with water and dried. To the residue was added ethanol (1.14L) and sodium ethoxide (21w% in ethanol) (262ml), followed by stirring overnight. After adding acetic acid (42.2g), the mixture was concentrated in vacuo. To the residue was added water and the mixture was adjusted to pH7 with aqueous saturated sodium bicarbonate. The mixture was extracted with chloroform and the extract was purified with silica gel column chromatography (hexane:ethyl acetate=2:1) to give ethyl 3-amino-1H-pyrrol-2-carboxylate (60.7g).
   APCI-MS (m/e): 155 (M+H)+ 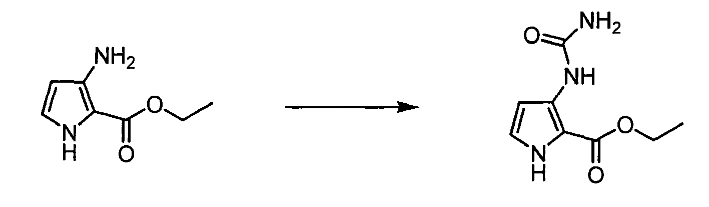
(2) Ethyl 3-amino-1H-pyrrol-2-carboxylate (82.6g) was dissolved in acetic acid (500ml) and water (50ml) and to the solution was added potassium cyanate (130.4g) in water (250ml) in a period of one hour and the mixture was stirred at room temperature for one hour. After concentrated in vacuo, thereto were added water (500ml) and ethyl acetate (200ml) and the mixture was neutralized with potassium carbonate. The resulting crystals were filtered, washed with water, ethyl acetate and dried to give ethyl 3-ureido-1H-pyrrolo-2-carboxylate (63.8g).
   APCI-MS (m/e): 198 (M+H)⁺ 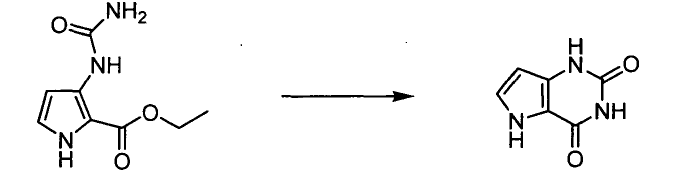
(3) To ethyl 3-ureido-1H-pyrrolo-2-carboxylate (69.4g) was added aqueous 6% sodium hydroxide solution (950ml) and the mixture was stirred under reflux for 30 minutes. After being cooled, the reaction solution was adjusted to pH6 with concentrated hydrochloric acid and after stirring, the crystals were filtered. The filtrate was washed with a small amount of water and methanol, concentrated in vacuo and subjected to azeotropic distillation with toluene to give 1,5-dihydro-pyrrolo[3,2-d]pyrimidine-2,4-dione (32.0g).
   APCI-MS (m/e): 152 (M+H)⁺ 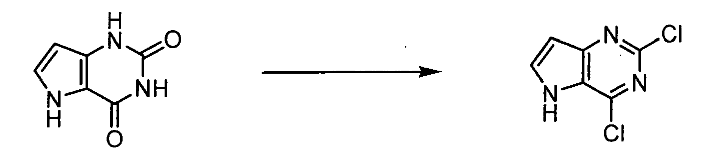
(4) To 1,5-dihydro-pyrrolo[3,2-d]pyrimidine-2,4-dione (35.0g) was added aqueous 1N sodium hydroxide solution (231ml) and after stirring for a while, the mixture was concentrated in vacuo. The residue was subjected to azeotropic distillation with toluene. To the residue was gradually added phenylphosphonic dichloride (239g) and then the temperature of the mixture was raised to 180°C, followed by stirring for 3 hours. Thereto was further gradually added phenylphosphoric dichloride (100g) and the mixture was stirred overnight. The reaction mixture was gradually poured into ice-water under stirring and the mixture was extracted with ethyl acetate. The organic layer was washed with aqueous sodium bicarbonate solution, dried and concentrated in vacuo. To the residue was added ethyl acetate/ diisopropyl ether to give 2,4-dichloro-5H-pyrrolo[3,2-d]pyrimidine as crystals (19.96g).
   APCI-MS (m/e): 188/190 (M+H)+ 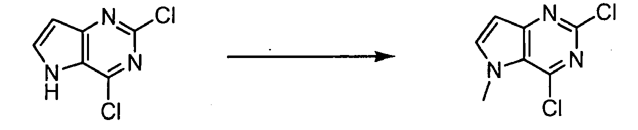
(5) To 2,4-dichloro-5H-pyrrolo[3,2-d]pyrimidine (19.9g) in acetonitrile (530ml) was gradually added 60% sodium hydride (5.08g) under ice-cooling and the mixture was stirred at room temperature for 30 minutes. To the reaction mixture was dropped methyl iodide (18.03g) at 8°C and the mixture was stirred at room temperature for overnight. After filtering insoluble materials over Celite, the filtrate was concentrated in vacuo. The residue was washed with diisopropyl ether to give 2,4-dichloro-5-methyl-5H-pyrrolo[3,2-d]pyrimidine (11.0g).
   APCI-MS (m/e): 202/204 (M+H)⁺ 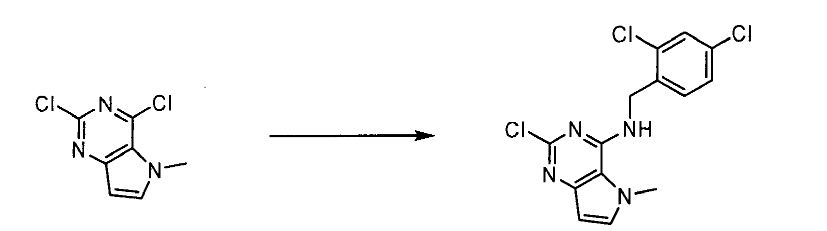
(6) By reacting and treating 2,4-dichloro-5-methyl-5H-pyrrolo[3,2-d]pyrimidine in the same manner as Example 1 (3), there was obtained (2-chloro-5-methyl-5H-pyrrolo[3,2-d]pyrimidin-4-yl)-(2,4-dichlorobenzyl)amine (20.6g).
   APCI-MS (m/e): 341/343 (M+H)+ 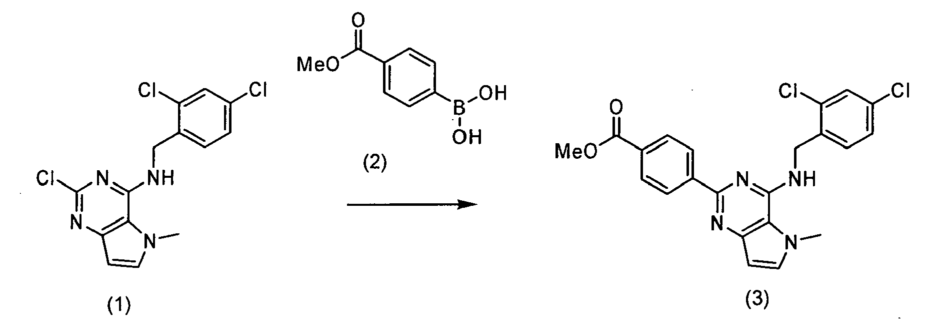
(7) By reacting and treating compound (1) and compound (2) in the same manner as example 44, there was obtained compound (3).
   APCI-MS (m/e): 441/443 (M+H)+ 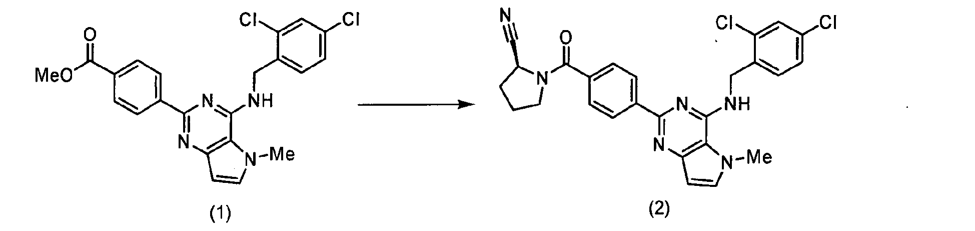
(8) By reacting and treating compound (1) in the same manner as example 1 (5), there was obtained corresponding carboxylic acid compound, and then by reacting and treating it in the same manner as example 1 (6), there was obtained compound (2).
APCI-MS (m/e): 523/525 (M+H)⁺

### Example 64

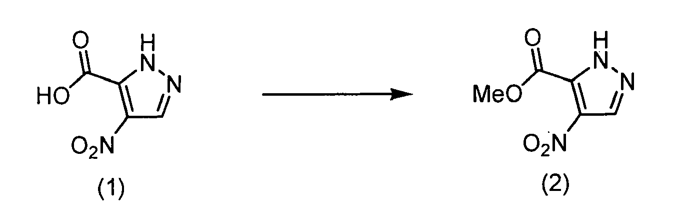

(1) To a suspension of 4-nitro-3-pyrazole (1) (51.06g) in methanol (515ml) was added concentrated sulfuric acid (10.1ml) and the mixture was refluxed under heating overnight. After being cooled, thereto was added aqueous saturated sodium bicarbonate solution to make the solution alkaline. After removal of methanol in vacuo, the residue was extracted with ethyl acetate 6 times. The combined organic layer was dried and the solvent was removed by distillation. The residue was crushed with ethyl acetate/hexane and dried to give compound (2) (47.61g) as a colorless powder.
   ESI-MS (m/e): 170 (M-H)⁻ 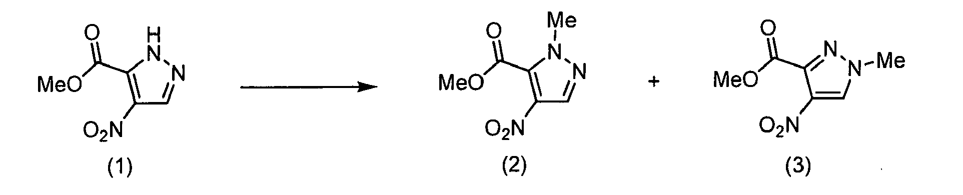
(2) To dried methanol (830ml) was carefully added sodium metal (6.71g), and the mixture was ice-cooled after sodium pieces are completely dissolved. Thereto were added compound (1) (43.61g) and then methyl iodide (43.64ml), and the mixture was stirred at 60∼70°C for 4 hours. After concentrated in vacuo, to the residue was chloroform and the solution was washed with aqueous saturated sodium bicarbonate solution and saturated brine, and dried. After removal of the solvent, the residue was purified with silica gel column chromatography (hexane:ethyl acetate=4:1→1:1) to give compound (2) (17.0g) as a yellow oil, and compound (3) (26.8g) as a colorless powder.
   Compound (2):
   APCI-MS (m/e): 186 (M+H)⁺
   ¹HNMR(500MHz/DMSO-d₆ (ppm):3.95(s,3H),4.00(s,3H),8.37(s,1H)
   Compound (3):
   APCI-MS (m/e): 186 (M+H)+
   ¹HNMR(500MHz/DMSO-d₆)δ(ppm):3.94(s,3H),4.00(s,3H),8.95(s,1H) 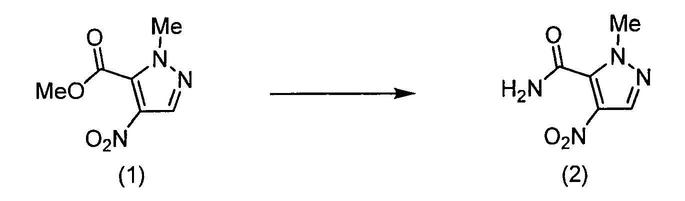
(3) To aqueous 28% ammonia (285ml) was added compound (1) (17.0g) and the mixture was stirred at 60°C for 6 hours. The reaction mixture was allowed to be cooled, and concentrated in vacuo to give compound (2) (16.0g) as a colorless solid.
   ESI-MS (m/e): 169 (M-H)⁻ 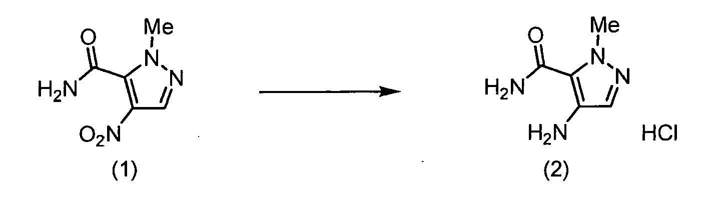
(4) To a suspension of compound (1) (19.8g) in methanol (350ml) was added 10% palladium-C and the mixture was stirred overnight at room temperature under hydrogen gas (50psi) in a moderate-pressured reduction apparatus. To 4N hydrochloric acid/ethyl acetate (120ml) was poured the reaction mixture and the insoluble materials were filtered off over Celite. The filtrate was concentrated in vacuo. The residue was crushed with adding ethyl acetate and dried to give compound (2) (19.95g) as a pale orange powder.
   APCI-MS (m/e): 141 (M+H)⁺ 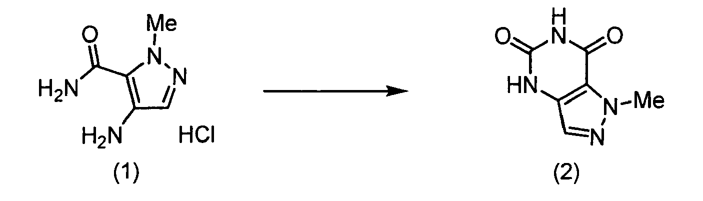
(5) Compound (1) (17.3g) and urea (32.9g) were added in a reaction vessel and stirred at 200°C for 1 hour. After being cooled, thereto was added warmed aqueous 1N sodium hydroxide solution (330ml). After the starting material was dissolved, the mixture was ice-cooled and was adjusted to pH 4 with acetic acid (55ml). The resulting crystals were filtered, washed with water three times and diethyl ether once, and dried to give compound (2) (12.6g) as a colorless powder. APCI-MS (m/e): 189 (M+Na)⁺ 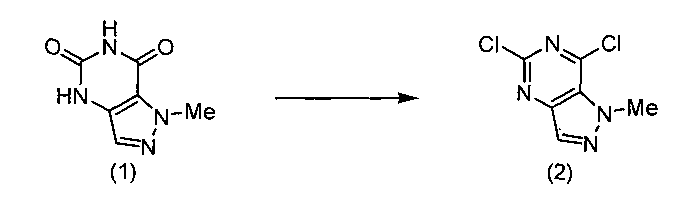
(6) To a suspension of compound (1) (14.0g) and oxyphosphorus chloride (84ml) was gradually added diethylaniline (16.9ml) and the mixture was stirred under reflux for 22 hours. The reaction mixture was allowed to be cooled, concentrated in vacuo and subjected to azeotropic distillation with toluene twice. The residue was poured into ice-water and thereto was added chloroform, followed by stirring. The mixture was separated by a separating funnel and chloroform layer was washed with saturated brine, dried and the solvent was removed by distillation. The residue was purified with silica gel column chromatography (hexane:ethyl acetate=10:1→2:1) to give compound (2) (15.3g) as a yellow solid.
   APCI-MS (m/e): 203/205 (M+H)⁺ 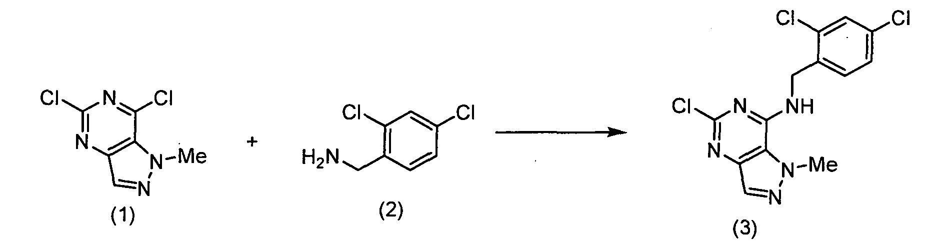
(7) To compound (1) (14.3g) in 1,4-dioxane (260ml) was added 2,4-dichlorobenzylamine (2) (18.6g) and triethylamine (17.8g), respectively under ice-cooling and the mixture was stirred for 4 hours at room temperature. To the reaction solution was added ethyl acetate and the mixture was washed with aqueous saturated sodium bicarbonate solution and saturated brine, and dried. After removal of the solvent, the residue was purified with silica gel column chromatography (hexane:ethyl acetate=2:1→1:2). The product was crushed with diethyl ether and dried to give compound (2) (20.0g) as a colorless powder. APCI-MS (m/e): 342/344 (M+H)+ 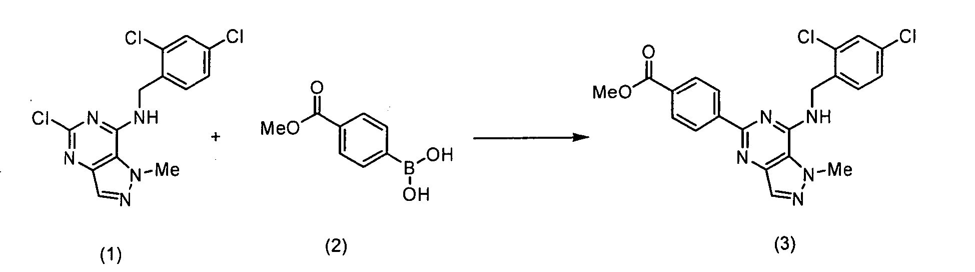
(8) By reacting and treating compound (1) and compound (2) in the same manner as example 44, there was obtained compound (3).
   APCI-MS (m/e): 442/444 (M+H)⁺ 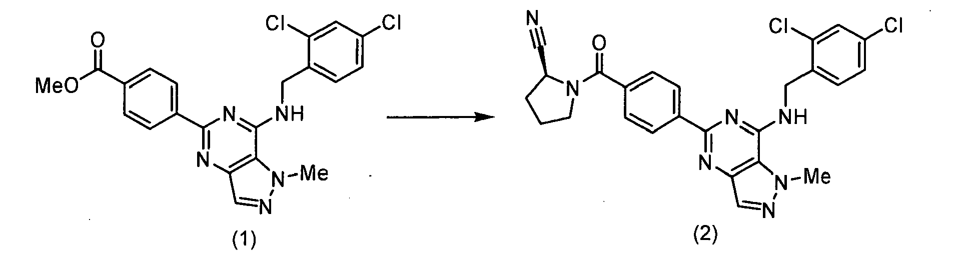
(9) By reacting and treating compound (1) in the same manner as example 1 (5), there was obtained corresponding carboxylic acid compound, and then by reacting and treating it with 2(S)-cyanopyrrolidine in the same manner as Example 1 (6), there was obtained compound (2).
APCI-MS (m/e): 524/526 (M+H)⁺

### Examples 65 to 72

By reacting and treating in the same manner as the above or below mentioned examples and reference examples, the following compounds were obtained.

| | | | | |
|---|---|---|---|---|
| 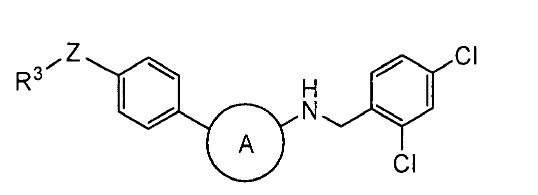 | | | | |

| Example | R³ | Z | Ring A | MS([M+H]⁺) |
|---|---|---|---|---|
| 65 | 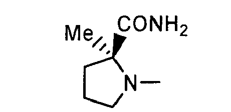 | -CO⁻ | 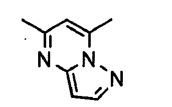 | 523/525, APCI |
| 66 | 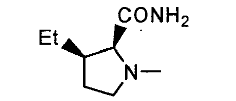 | -CO⁻ | 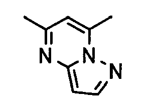 | 537/539, APCI |
| 67 | 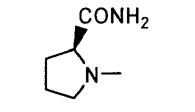 | -CO⁻ | 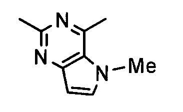 | 523/525, APCI |
| 68 | 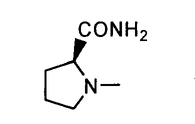 | -CO⁻ | 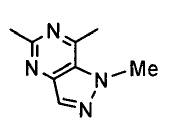 | 524/526, APCI |
| 69 | 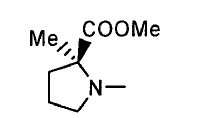 | -CO⁻ | 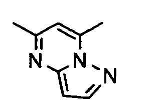 | 538/540, APCI |
| 70 | 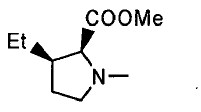 | -CO⁻ | 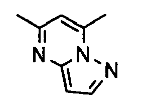 | 552/554, APCI |
| 71 | 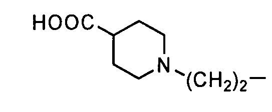 | single bond | 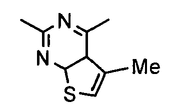 | 555/557, APCI |
| 72 | 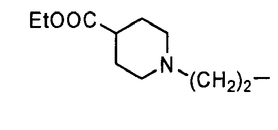 | single bond | 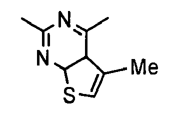 | 583/585, APCI |

### Example 73

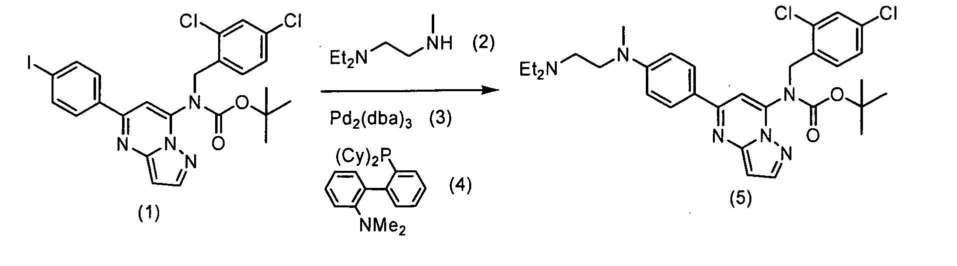

(1) To a mixture of reagent (3) (7.8mg), compound (4) (6.7mg) and sodium tert-butoxide (15mg) were dropped, compound (1) (50mg) and compound (2) (41µl) in 1,4-dioxane (3ml) under a nitrogen atmosphere and the mixture was stirred at 80°C overnight. After being cooled, to the reaction mixture was added ethyl acetate and the mixture was washed with water and saturated brine, and dried. After removal of the solvent, the residue was purified with NH-silica gel column chromatography (hexane:ethyl acetate=83:17→65:35) to give compound (5) (31mg) as a yellow amorphous.
   APCI-MS (m/e): 597/599 (M+H)⁺ 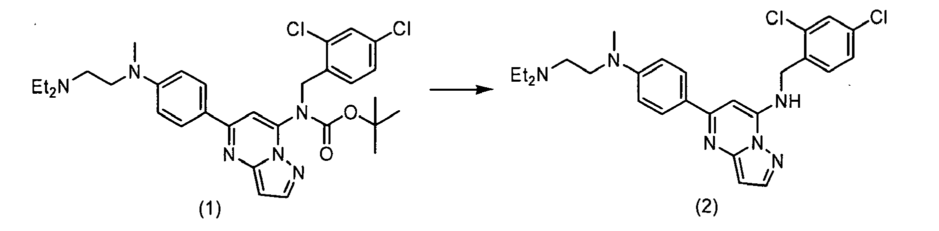
(2) By reacting and treating compound (1) in the same manner as example 46 there was obtained compound (2).
APCI-MS (m/e): 497/499 (M+H)⁺

### Example 74

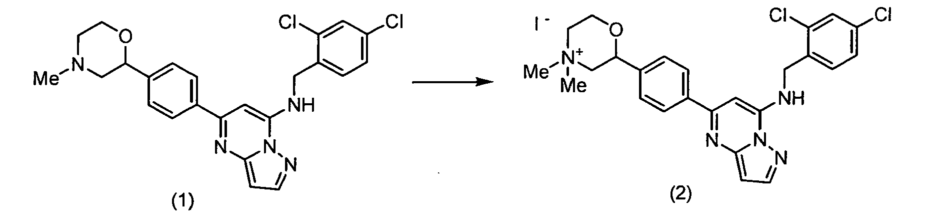

Compound (1) (100mg) was dissolved in a mixture of THF (2ml) and methylene chloride (2ml) and thereto was added methyl iodide (71mg), followed by stirring at room temperature for 2 days. The reaction mixture was concentrated in vacuo to give a yellow solid. By recrystallization from methanol/ethyl acetate, there was obtained compound (2) (92mg) as colorless crystals.
ESI-MS (m/e): 482/484 (M-H)⁻

### Examples 75 to 82

By reacting and treating in the same manner as the above or below mentioned examples and reference examples, the following compounds were obtained.

| | | | | |
|---|---|---|---|---|
| 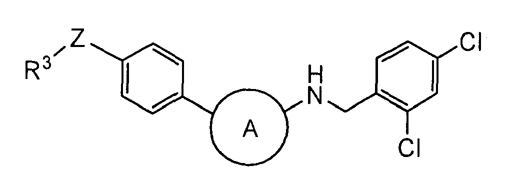 | | | | |

| Example | R³ | Z | Ring A | MS([M+H]⁺) |
|---|---|---|---|---|
| 75 | Et₂N(CH₂)₂- | -NH- | 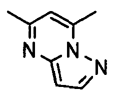 | 483/485, APCI |
| 76 | 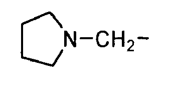 | -CONH⁻ | 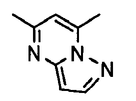 | 495/497, APCI |
| 77 | Et₂NCH₂- | -CONH⁻ | 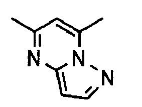 | 497/499, APCI |
| 78 | 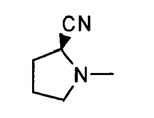 | -CO⁻ | 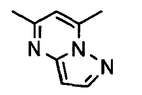 | 492/494, APCI |
| 79 (2HCl) | 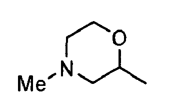 | single bond | 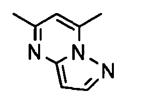 | 468/470, APCI |
| 80 | 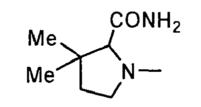 | -CO⁻ | 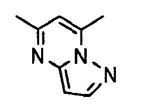 | 537/539, APCI |
| 81 | 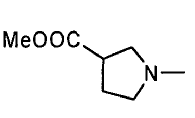 | -CO⁻ | 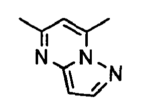 | 524/526, APCI |
| 82 | 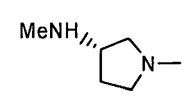 | -CO⁻ | 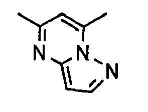 | 495/497, APCI |

### Example 83

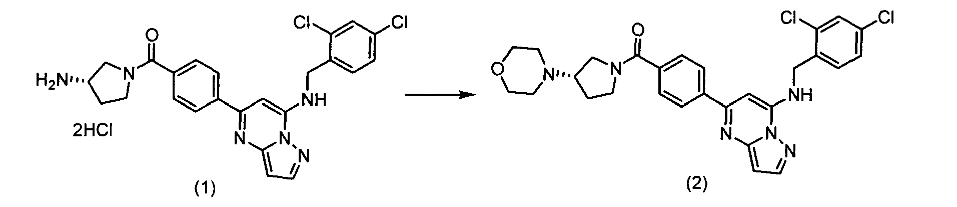

To compound (1) (94mg) in ethanol (0.8ml) and water (0.2ml) were added bis(2-chloroethyl) ether (28µl), sodium iodide (31mg) and potassium carbonate (83mg), and thereon was carried out microwave irradiation for 2 hours at 150°C using a microwave apparatus. After the reaction mixture was concentrated, to the residue was added chloroform and the mixture was washed with water, and dried. After removal of the solvent, the residue was purified with thin-layer silica gel plate (chloroform:methanol=9:1) to give compound (2) (36mg) as a pale green solid.
APCI-MS (m/e): 551/553 (M+H)⁺

### Example 84

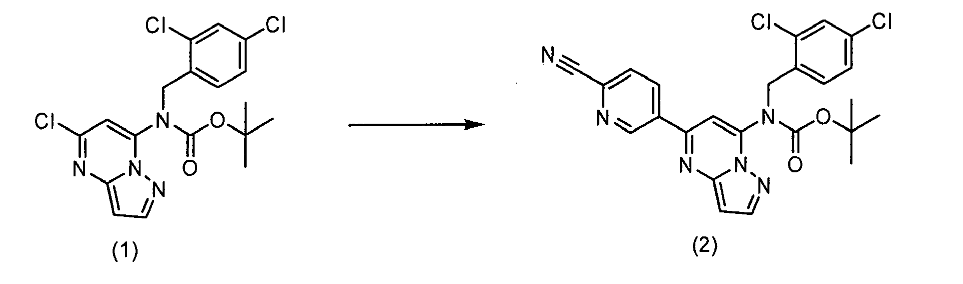

(1) By reacting and treating compound (1) in the same manner as example 44 there was obtained compound (2).
   APCI-MS (m/e): 495/497 (M+H)⁺ 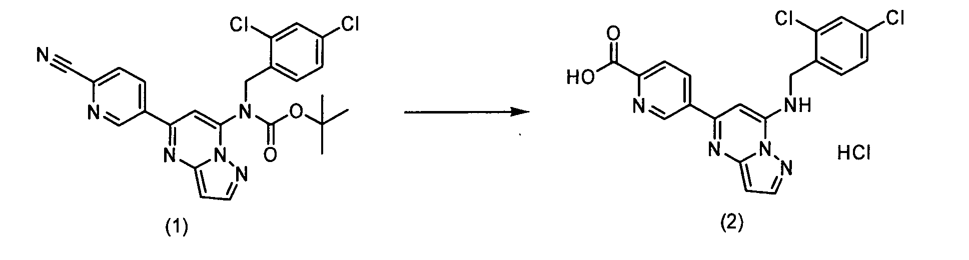
(2) To compound (1) (120mg) in 1,4-dioxane (2ml) was added concentrated hydrochloric acid (2ml) and the mixture was stirred at 60°C overnight, and further at 100°C overnight. The reaction mixture was concentrated in vacuo, the residue was crushed with diisopropyl ether/ethanol to give compound (2) (95mg) as an orange powder.
   APCI-MS (m/e): 414/416 (M-H)⁻ 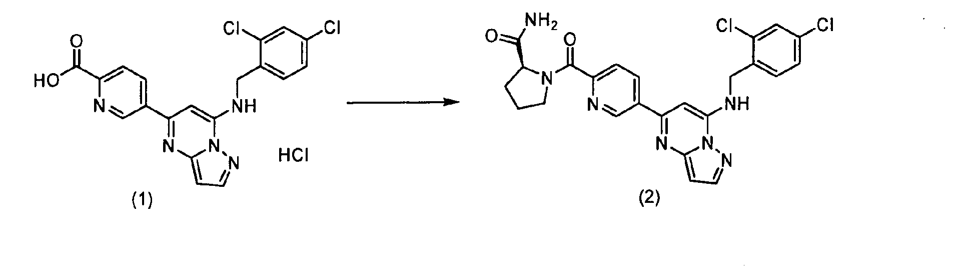
(3) To compound (1) (45mg) and (L)-prolineamide (13.7mg) in DMF (2ml) were added diethylphosphorocyanidate (24.5mg) and triethylamine (24.5mg), respectively at room temperature and the mixture was stirred overnight. To the reaction mixture was added ethyl acetate and the mixture was washed with aqueous saturated sodium bicarbonate solution and saturated brine, and dried. After removal of the solvent, the residue was purified with NH-silica gel column chromatography (chloroform:methanol=100:0→95:5) to give compound (2) (38.2mg) as a yellow powder.
APCI-MS (m/e): 510/512 (M+H)⁺

### Examples 85 to 128

By reacting and treating in the same manner as the above or below mentioned examples and reference examples, the following compounds were obtained.

| | | | | | |
|---|---|---|---|---|---|
| 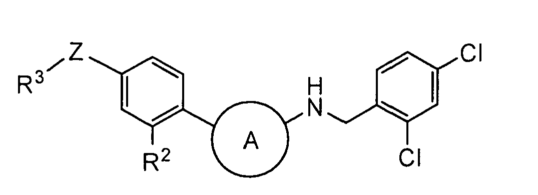 | | | | | |

| Example | R³ | Z | R² | Ring A | MS([M+H]⁺) |
|---|---|---|---|---|---|
| 85 | 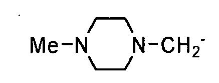 | single bond | H | 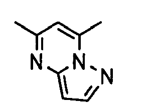 | 481/483, APCI |
| 86 | 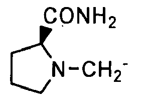 | single bond | H | 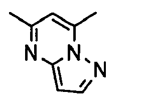 | 495/497, APCI |
| 87 | 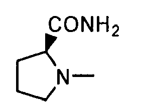 | -CO⁻ | H | 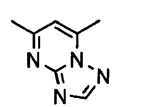 | 510/512, APCI |
| 88 | 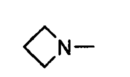 | -CO⁻ | H | 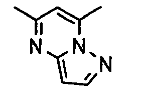 | 452/454, APCI |
| 89 | 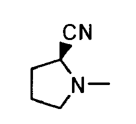 | -CO⁻ | H | 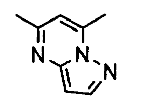 | 491/493, APCI |
| 90 | 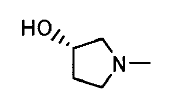 | -CO⁻ | H | 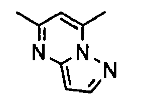 | 482/484, APCI |
| 91 | 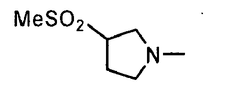 | -CO⁻ | H | 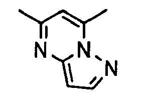 | 544/546, APCI |
| 92 | 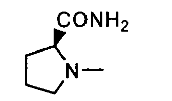 | -CO⁻ | F | 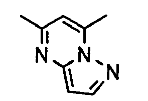 | 527/529, APCI |
| 93 | 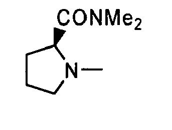 | -CO⁻ | H | 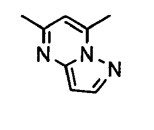 | 537/539, APCI |

| | | | | | | |
|---|---|---|---|---|---|---|
| 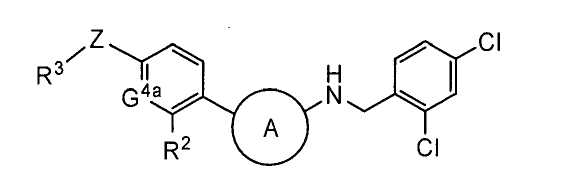 | | | | | | |

| Example | R³ | Z | G^{4a} | R² | Ring A | MS([M+H]⁺) |
|---|---|---|---|---|---|---|
| 94 | 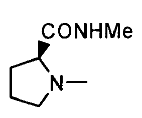 | -CO⁻ | CH | H | 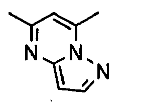 | 523/525, APCI |
| 95 (HCl) | 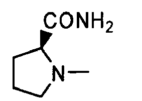 | -CO⁻ | C-F | H | 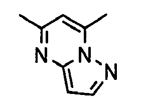 | 527/529, APCI |
| 96 | 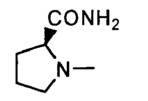 | -CO⁻ | CH | H | 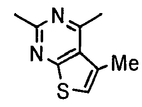 | 540/542, APCI |
| 97 | 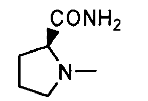 | -CO⁻ | CH | Me | 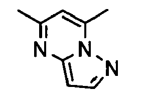 | 523/525, APCI |
| 98 | 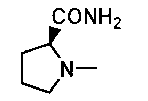 | -CO⁻ | CH | H | 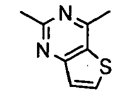 | 526/528, APCI |
| 99 | 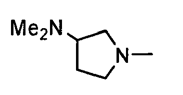 | -CO⁻ | CH | H | 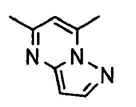 | 509/511, APCI |
| 100 | 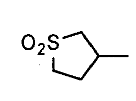 | -NHCO⁻ | CH | H | 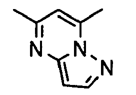 | 530/532, APCI |
| 101 (2HCl) | 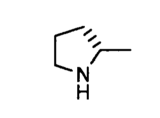 | -CONH⁻ | CH | H | 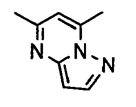 | 481/483, APCI |
| 102 | 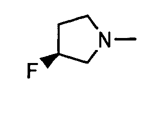 | -CO⁻ | CH | H | 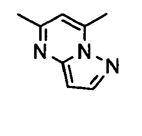 | 484/486, APCI |

| | | | | | | |
|---|---|---|---|---|---|---|
| 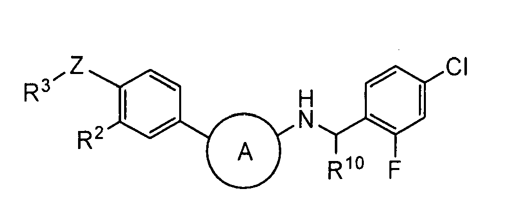 | | | | | | |

| Example | R³ | Z | R² | R¹⁰ | Ring A | MS([M+H]⁺) |
|---|---|---|---|---|---|---|
| 103 | 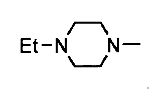 | single bond | Me | H | 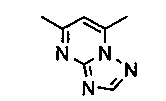 | 480/482, APCI |
| 104 | 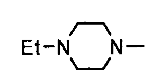 | single bond | Me | H | 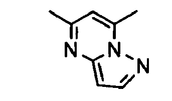 | 479/481, APCI |
| 105 | 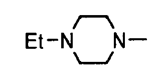 | single bond | H | H | 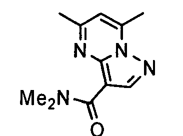 | 536/538, APCI |
| 106 | 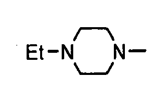 | single bond | H | H | 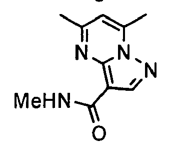 | 522/524, APCI |
| 107 | 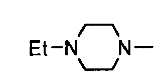 | single bond | H | H | 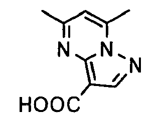 | 509/511, APCI |
| 108 (2HCl) | 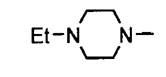 | single bond | H | H | 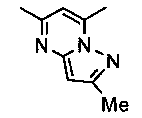 | 479/481, APCI |
| 109 (2HCl) | 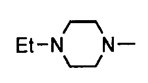 | single bond | H | Me(R form) | 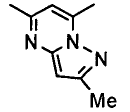 | 493/495, APCI |
| 110 | 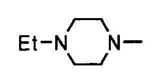 | single bond | H | H | 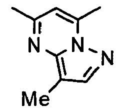 | 479/481, APCI |
| 111 (2HCl) | 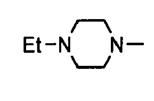 | single bond | H | Me(R form) | 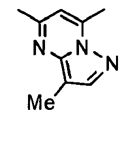 | 493/495, APCI |

| | | | | | | |
|---|---|---|---|---|---|---|
| 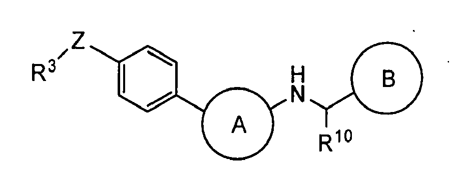 | | | | | | |

| Example | R³ | Z | R¹⁰ | Ring A | Ring B | MS([M+H]⁺) |
|---|---|---|---|---|---|---|
| 112 | 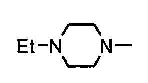 | single bond | Me(R form) | 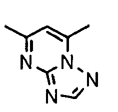 | 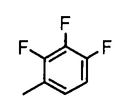 | 482, APCI |
| 113 | 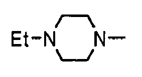 | single bond | H | 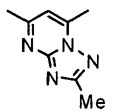 | 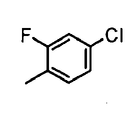 | 480/482, APCI |
| 114 (2HCl) | 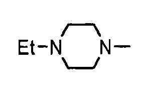 | single bond | Me(R form) | 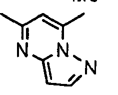 | 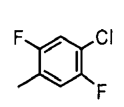 | 497/499, APCI |
| 115 (2HCl 3H₂O) | 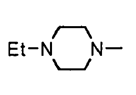 | single bond | Me(R form) | 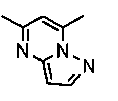 | 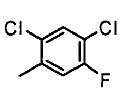 | 513/515, APCI |
| 116 | 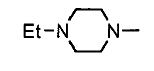 | single bond | H | 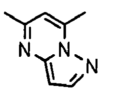 | 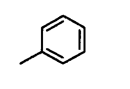 | 413, APCI |
| 117 | 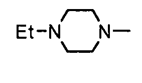 | single bond | Me(R form) | 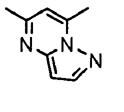 | 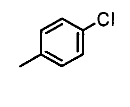 | 461/463, APCI |

| | | | | | | | | |
|---|---|---|---|---|---|---|---|---|
| 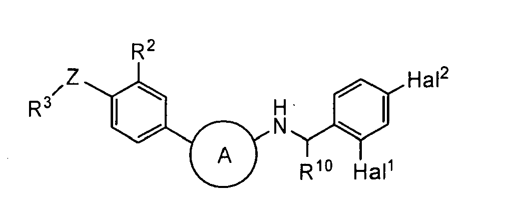 | | | | | | | | |

| Example | R³ | Z | R² | Ring A | R¹⁰ | Hal¹ | Hal² | MS([M+H]⁺) |
|---|---|---|---|---|---|---|---|---|
| 118 | 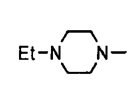 | single bond | -SO₂Me | 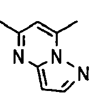 | H | F | Cl | 543/545, APCI |
| 119 (2HCl) | 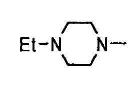 | single bond | H | 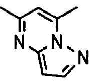 | Me(S form) | F | Cl | 479/481, APCI |
| 120 (2HCl) | 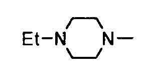 | single bond | H | 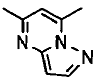 | Me(R form) | F | Cl | 479/481, APCI |
| 121 | 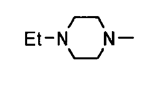 | single bond | -NHAc | 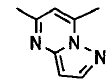 | H | F | Cl | 522/524, APCI |
| 122 | 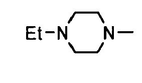 | single bond | -NHSO₂Me | 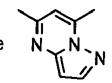 | H | F | Cl | 558/560, APCI |
| 123 | 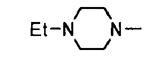 | single bond | -NH₂ | 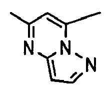 | H | F | Cl | 480/482, APCI |
| 124 | 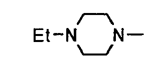 | single bond | -NO₂ | 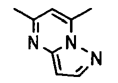 | H | F | Cl | 510/512, APCI |
| 125 (2HCl) | 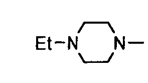 | single bond | H | 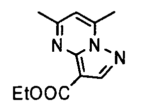 | H | Cl | Cl | 553/555, APCI |
| 126 (3HCl) | 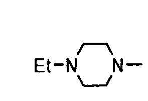 | single bond | H | 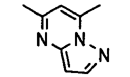 | Me(R form) | F | F | 463, APCI |
| 127 | 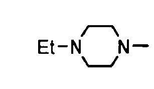 | single bond | H | 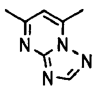 | H | F | Cl | 466/468, APCI |
| 128 | 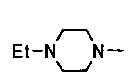 | single bond | H | 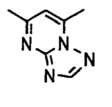 | H | Cl | Cl | 482/484, APCI |

### Example 129

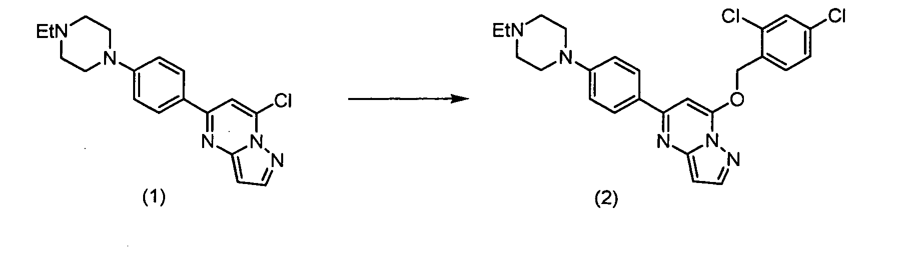

To 2,4-dichlorobenzylalcohol (39mg) in THF (1ml) was added 60% sodium hydride (14mg) at 0°C and the mixture was stirred for one hour. Thereto was added compound (1) (50mg) in THF (1ml) and the mixture was stirred at room temperature overnight. After adding aqueous sodium bicarbonate solution, the mixture was extracted with ethyl acetate, and dried. After removal of the solvent, the residue was purified with silica gel column chromatography (chloroform:methanol=100:0→92:8) to give compound (2) (32mg) as a colorless solid.
APCI-MS (m/e): 482/484 (M+H)⁺

### Examples 130 to 153

By reacting and treating in the same manner as the above or below mentioned examples and reference examples, the following compounds were obtained.

| | | | | | | | |
|---|---|---|---|---|---|---|---|
| 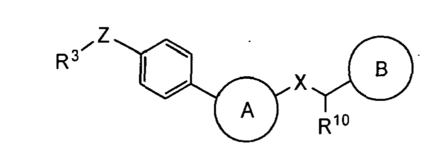 | | | | | | | |

| Example | R³ | Z | Ring A | X | R¹⁰ | Ring B | MS([M+H]⁺) |
|---|---|---|---|---|---|---|---|
| 130 | 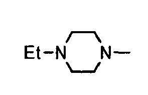 | single bond | 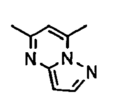 | NH | H | 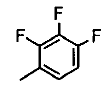 | 467, APCI |
| 131 | 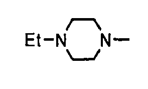 | single bond | 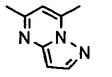 | NH | H | 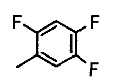 | 467, APCI |
| 132 | 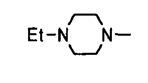 | single bond | 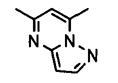 | NH | H | 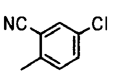 | 472/474, APCI |
| 133 | 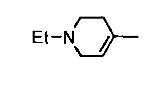 | single bond | 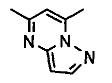 | NH | H | 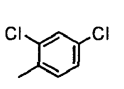 | 478/480, APCI |
| 134 | 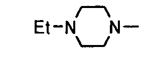 | single bond | 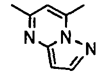 | O | H | 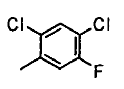 | 500/502, APCI |
| 135 | 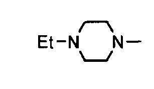 | single bond | 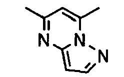 | NH | H | 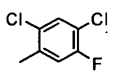 | 499/501, APCI |
| 136 | 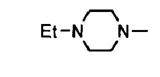 | single bond | 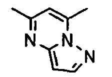 | NH | H | 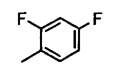 | 449, APCI |
| 137 (2HCl) | 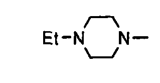 | single bond | 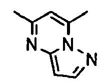 | NH | H | 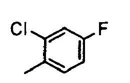 | 465/467, APCI |
| 138 (2HCl) | 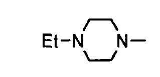 | single bond | 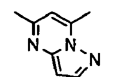 | NH | Me(R form) | 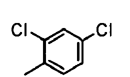 | 495/497, APCI |
| 139 | 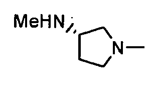 | single bond | 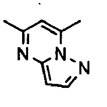 | NH | H | 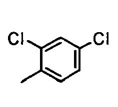 | 467/469, APCI |

| | | | | | | |
|---|---|---|---|---|---|---|
| 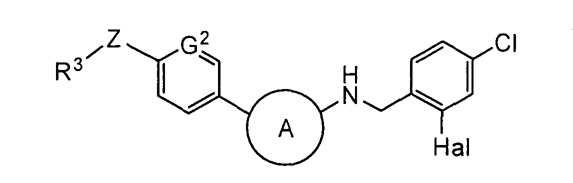 | | | | | | |

| Example | R³ | Z | G² | Ring A | Hal | MS([M+H]⁺) |
|---|---|---|---|---|---|---|
| 140 (HCl) | 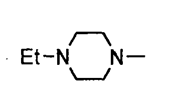 | single bond | N | 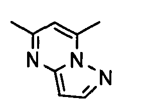 | F | 466/468, APCI |
| 141 (HCl) | 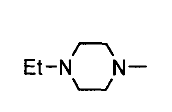 | single bond | CH | 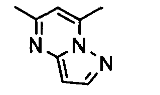 | F | 465/467, APCI |
| 142 | 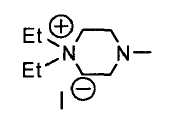 | single bond | CH | 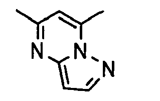 | Cl | 509/511, ESI |
| 143 | 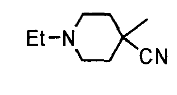 | single bond | CH | 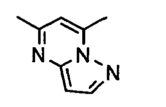 | Cl | 505/507, APCI |
| 144 | 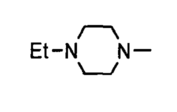 | single bond | CH | 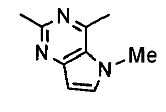 | Cl | 495/497, APCI |

| | | | | | | |
|---|---|---|---|---|---|---|
| 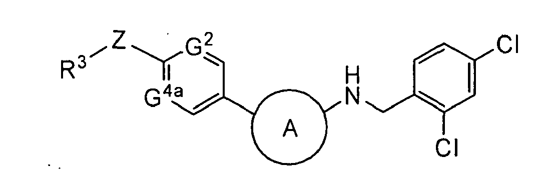 | | | | | | |

| Example | R³ | Z | G² | G^{4a} | Ring A | MS([M+H]⁺) |
|---|---|---|---|---|---|---|
| 145 | 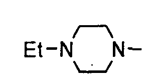 | single bond | CH | CH | 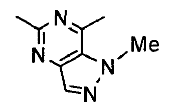 | 496/498, APCI |
| 146 | 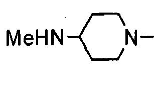 | single bond | CH | CH | 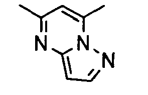 | 481/483, APCI |
| 147 | 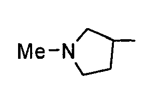 | -N(Me)⁻ | CH | CH | 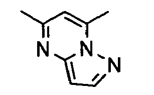 | 481/483, APCI |
| 148 | 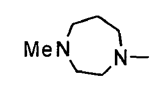 | single bond | CH | CH | 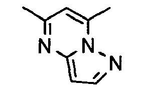 | 481/483, APCI |
| 149 | 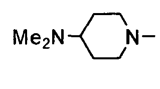 | single bond | CH | CH | 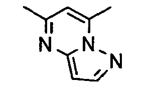 | 495/497, APCI |
| 150 | 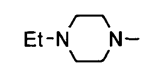 | single bond | CH | C-COOMe | 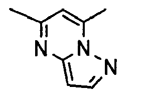 | 539/541, APCI |
| 151 | 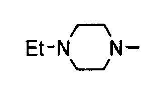 | single bond | CH | CH | 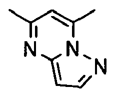 | 481/483, APCI |
| 152 (HCl) | 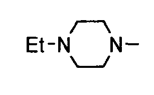 | single bond | N | CH | 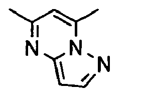 | 482/484, APCI |
| 153 | 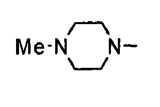 | single bond | CH | CH | 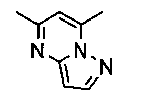 | 467/469, APCI |

### Example 154

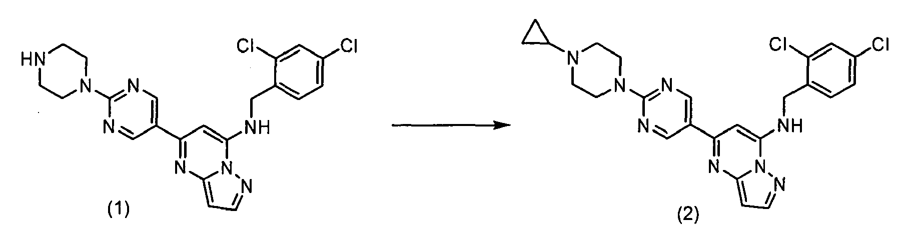

To compound (1) (70mg)obtained in reference example 30 and (1-ethoxycyclopropoxy)trimethylsilane (123µl) in methanol (2ml) were added acetic acid (90µl), sodium cyanoborohydride (38.6mg) and molecular sieve MS3A and the mixture was stirred under reflux for 4 hours. After being cooled, thereto was added ethyl acetate, and the mixture was washed with aqueous saturated sodium bicarbonate solution and saturated brine, and dried. After removal of the solvent, the residue was purified with NH-silica gel column chromatography (hexane:ethyl acetate=80:20→67:33) to give compound (2) (35.6mg) as a light yellow powder.
APCI-MS (m/e): 495/497 (M+H)⁺

### Example 155

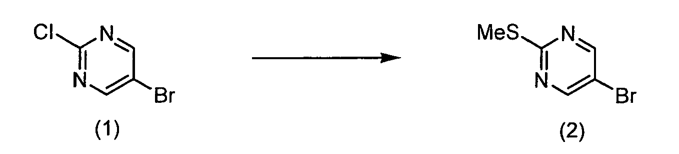

(1) To 5-bromo-2-chloropyrimidine (1) (3.87g) in ethanol (50ml) was added sodium methanethiolate (1.68g) and the mixture was stirred at room temperature for 4 hours and further at 60°C for 2.5 hours. After the reaction mixture was concentrated in vacuo, to the residue was added ethyl acetate and the mixture was washed with water and saturated brine, and dried. After removal of the solvent, the residue was purified with silica gel column chromatography (hexane:ethyl acetate=100:0→91:9) to give compound (2) (3.02g) as a colorless solid.
   APCI-MS (m/e): 205/207 (M+H)⁺ 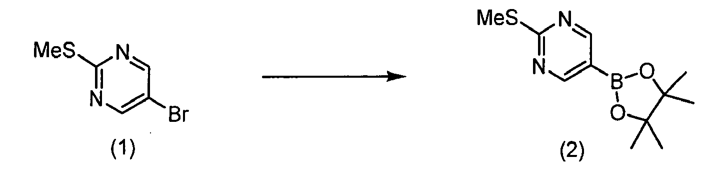
(2) By reacting and treating compound (1) in the same manner as reference example 22 (2) there was obtained compound (2).
   APCI-MS (m/e): 253 (M+H)+ 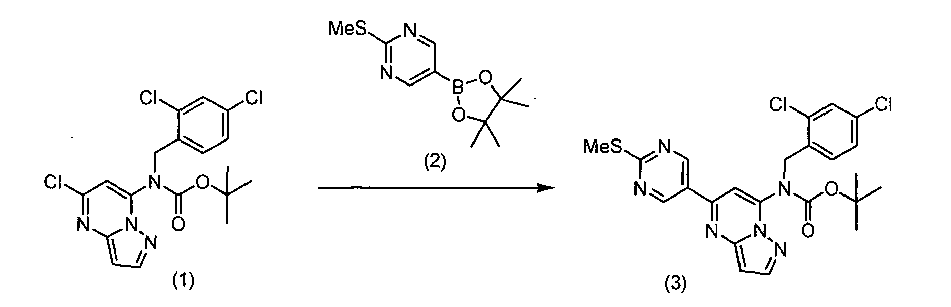
(3) By reacting and treating compound (1) and compound (2) in the same manner as example 44 there was obtained compound (3).
   APCI-MS (m/e): 517/519 (M+H)⁺ 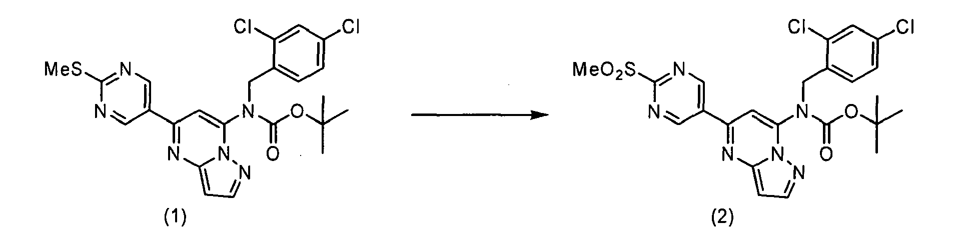
(4) To compound (1) (2.07g) in methylene chloride (40ml) was added m-chloroperbenzoic acid (25% water) (2.30g) and the mixture was stirred at room temperature for 2.5 hours. Thereto was added additional m-chloroperbenzoic acid (25% water) (0.45g) and the mixture was stirred at room temperature for one hour. To the reaction mixture was added methylene chloride and the mixture was washed with aqueous saturated sodium bicarbonate solution and saturated brine, and dried. After removal of the solvent, the residue was purified with silica gel column chromatography (hexane:ethyl acetate=75:25→ 60:40) to give compound (2) (1.23g) as a yellow powder.
   APCI-MS (m/e): 549/551 (M+H)⁺ 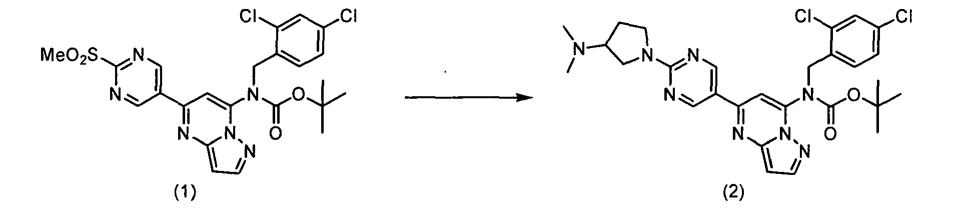
(5) To compound (1) (45mg) in 1,4-dioxane (3ml) was added 3-dimethylaminopyrrolidine (18.7mg), and the mixture was stirred at 80°C overnight. To the reaction mixture was added ethyl acetate and the mixture was washed with water and saturated brine, and dried. After removal of the solvent, the residue was purified with silica gel column chromatography (chloroform:methanol=96:4→92:8) to give compound (2) (42mg) as a brown amorphous.
   APCI-MS (m/e): 583/585 (M+H)⁺ 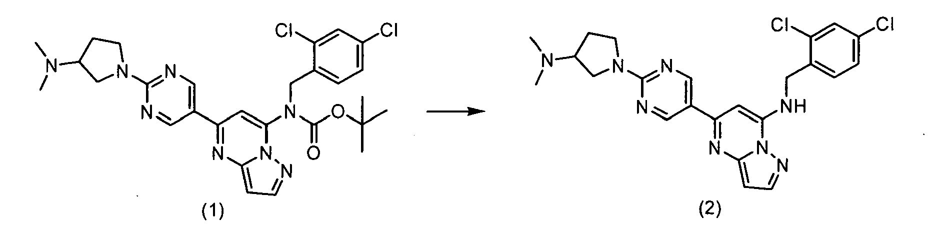
(6) By reacting and treating compound (1) in the same manner as example 46 (2) there was obtained compound (2).
APCI-MS (m/e): 483/485 (M+H)⁺

### Examples 156 to 171

By reacting and treating in the same manner as the above or below mentioned examples and reference examples, the following compounds were obtained.

| | | | | | | | |
|---|---|---|---|---|---|---|---|
| 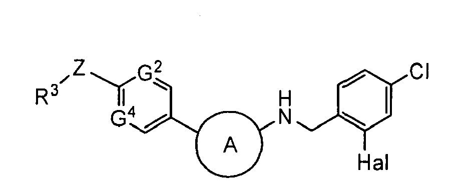 | | | | | | | |

| Example | R³ | Z | G² | G⁴ | Ring A | Hal | MS([M+H]⁺) |
|---|---|---|---|---|---|---|---|
| 156 | 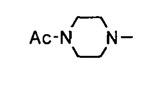 | single bond | N | N | 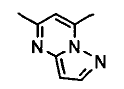 | Cl | 497/499, APCI |
| 157 | 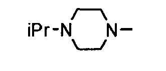 | single bond | N | N | 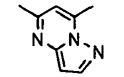 | Cl | 497/499, APCI |
| 158 | 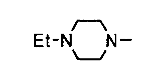 | single bond | N | N | 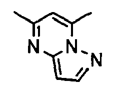 | Cl | 483/485, APCI |
| 159 | 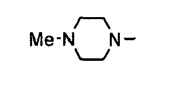 | single bond | N | N | 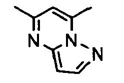 | Cl | 469/471, APCI |
| 160 | 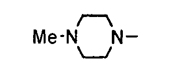 | single bond | CH | N | 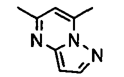 | Cl | 468/470, APCI |
| 161 | 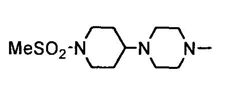 | single bond | CH | CH | 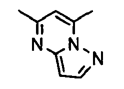 | F | 519/521, APCI |
| 162 | 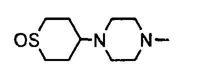 | single bond | CH | CH | 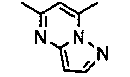 | F | 559/561, APCI |
| 163 | 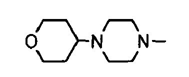 | single bond | CH | CH | 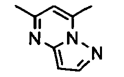 | F | 495/497, APCI |
| 164 | 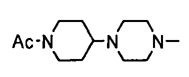 | single bond | CH | CH | 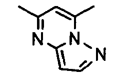 | F | 481/483, APCI |
| 165 | 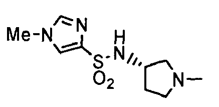 | single bond | CH | CH | 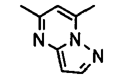 | F | 581/583, APCI |
| 166 | 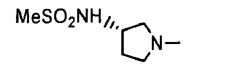 | single bond | CH | CH | 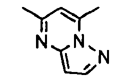 | F | 515/517, APCI |
| 167 | 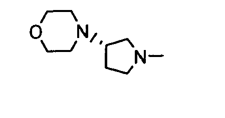 | single bond | CH | CH | 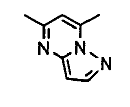 | F | 507/509, APCI |

| | | | | | | |
|---|---|---|---|---|---|---|
| 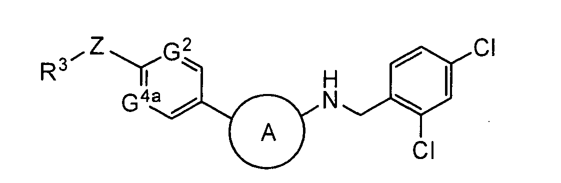 | | | | | | |

| Example | R³ | Z | G² | G^{4a} | Ring A | MS([M+H]⁺) |
|---|---|---|---|---|---|---|
| 168 | 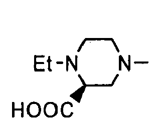 | single bond | CH | CH | 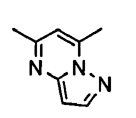 | 525/527, APCI |
| 169 (2HCl) | Et₂N(CH₂)₂- | -N(CH₂COOH)⁻ | CH | CH | 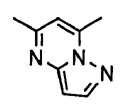 | 567/569, ESI |
| 170 (2HCl) | Et₂N(CH₂)₂- | -N[(CH₂)₂COOH]⁻ | CH | CH | 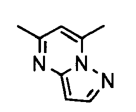 | 495/497, APCI |
| 171 | 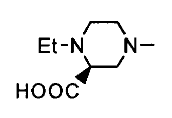 | single bond | N | N | 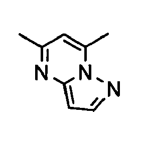 | 524/526, APCI |

### Example 172

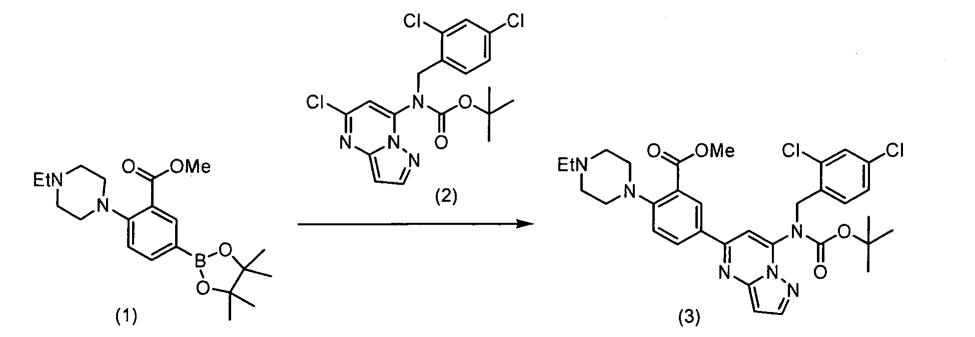

(1) By reacting and treating compound (1) and compound (2) in the same manner as example 44 there was obtained compound (3).
   APCI-MS (m/e): 639/641 (M+H)⁺ 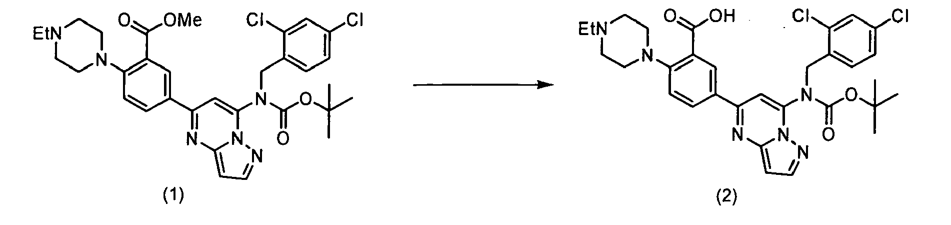
(2) By reacting and treating compound (1) in the same manner as example 1 (5) there was obtained compound (2).
   APCI-MS (m/e): 625/627 (M+H)⁺ 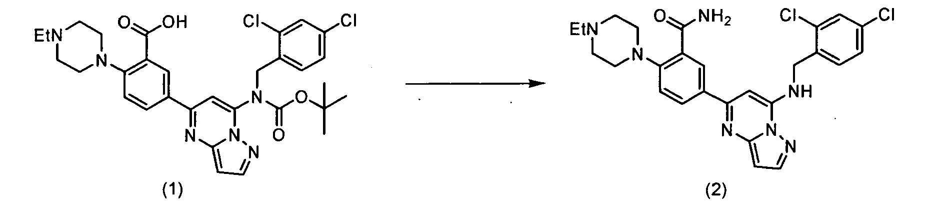
(3) To compound (1) (100mg) in THF (5ml) was added N,N'-carbonyldiimidazole (162mg) and the mixture was stirred at room temperature for 14 hours. To the reaction mixture was added 28% aqueous ammonia (5ml) and the mixture was vigorously stirred at room temperature for 3 hours. Thereto was added ethyl acetate and the mixture was washed with saturated brine and dried. After removal of the solvent, to the residue was added 4N hydrochloric acid/1,4-dioxane (10ml) and the mixture was stirred at room temperature for 5 hours. After the reaction mixture was concentrated in vacuo, to the residue was added aqueous sodium bicarbonate solution and the mixture was extracted with ethyl acetate and dried. After removal of the solvent, the residue was purified with silica gel column chromatography (chloroform:methanol=100:0→94:6) and recrysatallized from ethyl acetate/isopropyl ether to give compound (2) (36mg) as colorless crystals.
APCI-MS (m/e): 524/526 (M+H)⁺

### Example 173

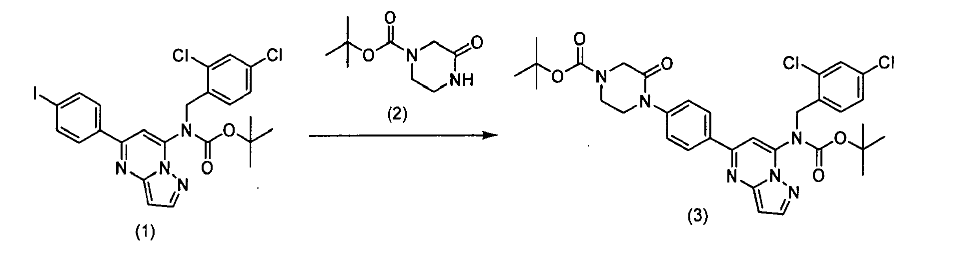

(1) Compound (1) (250mg), compound (2) (168mg), glycine (15.8mg), cupper iodide(I) and potassium phosphate powder (222mg) were added to 1,4-dioxane (0.84ml) and the mixture was stirred at 100°C overnight. After being cooled, the insoluble materials were filtered and the filtrate was concentrated in vacuo. The residue was purified with silica gel column chromatography (hexane:ethyl acetate=70:30→45:55) to give compound (3) (158mg) as a pale yellow amorphous.
   APCI-MS (m/e): 667/669 (M+H)⁺ 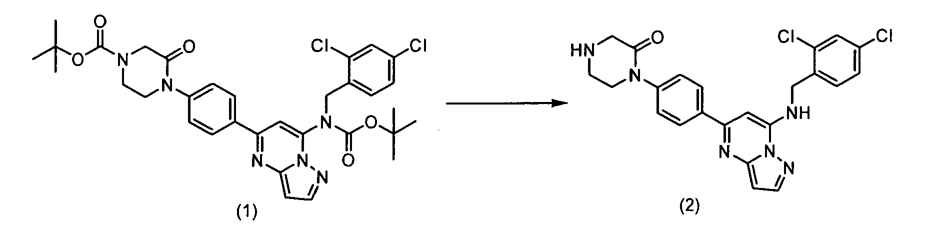
(2) By reacting and treating compound (1) in the same manner as example 46 there was obtained compound (2).
   APCI-MS (m/e): 467/469 (M+H)⁺ 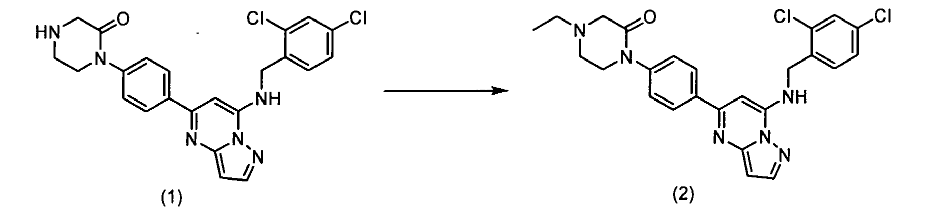
(3) To compound (1) (50mg) in methylene chloride (3ml) were added acetic acid (6.1µl), sodium triacetoxyborohydride (68mg) and acetoaldehyde (12µl), respectively at room temperature and the mixture was stirred for 3 hours. After addition of ethyl acetate, the reaction mixture was washed with aqueous saturated sodium bicarbonate solution and saturated brine, and dried. After removal of the solvent, the residue was purified with silica gel column chromatography (chloroform:methanol100:0→95:5) and was lyophilized with tert-butanol to give compound (2) (24mg) as a light yellow powder.
APCI-MS (m/e): 495/497 (M+H)⁺

### Example 174

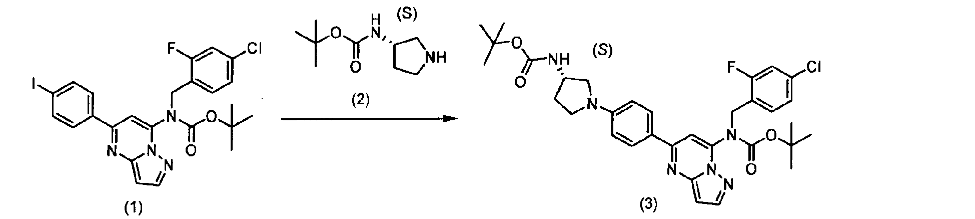

(1) By reacting and treating compound (1) and compound (2) in the same manner as example 73 there was obtained compound (3).
   APCI-MS (m/e): 637/639 (M+H)⁺ 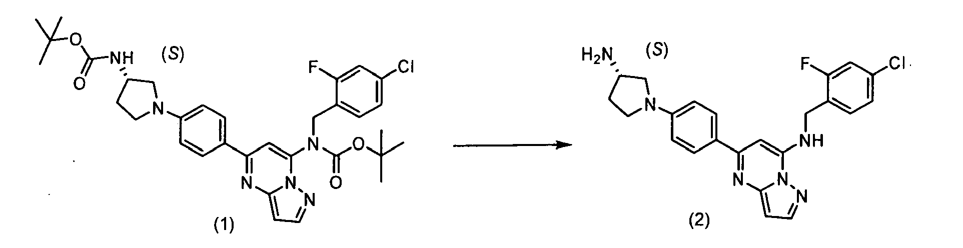
(2) By reacting and treating compound (1) in the same manner as example 46 there was obtained compound (2).
APCI-MS (m/e): 437/439 (M+H)⁺

### Example 175

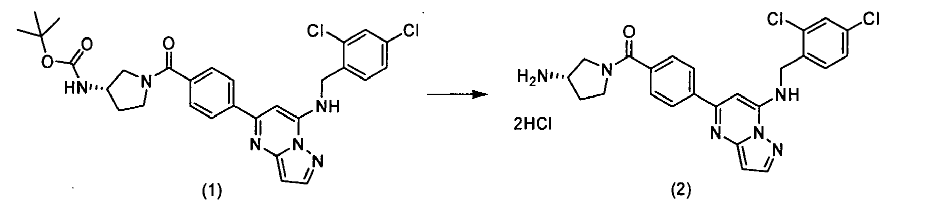

By reacting and treating compound (1) in the same manner as example 46 there was obtained compound (2).
APCI-MS (m/e): 481/483 (M+H)⁺

### Examples 176 to 189

By reacting and treating in the same manner as the above mentioned examples and reference examples, the following compounds were obtained.

| | | | |
|---|---|---|---|
| 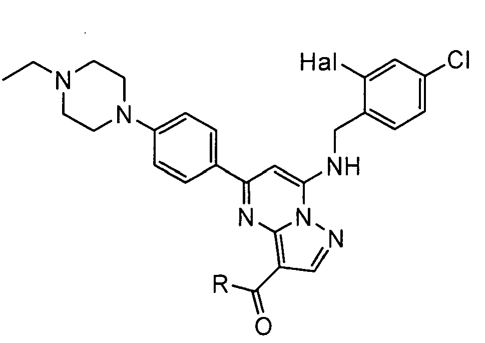 | | | |

| Example | R | Hal | MS([M+H]⁺) |
|---|---|---|---|
| 176 | Et₂N- | F | 564/566, APCI |
| 177 | 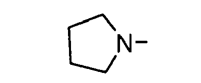 | F | 562/564, APCI |
| 178 | 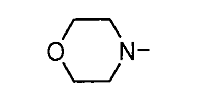 | F | 578/580, APCI |
| 179 | 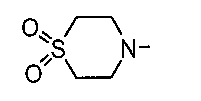 | F | 626/628, APCI |
| 180 | 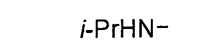 | F | 550/552, APCI |
| 181 | 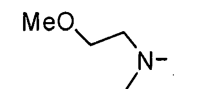 | F | 580/582, APCI |
| 182 | 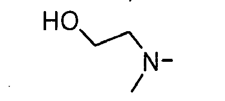 | F | 566/568, APCI |
| 183 | 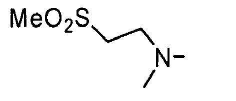 | F | 628/630, APCI |

| | | | |
|---|---|---|---|
| 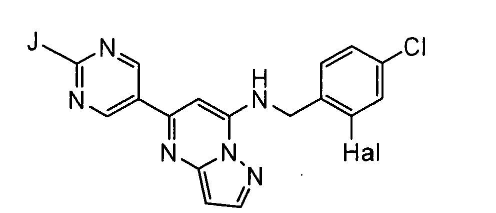 | | | |

| Example | J | Hal | MS([M+H]⁺) |
|---|---|---|---|
| 184 | 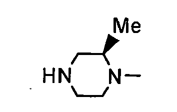 | Cl | 469/471, APCI |
| 185 | 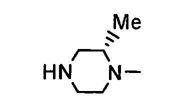 | Cl | 469/471, APCI |
| 186 | 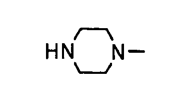 | Cl | 455/457, APCI |

| | | | | |
|---|---|---|---|---|
| 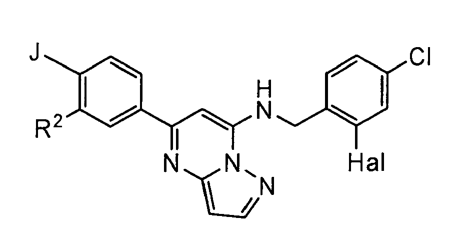 | | | | |

| Example | J | R² | Hal | MS([M+H]⁺) |
|---|---|---|---|---|
| 187 (2HCl) | 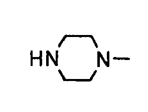 | -SO₂Me | F | 515/517, APCI |
| 188 (2HCl) | 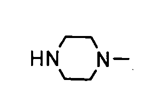 | -NO₂ | F | 482/484, APCI |
| 189 (2HCl) | 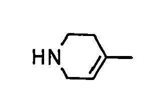 | H | Cl | 450/452, APCI |

### Reference example 1

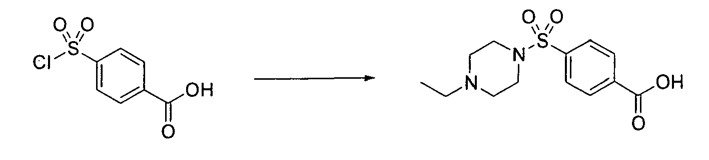

To a solution of 4-chlorosulfonylbenzenzoic acid (9.19g) in THF (100ml) were added under ice cooling ethylpiperazine (5.14g) and an aqueous solution (100ml) of potassium carbonate (5.53g), and the mixture was vigorously stirred for 2 hours. The reaction mixture was concentrated in vacuo and the residue was neutralized with hydrochloric acid. The resulting crystals were collected by filtration, washed with water, methanol and diethyl ether, successively and dried to give 4-(4-ethylpiperazine-1-sulfonyl)-benzoic acid (9.08g) as a colorless powder.
APCI-MS (m/e): 299 (M+H)⁺

### Reference example 2

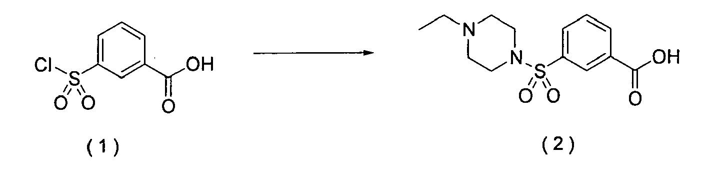

The compound (2) was prepared by reacting and treating the compound (1) in the same manner as reference example 1.
APCI-MS (m/e): 299 (M+H)⁺

### Reference example 3

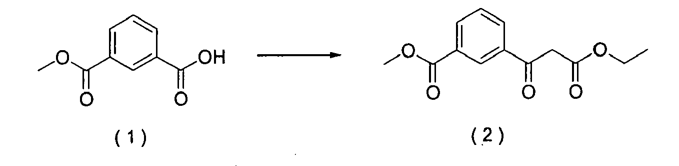

To a solution of monomethyl isophthalate (5g) in THF (100ml) was added under ice cooling N,N'-carbonyldiimidazole (4.87g), and the mixture was stirred at room temperature for 2 hours. To the reaction mixture was added under ice cooling magnesium mono methyl malonate (30.6g), and the mixture was stirred overnight at room temperature. The reaction mixture was concentrated and to the residue was added ethyl acetate. The mixture was washed with hydrochloric acid, water, saturated bicarbonate solution and saturated brine, and dried over sodium sulfate. After removal of the solvent, the residue was left to solidify to give methyl 3-(2-ethoxycarbonylacetyl)-benzoate (3.55g) as a yellowish solid.
APCI-MS (m/e): 251 (M+H)⁺

The following compounds were prepared in the same manner as Reference example 3.

### Reference example 4

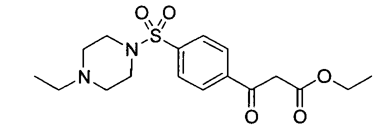

### APCI-MS (m/e): 369 (M+H)⁺

### Reference example 5

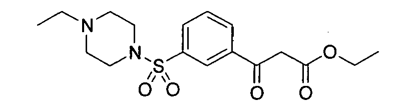

APCI-MS (m/e): 369 (M+H)⁺

The following compounds were prepared in the same manner as example 1 (2).
Reference example 6 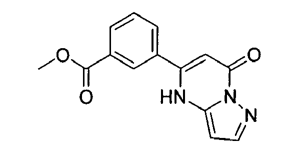 APCI-MS (m/e): 270 (M+H)⁺

### Reference example 7

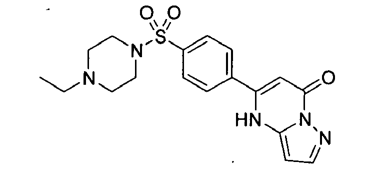

APCI-MS (m/e): 388 (M+H)⁺

### Reference example 8

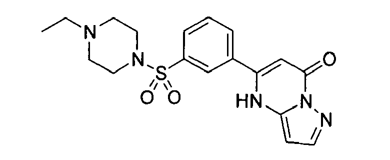

APCI-MS (m/e): 388 (M+H)⁺

### Reference example 9

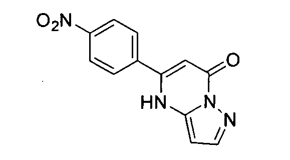

APCI-MS (m/e): 257 (M+H)⁺

### Reference example 10

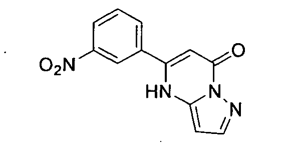

APCI-MS (m/e): 257 (M+H)⁺

The following compounds were prepared in the same manner as example 1 (3).
Reference example 11 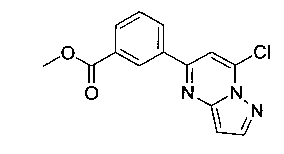 APCI-MS (m/e): 288/290 (M+H)⁺

### Reference example 12

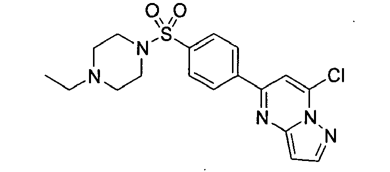

APCI-MS (m/e): 406/408 (M+H)⁺

### Reference example 13

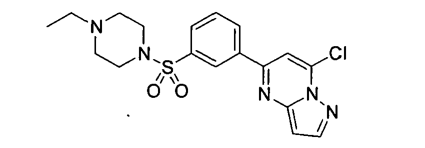

APCI-MS (m/e): 406/408 (M+H)⁺

### Reference example 14

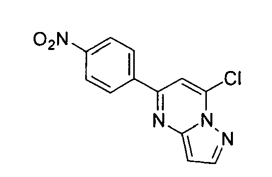

APCI-MS (m/e): 275/277 (M+H)⁺

### Reference example 15

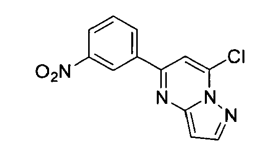

APCI-MS (m/e): 275/277 (M+H)⁺

The following compounds were prepared in the same manner as example 1 (4).

### Reference example 16

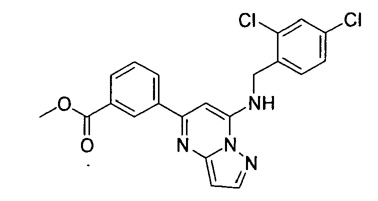

APCI-MS (m/e): 427/429 (M+H)⁺

### Reference example 17

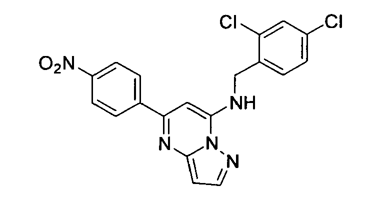

APCI-MS (m/e): 414/416 (M+H)⁺

### Reference example 18

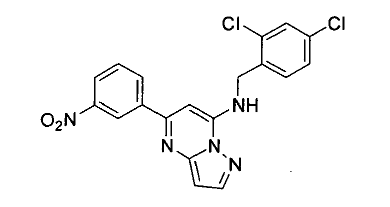

APCI-MS (m/e): 414/416 (M+H)⁺

### Reference example 19

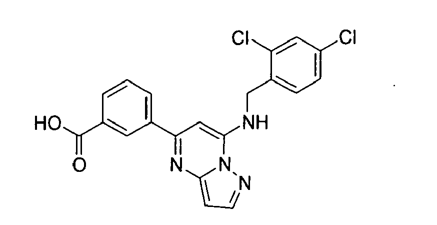

The above compound was prepared in the same manner as example 1 (5).
APCI-MS (m/e): 413/415 (M+H)⁺

### Reference example 20

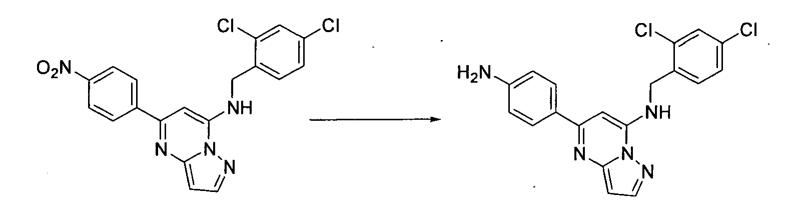

Ethanol (160ml) was added to (2,4-dichlorobenzyl)-[5-(4-nitrophenyl)-pyrazolo[1,5-a]pyrimidin-7-yl]amine (6.60g) and stannous chloride (12.6g), and the mixture was refluxed with stirring for 1.5 hours. After the reaction solution was cooled to room temperature, thereto was added saturated sodium bicarbonate solution, and the insoluble materials were filtered off over Celite. To the filtrate was added chloroform (500ml) and the solution was washed with saturated brine and dried over sodium sulfate. After removal of the solvent, the residue was crystallized from ethyl acetate, washed with a small amount of methanol and chloroform, and then dried to give [5-(4-aminophenyl)-pyrazolo[1,5-a]pyrimidin-7-yl]-(2,4-dichlorobenzyl)amine (2.50g) as pale yellow crystals.
APCI-MS (m/e): 384/386 (M+H)⁺

### Reference example 21

The following compounds were prepared in the same manner as reference example 20. 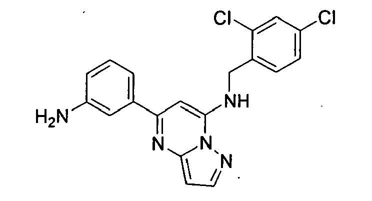 APCI-MS (m/e): 384/386 (M+H)⁺

### Reference example 22

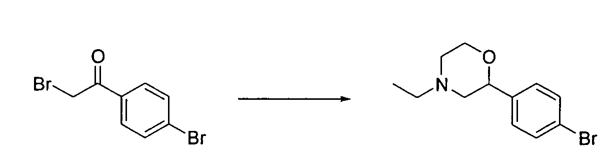

(1) To N-ethylaminoethanol was slowly added under ice cooling formic acid, and to the mixture was added 4-bromophenacyl bromide (9.37g). The mixture was stirred overnight at 100∼110°C and allowed to cool. Thereto was added ethyl acetate and water, and the solution was separated by a separating funnel. To the aqueous layer was added potassium carbonate, and the mixture was extracted three times with ethyl acetate. The combined organic layers were dried over magnesium sulfate and the solvent was removed. The residue was purified by NH-silica gel column chromatography(hexane/ethyl acetate = 100/0 →90/10) to give 2-(4-bromophenyl)-4-ethylmorpholine (5.90g) as a pale yellow oil.
   APCI-MS (m/e): 270/272 (M+H)⁺ 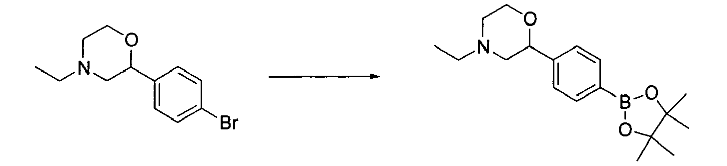
(2) To a mixture of 2-(4-bromophenyl)-4-ethylmorpholine (3.0g), 1,1'-bis(diphenylphosphino)ferrocene palladium(II) chloride (272mg), potassium acetate (3.27g) and bis(pinacolate)diboron (4.23g) was added DMSO (30ml), and the mixture was stirred under nitrogen atmosphere at 80°C for 4 hours and then overnight at 100°C. The reaction solution was allowed to cool, and thereto was added saturated sodium bicarbonate solution. The mixture was extracted with ethyl acetate and the extract was dried over magnesium sulfate. After removal of the solvent, the residue was purified twice by NH-silica gel column chromatography (hexane/ethyl acetate =100/0→70/30) to give 4-ethyl-2-{4-(4,4,5,5-tetramethyl[1,3,2]dioxaboran-2-yl)-phenyl}-morpholine (2.44g) as a pale yellow oil.
   ¹HNMR(400Mz/CDCl₃)δ(ppm):1.10(t,3H,J=7.2Hz),1.34(s,12H),1.99(t,1H,J=10.5H z),2.21(dt,1H,J=3.4,11.5Hz),2.44(d,2H),2.83(d,1H),2.95(d,1H),3.85(dt,1H,J=2.3,1 0.2Hz), 4.06(br d,1H),4.59(dd,1H,J=2.3,10.2Hz), 7.37(d,2H), 7.78(d,2H)

### Reference example 23

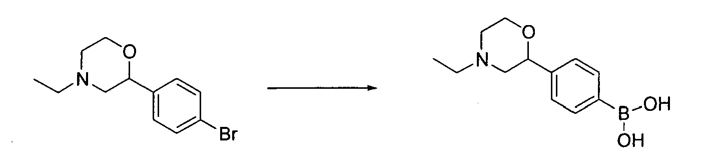

Into a solution of 2-(4-bromophenyl)-4-ethylmorpholine (2.99g) in THF (30ml) was slowly dropped under nitrogen atmosphere at - 78°C n-butyl lithium (1.7M in hexane) (7.2ml), and the mixture was stirred for 30 minutes. To the reaction solution was slowly added trimethylborate (2.48ml) and the mixture was stirred. The mixture was gradually warmed to room temperature and stirred overnight. Thereto was added a saturated aqueous ammonium chloride solution, and the mixture was stirred at room temperature for one hour. Thereto was added phosphate buffer (0.5M, pH8.0), and the solution was extracted three times with ethyl acetate. The combined organic layers were dried over magnesium sulfate. After removal of the solvent, to the residue was added diethyl ether. The resulting solid was dried to give 4-(4-ethylmorpholin-2-yl)boric acid (795mg) as an orange solid. This crude product without purification was served to the reaction in example 44.
¹HNMR(400Mz/CDCl₃)δ(ppm):1.11(t,3H),2.07(br,1H),2.23(t,1H,J=11.0Hz),2.48(d, 2H,J=5.6Hz),2.84(d, 1H,J= 10.2Hz),2.97(d, 1H,J=10.2Hz),3.84(t, 1H,J= 11.3Hz),3.8 7(br,1H),4.59(d,1H,J=10.0Hz),7.33(br s,3H),7.74(br s,1H),7.79(br s,2H)

### Reference example 24

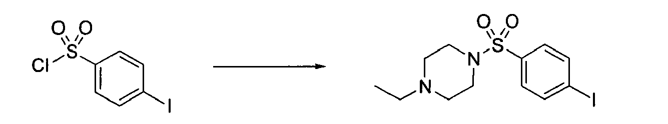

(1) Into a solution of 4-iodobenzenesulfonyl chloride (4.54g) in chloroform (30ml) were slowly dropped at 0°C a chloroform solution (20ml) of ethylpiperazine (1.83g) and triethylamine (2.02g), and the mixture was stirred at 0°C for one hour and then stirred overnight at room temperature. Thereto was added chloroform, and the mixture was washed with saturated sodium bicarbonate solution and saturated brine, and dried over magnesium sulfate. After removal of the solvent, the residue was purified with silica gel column chromatography (chloroform/methanol=100/0→90/10) and recrystallized from ethyl acetate to give 1-ethyl-4-(4-iodobenzenesulfonyl)-piperazine (5.36g) as colorless crystals. APCI-MS (m/e): 381 (M+H)⁺ 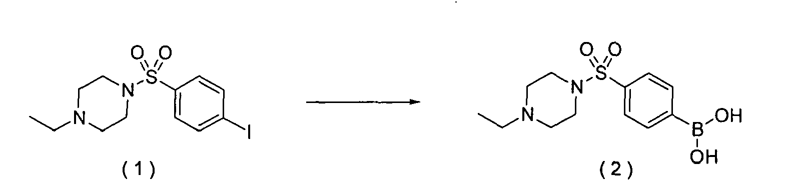
(2) The compound (2) was prepared by reacting and treating the compound (1) in the same manner as reference example 23.
   ¹HNMR(400Mz/DMSO-d₆)δ(ppm):0.94(t,3H,J=6.6Hz),2.23-2.60(br,6H), 2.85(br s,4H),7.69(d,2H,J=8.2Hz),8.02(d,2H,J=8.2Hz),8.46(s,2H)

### Reference example 25

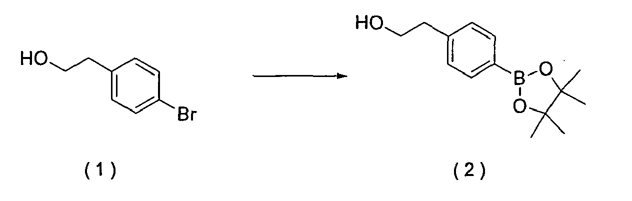

(2) The compound (2) was prepared by reacting and treating the compound (1) in the same manner as reference example 22 (2).
¹HNMR(400Mz/CDCl₃)δ(ppm):1.34(s,12H),2.89(t,2H,J=6.7Hz),3.86(t,2H,J=6.7Hz ),7.25(d,2H,J=7.9Hz),7.77(d,2H,J=7.9Hz)

### Reference example 26

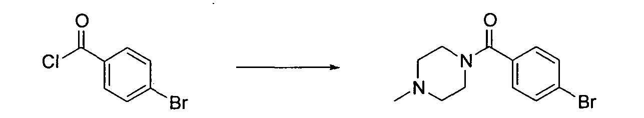

(1) 4-Bromobenzoyl chloride (37.37g) was dissolved in a mixture of methylene chloride (150ml) and THF (50ml), and to the solution were slowly added a solution of methylpiperidine (18.95g) in THF (10ml) and triethylamine (47ml), followed by stirring for 30 minutes. Thereto was added an aqueous potassium carbonate solution, and the solution was extracted three times with chloroform/methanol (9/ 1). The combined organic layers were dried over magnesium sulfate. After removal of the solvent, the residue was purified twice by NH-silica gel column chromatography (hexane/ethyl acetate100/0→50/50) to give (4-bromophenyl)-(4-methylpiperazin-1-yl)-methanone (45.51g) as a pale yellow solid.
   APCI-MS (m/e): 283/285 (M+H)⁺ 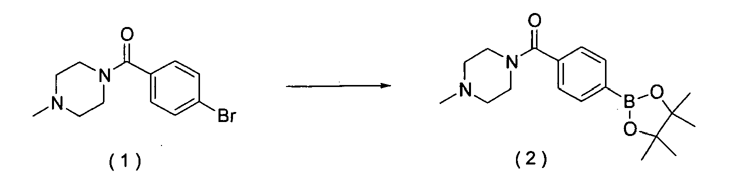
(2) The compound (2) was prepared by reacting and treating the compound (1) in the same manner as reference example 22 (2). The compound (2) in crude without purification was served to the reaction in example 51.
   ¹HNMR(400Mz/CDCl₃)δ(ppm):1.35(s,12H),2.31(s,3H),2.48(br s,2H),2.62(s,2H),3.39(br s,2H),3.80(br s,2H),7.38(d,2H,J=8.2Hz),7.84(d,2H,J=8.2Hz)

### Reference example 27

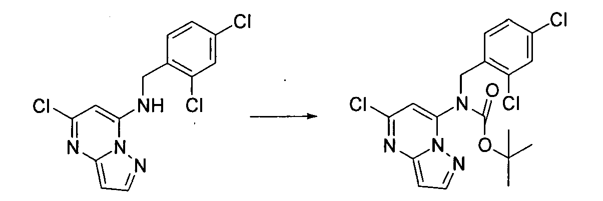

(1) To a solution of (5-chloropyrazolo[1,5-a]pyrimidin-7-yl)-(2,4-dichlorobenzyl)amine (655mg) in chloroform (7ml) were added di-tert-butyl dicarbonate (437mg) and 4-dimethylaminopyridine (24mg), and the mixture was refluxed with stirring for 1.5 hours. Thereto was an additional di-tert-butyl dicarbonate (83mg) and the mixture was refluxed with stirring for 30 minutes. After removal of the solvent, the residue was purified by silica gel column chromatography (hexane:ethyl acetate = 10:0→10:1) to give tert-butyl (5-chloropyrazolo[1,5-a]pyrimidin -7-yl)-(2,4-dichlorobenzyl)-carbamate (953.3mg) as a colorless viscous oil.
   APCI-MS (m/e): 427/429 [M+H]⁺ 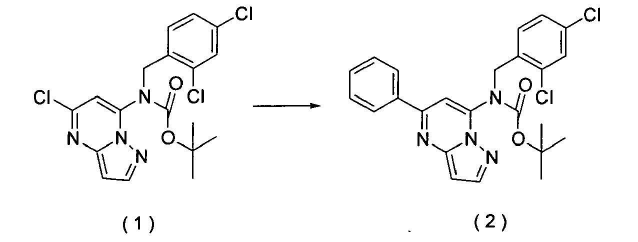
(2) Compound (2) was prepared by reacting and treating compound (1) in the same manner as example 44.
   APCI-MS (m/e): 469/471 [M+H]⁺ 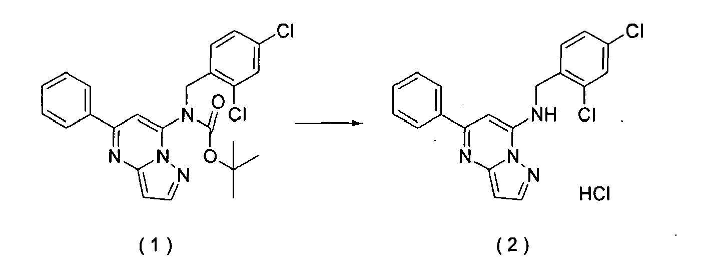
(3) Compound (2) was prepared by reacting and treating compound (1) in the same manner as example 46 (2).
   APCI-MS(m/e):369/371[M+H]⁺

### Reference example 28

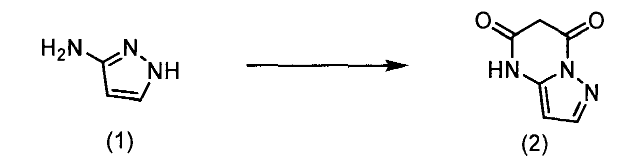

(1) To aminopyrazole (16g) and dimethyl malonate (26.4g) in methanol (500ml) was dropped sodium methoxide (28% methanol) (77.2g) and the mixture was stirred under reflux for 18 hours. After being cooled and being concentrated in vacuo, the resulting crystals were filtered and washed with a small amount of methanol. The obtained powders were dissolved in water (500ml) and the solution was adjusted to pH3-4 with concentrated hydrochloric acid. The resulting crystals were filtered and washed with water, ethanol and diethyl ether, and dried to give 4H-pyrazolo[1,5-a]pyrimidin-5,7-dione (22.1g) as a colorless powder.
   APCI-MS (m/e): 152 (M+H)⁺ 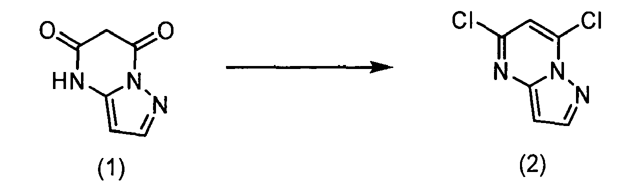
(2) By reacting and treating compound (1) in the same manner as example 1 (3) there was obtained compound (2).
   APCI-MS (m/e): 188/190 (M+H)⁺ 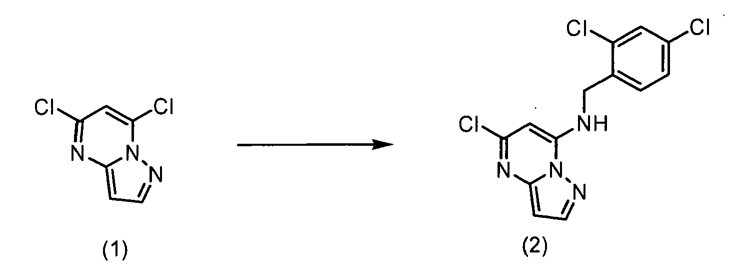
(3) To compound (1) (10.85g) in 1,4-dioxane (200ml) were added 2,4-dichlorobenzylamine (15.2g) and triethylamine (14.6g) at room temperature, and the mixture was stirred overnight. After the reaction mixture was concentrated in vacuo, to the residue was added ethyl acetate and the mixture was washed with aqueous saturated sodium bicarbonate solution and saturated brine, and dried. After removal of the solvent, the residue was purified with silica gel column chromatography (hexane:ethyl acetate=4:1) to give compound (2) (18.0g) as a colorless powder.
APCI-MS (m/e): 327/329 (M+H)⁺

### Reference example 29

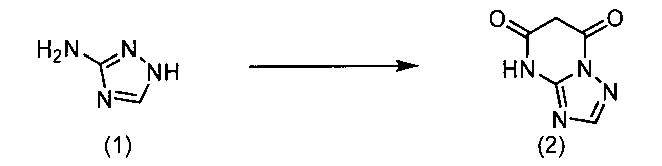

(1) By reacting and treating compound (1) in the same manner as reference example 34 there was obtained compound (2).
   APCI-MS (m/e): 151 (M+H)⁺ 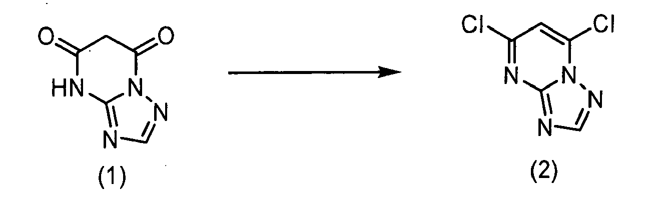
(2) By reacting and treating compound (1) in the same manner as example 1 (3) there was obtained compound (2).
   APCI-MS (m/e): 187/189 (M+H)⁺

### Reference example 30

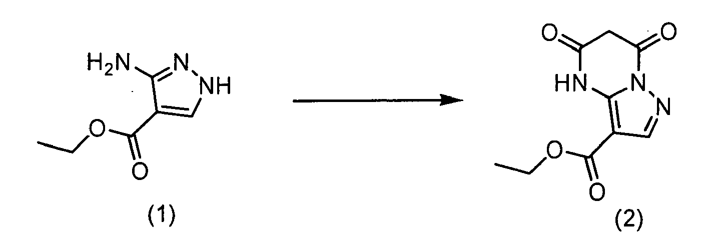

(1) By reacting and treating compound (1) in the same manner as reference example 34 there was obtained compound (2).
   APCI-MS (m/e): 224 (M+H)⁺ 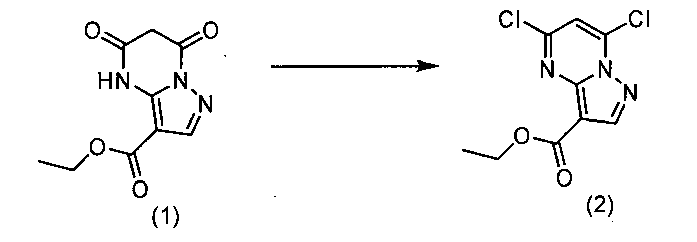
(2) By reacting and treating compound (1) in the same manner as example 1 (3) there was obtained compound (2).
   APCI-MS (m/e): 260/262 (M+H)⁺

By treating in the same manner as the above reference example the following compounds were obtained.

### Reference example 31

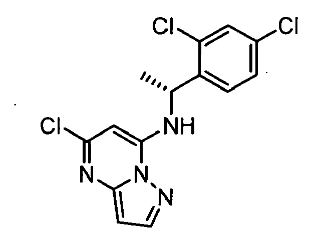

APCI-MS (m/e): 341/343 (M+H)⁺

### Reference example 32

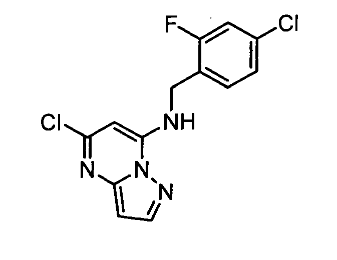

APCI-MS(m/e): 311/313 (M+H)⁺

### Reference example 33

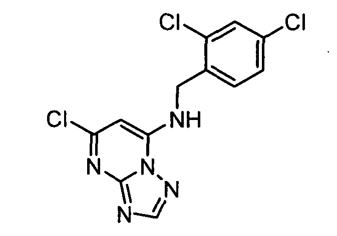

APCI-MS (m/e): 328/330 (M+H)⁺

### Reference example 34

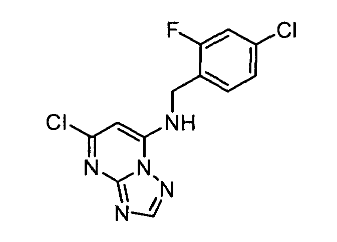

APCI-MS (m/e): 310/312 (M+H)⁺

### Reference example 35

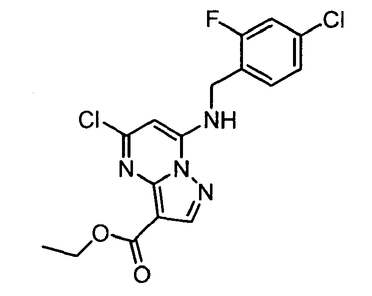

APCI-MS (m/e): 383/385 (M+H)⁺

By treating in the same manner as the above reference example the following compounds were obtained.

### Reference example 36

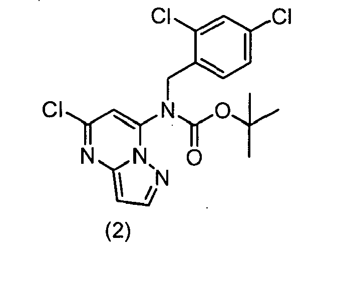

APCI-MS (m/e): 427/429 (M+H)⁺

### Reference example 37

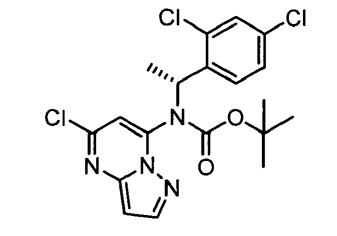

APCI-MS (m/e): 441/443 (M+H)⁺

### Reference example 38

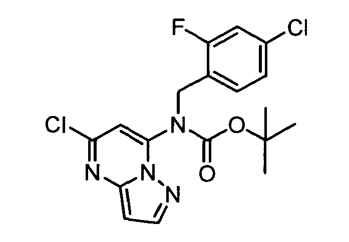

APCI-MS (m/e): 411/413 (M+H)⁺

### Reference example 39

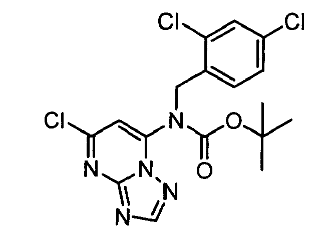

APCI-MS (m/e): 428/430 (M+H)⁺

### Reference example 40

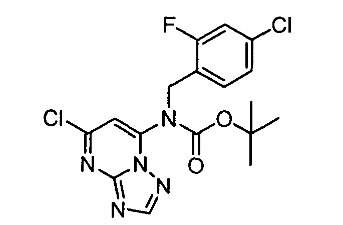

APCI-MS (m/e): 412/414 (M+H)⁺

### Reference example 41

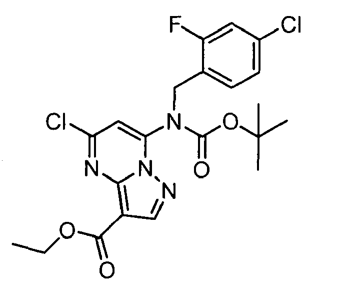

APCI-MS (m/e): 483/485 (M+H)⁺

### Reference example 42

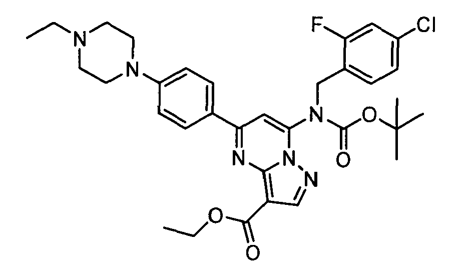

APCI-MS (m/e): 637/639 (M+H)⁺

### Reference example 43

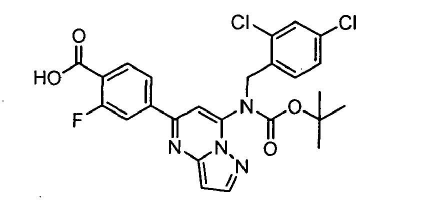

APCI-MS (m/e): 529/531 (M-H)⁻

### Reference example 44

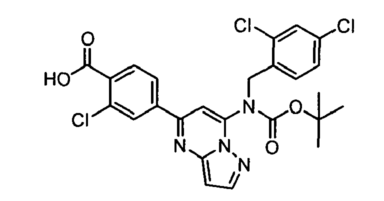

APCI-MS (m/e): 545/547 (M-H)⁻

### Reference example 45

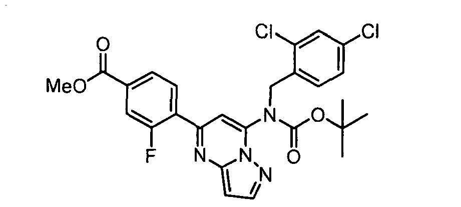

APCI-MS (m/e): 545/547 (M+H)⁺

### Reference example 46

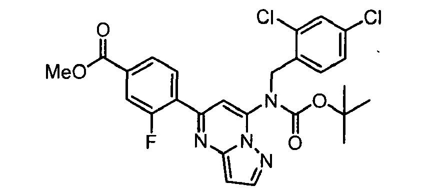

APCI-MS (m/e): 531/533 (M+H)⁺

### Reference example 47

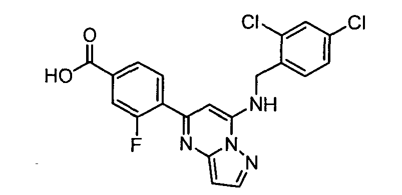

APCI-MS (m/e): 431/433 (M+H)⁺

### Reference example 48

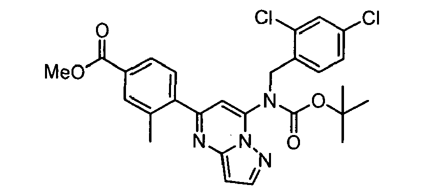

APCI-MS (m/e): 541/543 (M+H)⁺ Reference example 49 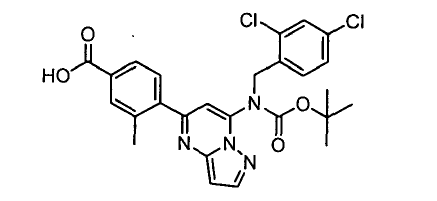 APCI-MS (m/e): 527/529 (M+H)⁺

### Reference example 50

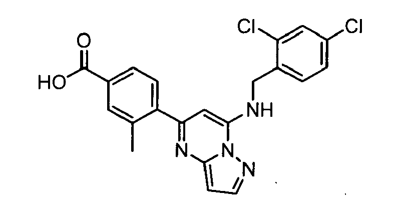

APCI-MS (m/e): 427/429 (M+H)⁺

### Reference example 51

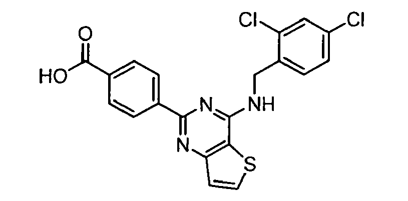

APCI-MS (m/e): 430/432 (M+H)⁺

### Reference example 52

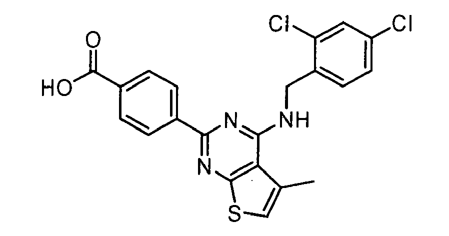

APCI-MS (m/e): 444/446 (M+H)⁺

### Reference example 53

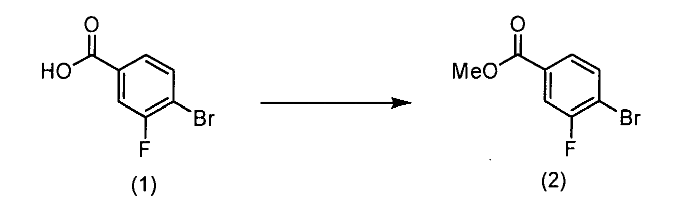

(1) By reacting and treating compound (1) in the same manner as example 68 (1) there was obtained compound (2).
   ¹HNMR(400MHz/CDCl₃)δ(ppm):3.94(s,3H),7.77(dd,1H,J=1.8Hz,9.0Hz),7.71(dd,1 H,J=1.8Hz,10.2Hz),7.64(dd,1H,J=6.7Hz,8.5Hz) 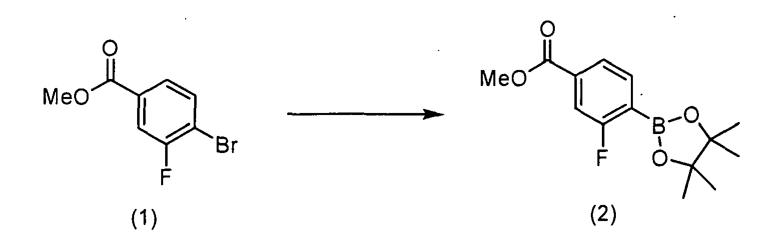
(2) By reacting and treating compound (1) in the same manner as reference example 22 (2) there was obtained compound (2).
   ¹HNMR(400MHz/CDCl₃)δ(ppm):1.37(s,12H),3.93(s, 3H), 7.86(d,1H,J=10.0Hz),7.80-7.81(m,2H)

### Reference example 54

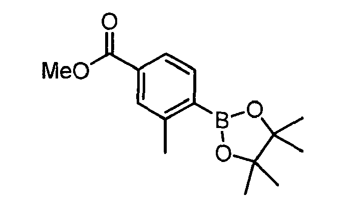

APCI-MS (m/e): 277 (M+H)⁺

### Reference example 55

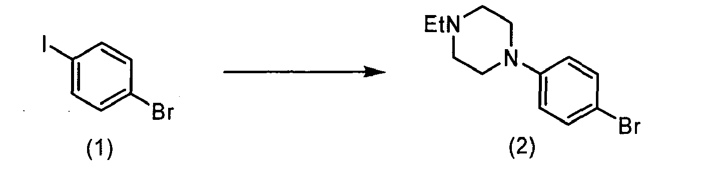

(1) To 4-bromo-iodobenzene (40.0g) and N-ethylpiperazine (32.3g) in 2-propanol (400ml) were added ethylene glycol (26.3g), cupper iodide (I) (5.39g) and potassium phosphate powder (91.9g) and the mixture was stirred under reflux. After being cooled the insoluble materials were filtered off and the filtrate was concentrated in vacuo. To the residue was added ethyl acetate and the mixture was washed with aqueous saturated sodium bicarbonate solution and dried. After removal of the solvent, the residue was purified with silica gel column chromatography(chloroform:methanol= 100:0→95:5) to give 1-(4-bromophenyl)-4-ethylpiperazine (2) (30.1g) as a colorless solid.
   APCI-MS (m/e): 269/271 (M+H)⁺ 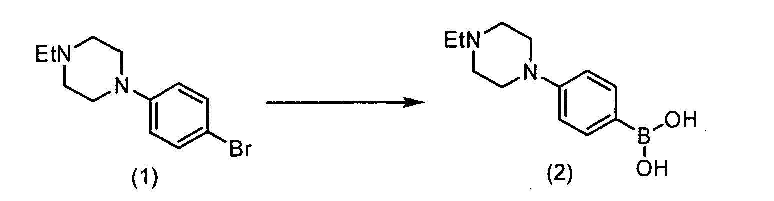
(2) By reacting and treating compound (1) in the same manner as reference example 23 there was obtained compound (2).
APCI-MS (m/e): 235 (M+H)⁺

### Reference example 56

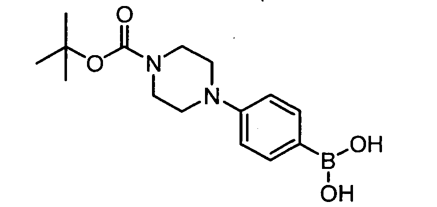

APCI-MS (m/e): 307 (M+H)⁺

### Reference example 57

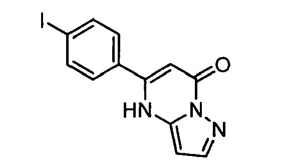

APCI-MS (m/e): 338 (M+H)⁺

### Reference example 58

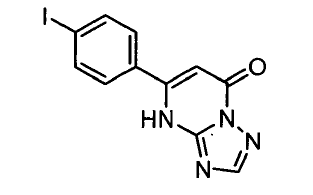

APCI-MS (m/e): 339 (M+H)⁺

### Reference example 59

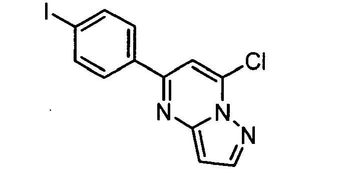

APCI-MS (m/e): 356/358 (M+H)⁺

### Reference example 60

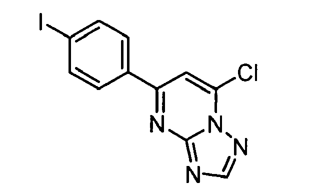

APCI-MS (m/e): 357/359 (M+H)⁺

### Reference example 61

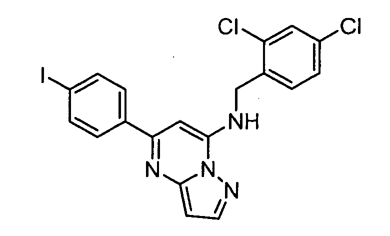

APCI-MS (m/e): 495/497 (M+H)⁺

### Reference example 62

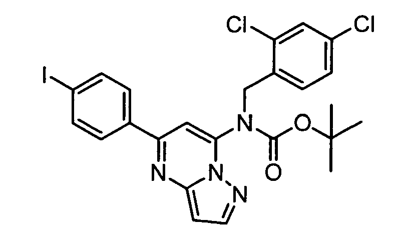

APCI-MS (m/e): 595/597 (M+H)⁺

### Reference example 63

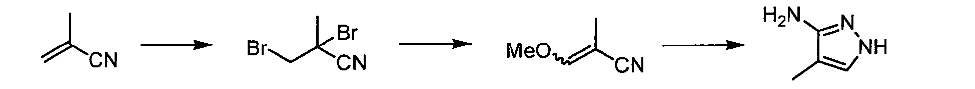

To methyl acrylonitrile (40g) was gradually added bromine at 45°C in a period of 2 hours, and the mixture was stirred for 2 hours. To potassium hydroxide (67.4g) in methanol (500ml) was gradually added the above reaction mixture at 0°C in a period of 30 minutes. Two hours later the temperature was raised to room temperature and the mixture was stirred for a day. The reaction mixture was concentrated in vacuo. To the residue was added water and the mixture was extracted with diethyl ether twice. The combined organic layer was washed with saturated brine, dried over magnesium sulfate, and filtered. After removal of the solvent, to the residue were added ethanol (50ml) and hydrazine hydrate (35.8g), and the mixture was stirred at 80°C for a day. Thereto was added additional hydrazine (10g) and the mixture was stirred at 80°C for additional 1 day. After the reaction was concentrated in vacuo, the residue was dissolved in a mixture of chloroform/methanol(9/1). Thereto was added silica gel and the mixture was allowed to stand. After filtration, the filtrate was concentrated in vacuo to give 4-methyl-1H-pyrazol-3-ylamine (46.7g) as a dark purple oil.
APCI-MS (m/e): 98 [M+H]⁺

### Reference example 64

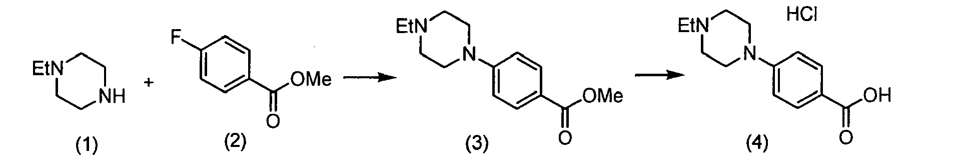

(1) To compound (1) (20.0g) and compound (2) (32.0g) in dimethylacetamide (90ml) was added potassium carbonate powder (27.6g) and the mixture was stirred at 150°C overnight. After being cooled, to the mixture was added ethyl acetate, and the mixture was washed with water and saturated brine, and dried. After removal of the solvent, the residue was purified with silica gel column chromatography (chloroform:methanol=100:0→95:5) to give a yellowish orange solid. The solid was recrystallized from diisopropyl ether/hexane to give compound (3) (25.8g) as yellow crystals. Compound (3) was dissolved in 6N hydrochloric acid (120ml), and the solution was stirred under reflux for 9 hours. The reaction mixture was cooled and allowed to stand for 2 hours. The resulting crystals were taken by filtration, washed with a small amount of cold water and dried to give compound (4) (15.9g) as a pale yellow solid.
   APCI-MS (m/e): 235 (M+H)⁺ 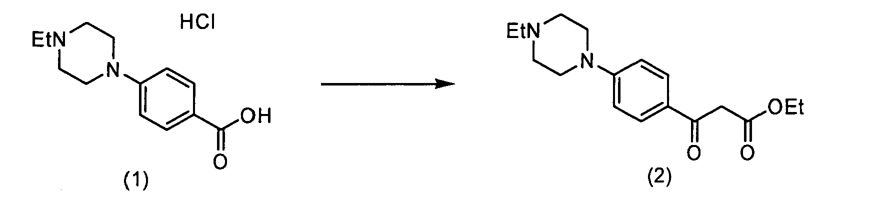
(2) By reacting and treating compound (1) in the same manner as reference example 3 there was obtained compound (2).
   APCI-MS (m/e): 305 (M+H)⁺ 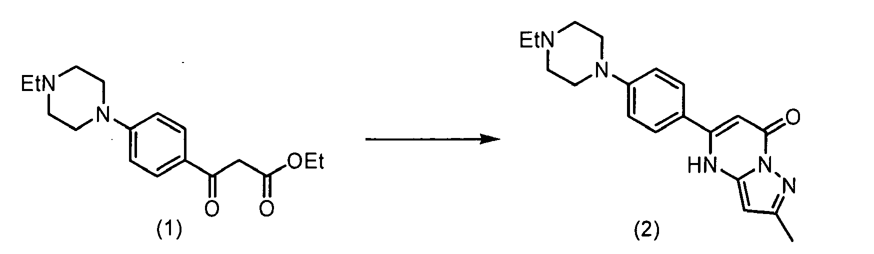
(3) By reacting and treating compound (1) in the same manner as example 1 (2) there was obtained compound (2).
   APCI-MS (m/e): 338 (M+H)⁺ 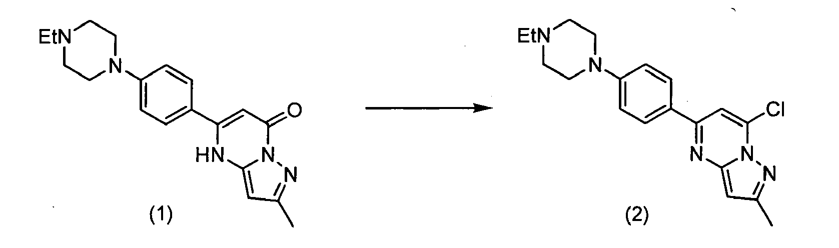
(4) By reacting and treating compound (1) in the same manner as example 1 (3) there was obtained compound (2).
APCI-MS (m/e): 356/358 (M+H)⁺

By treating in the same manner as the above reference example the following compounds were obtained.

### Reference example 65

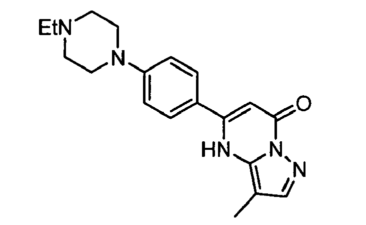

APCI-MS (m/e): 338 (M+H)⁺

### Reference example 66

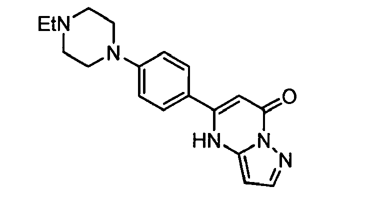

APCI-MS (m/e): 324 (M+H)+

### Reference example 67

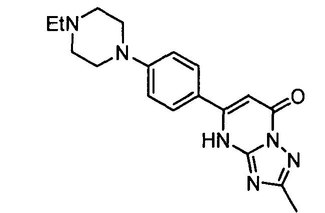

APCI-MS (m/e): 339 (M+H)+

### Reference example 68

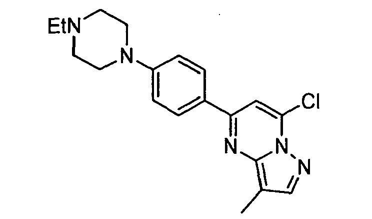

APCI-MS (m/e): 356/358 (M+H)+

### Reference example 69

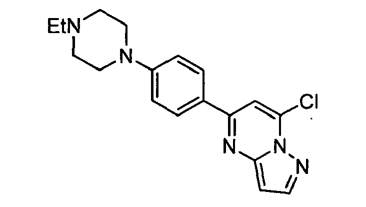

APCI-MS (m/e): 342/344 (M+H)+

### Reference example 70

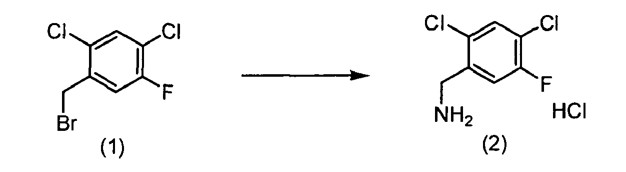

To compound (1) (624mg) in THF (30ml) was added 60% sodium hydride (145mg) and the mixture was stirred for 30 minutes. And then thereto was added tert-dibutyl iminodicarboxylate (788mg) and the mixture was stirred at 55°C for 1 hour. Thereto was added additional tert-dibutyl iminodicarboxylate (788mg) and the mixture was stirred at 55°C overnight. After the reaction mixture was cooled, thereto was added ethyl acetate, and the mixture was washed with water, and dried. After removal of the solvent the residue was purified with silica gel column chromatography (hexane:ethyl acetate= 100:0→ 95:5) to give an oily product. To the product in ethyl acetate was added 4N hydrochloric acid /ethyl acetate (20ml), and the mixture was stirred at room temperature for 1.5 hours. The reaction mixture was concentrated in vacuo. The residue was crushed with diethyl ether/hexane and dried to give compound (2) (490mg) as a colorless solid.
APCI-MS (m/e): 194/196 (M+H)⁺

### Reference example 71

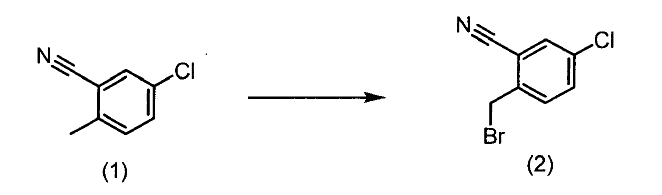

(1) To 5-chloro-2-methylbenzonitrile (7.8g) in carbon tetrachloride (50ml) were added N-bromosuccinimide (9.26g) and benzoylperoxide (515mg), and the mixture was stirred under reflux overnight. The reaction mixture was cooled and purified with silica gel column chromatography (hexane:ethyl acetate=30:1) to give compound (2) (8.87g) as a yellow transparent oil.
   ¹HNMR(400MHz/CDCl₃)δ(ppm):4.60(s,2H),7.50(d,1H,J=8.5Hz),7.57(dd,1H,J=2.3, 8.5Hz),7.65(d,1H,J=2.3Hz) 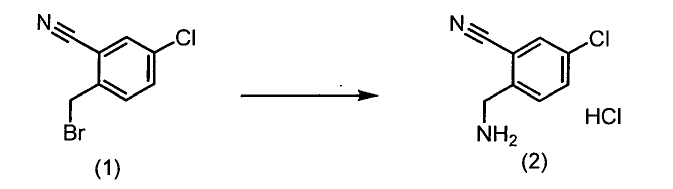
(2) By reacting and treating compound (1) in the same manner as reference example 70 there was obtained compound (2).
   APCI-MS (m/e): 167/169 (M+H)+

### Reference example 72

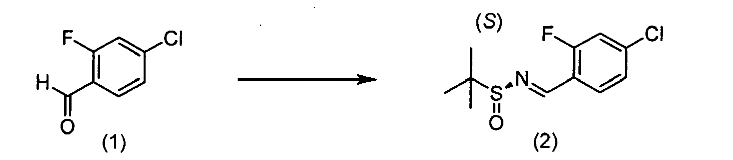

(1) To 4-chloro-2-fluorobenzaldehyde (26.2g) and (S)(-)-2-methyl-2-propanesulfonamide (21.0g) in THF (940ml) was gradually dropped titanium ethoxide (IV) (80.9g) at room temperature, and the mixture was stirred under reflux for 2.5 hours. After being cooled, the reaction mixture was gradually poured into cooled saturated brine under stirring and the insoluble materials were filtered off over Celite. To the filtrate was added methylene chloride (1800ml) and the mixture was separated by a separating funnel, followed by dry. The solvent was removed to give compound (2) (42.3g) as a colorless solid.
   APCI-MS (m/e): 262/264 (M+H)+ 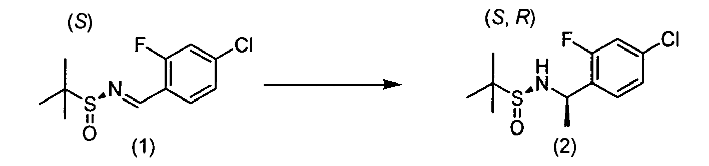
(2) To methylmagnesium bromide (in 3M diethyl ether) (81ml) was dropped compound (1) (42.3g) in THF (1.2L) under a nitrogen atmosphere in a dry-ice/acetone bath(inner temperature: -30∼-5°C) in a period of 40 minutes and temperature of the mixture was raising to room temperature under stirring overnight. To the reaction mixture was gradually added aqueous ammonium chloride solution under ice-cooling and the mixture was extracted with methylene chloride, followed by dry. After removal of the solvent, the residue was purified with silica gel column chromatography (hexane:ethyl acetate=9:1→ 1:0) and recrystallized from hexane to give compound (2) (44.5g) as colorless crystals.
   APCI-MS (m/e): 278/280 (M+H)⁺ 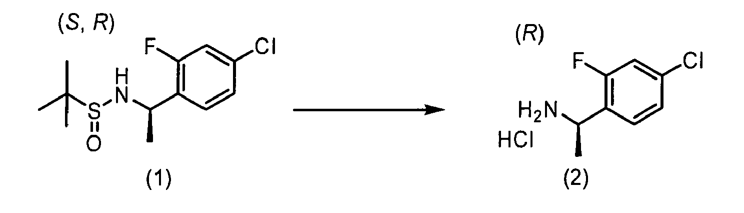
(3) To compound (1) (36.9g) in methanol (216ml) was added 4N hydrochloric acid /1,4-dioxane (216ml) under ice-cooling, and the mixture was stirred at room temperature for 2 hours. The reaction mixture was concentrated and the residue was crushed with diethyl ether, followed by dry to give compound (2) (27.7g) as a colorless solid.
APCI-MS (m/e): 174/176 (M+H)⁺

By treating in the same manner as the above reference example the following compounds were obtained.

### Reference example 73

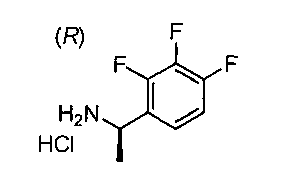

APCI-MS (m/e): 176 (M+H)⁺

### Reference example 74

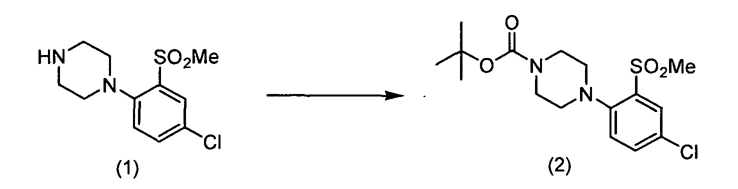

(1) By reacting and treating compound (1) in the same manner as reference example 27 there was obtained compound (2).
   APCI-MS (m/e): 375/377 (M+H)+ 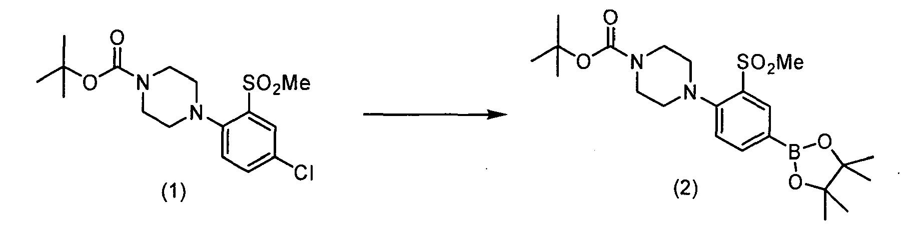
(2) To compound (1) (525mg) in 1,4-dioxane (8.4ml) were added bis (pinacolate)diboron (498mg), bis(dibenzilideneacetone)palladium (48.3mg), tricyclohexylphosphine tetrafluoroborate (68mg) and potassium acetate (207mg), and the mixture was stirred under a nitrogen atmosphere at 80°C overnight. Thereto were added water and ethyl acetate, and the insoluble materials were filtered off. The filtrate was extracted with ethyl acetate and dried. After removal of the solvent, the residue is crystallized from diethyl ether/isopropyl ether to give the object compound (2) (459mg) as colorless crystals.
APCI-MS (m/e): 467 (M+H)+

By treating in the same manner as the above reference example the following compounds were obtained.

### Reference example 75

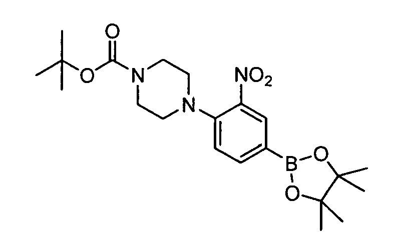

APCI-MS (m/e): 434 (M+H)+

### Reference example 76

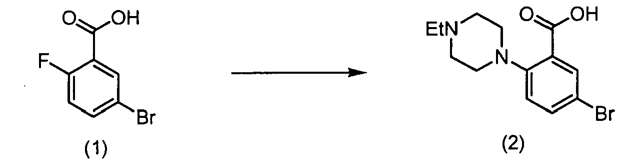

(1) To compound (1) (3.0g) were added piperazine (7.83g) and N,N-dimethyacetamide (10ml) and the mixture was stirred at 130°C overnight. The reaction mixture was concentrated in vacuo and the residue was crushed with diethyl ether. The crushed residue was washed with a small amount of water and dried to give compound (2) (3.11g) as a yellow solid.
   APCI-MS (m/e): 313/315 (M+H)+ 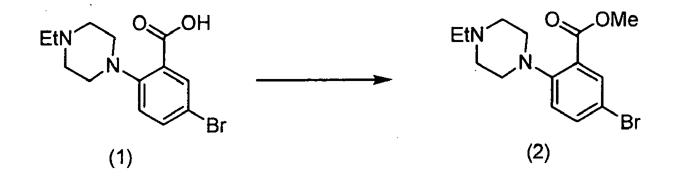
(2) To compound (1) (3.03g) in methanol (250ml) was added concentrated sulfuric acid (10ml) and the mixture was stirred under reflux for 4 hours. The reaction mixture was concentrated in vacuo and to the residue were added water and potassium carbonate to adjust pH of the solution to 9. The solution was extracted with diethyl ether twice and dried. After removal of the solvent, there was obtained compound (2) (2.61g) as a pale brown caramel.
   APCI-MS (m/e): 327/329 (M+H)+ 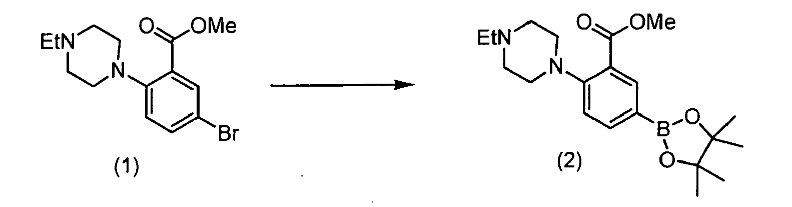
(3) By reacting and treating compound (1) in the same manner as reference example 22 (2) there was obtained compound (2).
APCI-MS (m/e): 375 (M+H)⁺

### Reference example 77

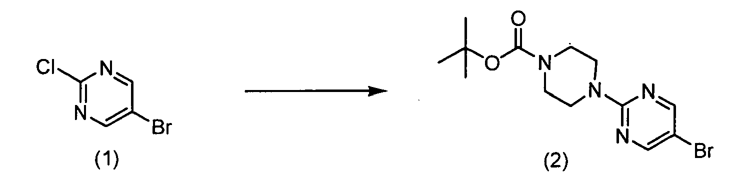

(1) To 5-bromo-2-chloropyrimidine 2.90g, 1-tert-butoxycarbonylpiperazine 4.19g in 1,4-dioxane 70ml was added potassium carbonate 3.73g, and the mixture was stirred under reflux for 1.5 hours. Thereto was added water and the mixture was extracted with diethyl ether twice. The combined organic layer was dried and the solvent was removed and purified with silica gel column chromatography (hexane:ethyl acetate=15:1→8:1) to give compound (2) (5.09g) as a colorless solid.
   APCI-MS (m/e): 343/345 (M+H)⁺ 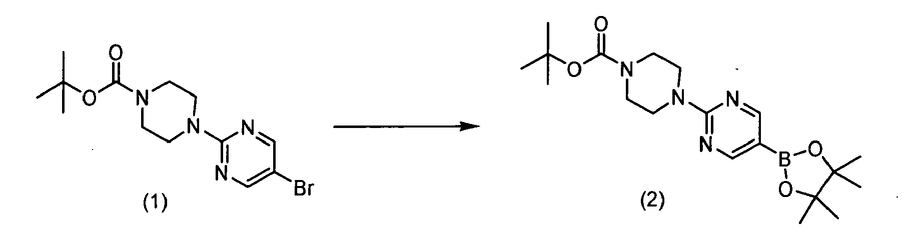
(2) By reacting and treating compound (1) in the same manner as reference example 22 (2) there was obtained compound (2).
APCI-MS (m/e): 391 (M+H)+

### Reference example 78

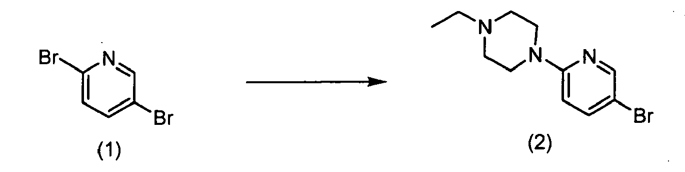

(1) To compound (1) (29.6g) and ethypiperazine (11.4g) in toluene (150ml) were added tris(dibenzilideneacetone)dipalladium (1.83g), xantphos (3.46g), sodium tert-butoxide (14.9g) and the mixture was stirred under a nitrogen atmosphere at 100°C for 3 hours. After the reaction mixture was cooled, thereto was added ethyl acetate and the mixture was washed with water. The organic layer was extracted with hydrochloric acid twice, and the extract combined with the previous aqueous layer was washed with diethyl ether twice. The solution was made weak alkaline with potassium carbonate and extracted with ethyl acetate twice. The combined organic layer was dried and the solvent was removed to give compound (2) (18.1g) as a pale orange solid.
   APCI-MS (m/e): 270/272 (M+H)+ 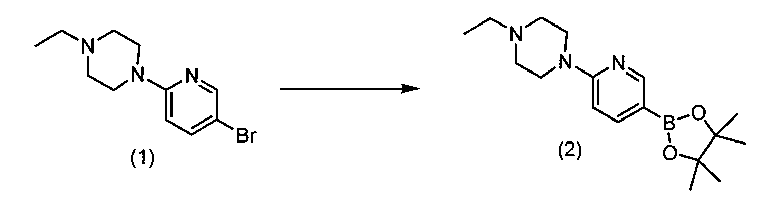
(2) By reacting and treating compound (1) in the same manner as reference example 22 (2) there was obtained compound (2).
APCI-MS (m/e): 318 (M+H)⁺

### Reference example 79

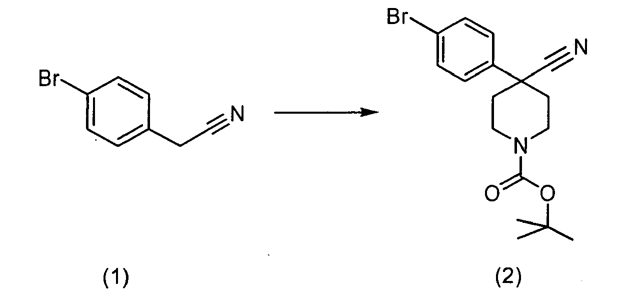

(1) To compound (1) (4.0g) in DMSO (60ml) was gradually added 60% sodium hydride (1.35g) under ice-cooling, and the mixture was stirred at 0°C for 1 hour. Thereto was dropped N - tert-butoxycarbonyl-N,N-bis(2-chloroethyl)amine (4.59g) in DMSO (30ml) and the mixture was stirred at room temperature for 3 hours. The reaction mixture was poured to ice water, and the mixture was extracted with methylene chloride. The extract was washed with water, and dried. After removal of the solvent, the residue was purified with NH-silica gel chromatography (hexane/ethyl acetate=100/0→100/20) to give compound (2) (2.03g) as a yellow oily product.
   APCI-MS (m/e): 265/267 (M+2H-BOC)⁺ 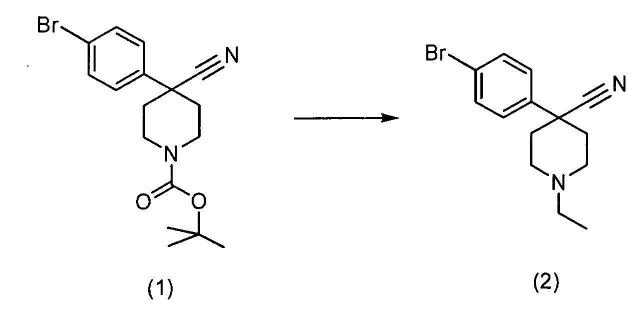
(2) To compound (1) (1.0g) in methylene chloride (6.0ml) was added trifluoroacetic acid (3.0ml) and the mixture was stirred at room temperature overnight. After the reaction mixture was concentrated in vacuo, to the residue was added aqueous saturated sodium bicarbonate solution and the mixture was extracted with ethyl acetate. The organic layer was washed with saturated brine, and dried. After removal of the solvent, to the residue in DMF (10ml) was added 60% sodium hydride (134mg) at 0°C and the mixture was stirred at room temperature for 30 minutes. Thereto was added ethyl iodide (592µl) at 0°C and the mixture was stirred at room temperature overnight. Thereto was added water and the mixture was extracted with ethyl acetate. The organic layer was dried. After removal of the solvent, the residue was purified with silica gel column chromatography (chloroform/methanol=100/0→91/9) to give compound (2) (403mg) as a colorless oil.
   APCI-MS (m/e): 293/295 (M+H)+ 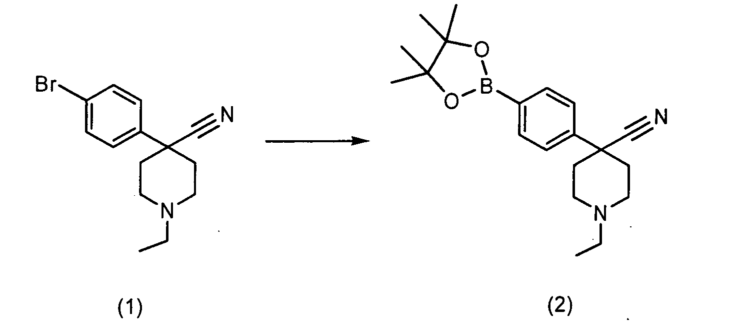
(3) By reacting and treating compound (1) in the same manner as reference example 22 (2) there was obtained compound (2).
   APCI-MS (m/e): 341 (M+H)+

### INDUSTRIAL APPLICABILITY

The compound of the present invention and a pharmaceutically acceptable salt thereof has an excellent activity for controlling the function of CCR4, or TARC and/or MDC, and is useful as the prophylactic or therapeutic agent for allergic diseases, inflammatory diseases and autoimmune diseases such as bronchial asthma or atopic dermatitis.

## Claims

1. An aromatic compound represented by the following formula (1): 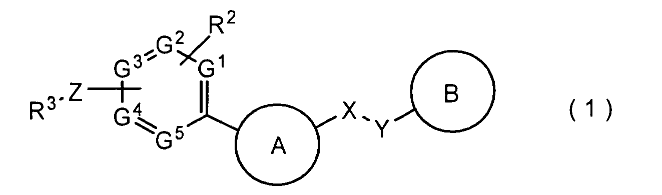 wherein ring A is a group selected from the group consisting of the following formulas; 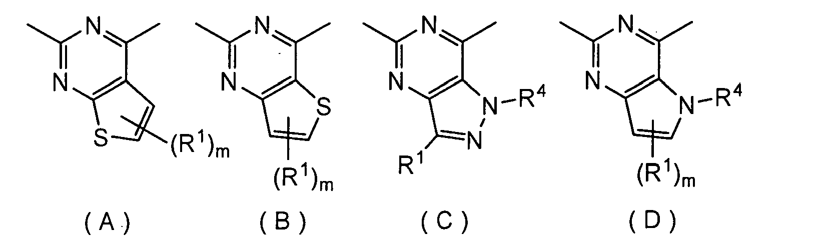 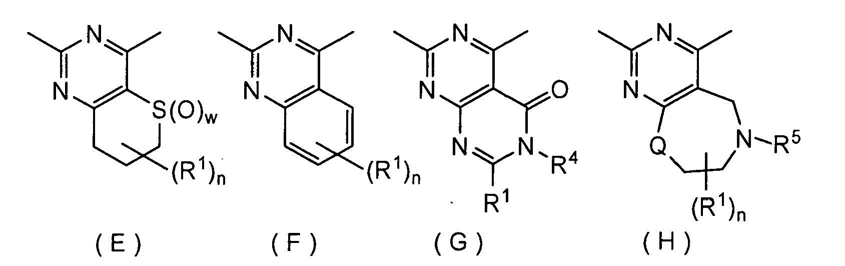 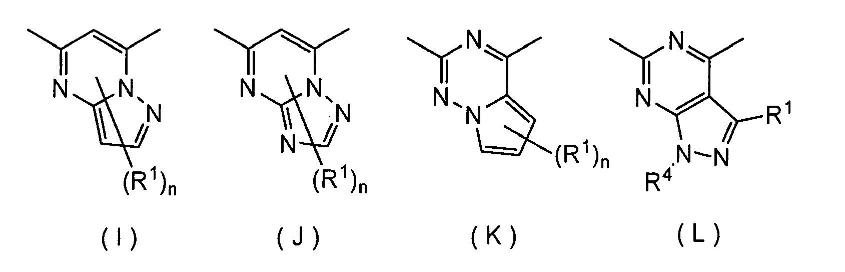 , ring B is an optionally substituted aromatic carbocyclic ring or an optionally substituted heterocyclic ring,
G¹, G², G³, G⁴ and G⁵ are each the same or different, and CH or N, provided that two or more among G¹, G², G³, G⁴ and G⁵ are CH,
Q is oxygen atom, sulfur atom or -N(R⁶),
m is an integer of 1 or 2, n is an integer of 1 to 3,
w is an integer of 0, 1 or 2,
X is -N(R⁷)-, -O- or -C(R⁸)(R⁹)-,
Y is -C(R¹⁰)(R¹¹)-, -CO- or -SO₂-,
Z is a single bond, -CO-, -SO₂-, -N(R¹²)-, -CON(R¹³)-, -SO₂N(R¹³)-, -N(R¹³)CO-, - N(R¹³)SO₂-, -N(R¹⁴)CON(R¹⁵)- or -N(R¹⁴)SO₂N(R¹⁵)-,
R¹ is hydrogen atom, alkyl group, alkoxy group, halogen atom, carboxy group, alkoxycarbonyl group, optionally substituted carbamoyl group, optionally substituted amino group, nitro group or optionally substituted ureido group,
R² is hydrogen atom, alkyl group, alkoxy group, halogen atom, haloalkyl group, carboxy group, alkoxycarbonyl group, optionally substituted carbamoyl group or optionally substituted amino group,
R³ is optionally substituted carbocyclic group, optionally substituted heterocyclic group or optionally substituted alkyl group,
R⁴ is hydrogen atom or alkyl group,
R⁵ is hydrogen atom, alkyl group or optionally substituted alkanoyl group,
R⁶ is hydrogen atom, alkyl group or optionally substituted alkanoyl group,
R⁷ is hydrogen atom or alkyl group,
R⁸ and R⁹, or R¹⁰ and R¹¹ are each the same or different, and hydrogen atom or alkyl group,
R¹² is hydrogen atom, alkyl group, alkanoyl group or carboxyalkyl group,
R¹³ is hydrogen atom or alkyl group, and
R¹⁴ and R¹⁵ are each the same or different, and hydrogen atom or alkyl group,
or a pharmaceutically acceptable salt thereof.

2. The aromatic compound or a pharmaceutically acceptable salt thereof according to claim 1, wherein ring A is a group selected from the groups consisting of the following formulas: 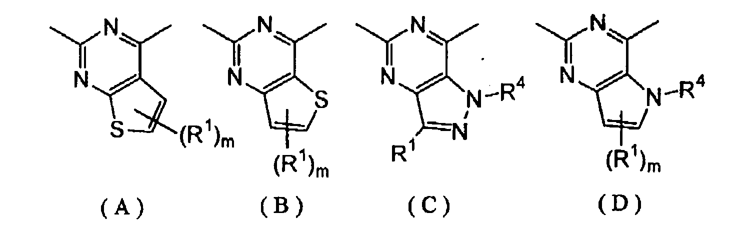 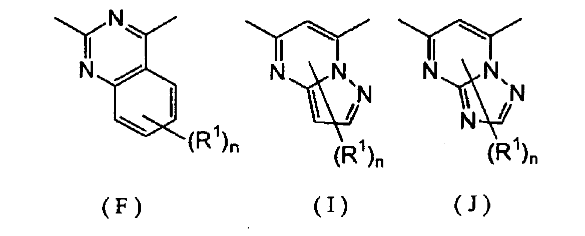 , wherein each signal is the same as defined above.

3. The aromatic compound or a pharmaceutically acceptable salt thereof according to claim 1 or 2, wherein ring A is a group selected from the group consisting of the following formulas: 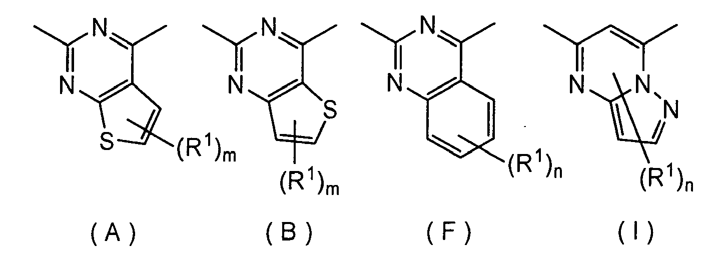 , wherein each signal is the same as defined above.

4. The aromatic compound or a pharmaceutically acceptable salt thereof according to any one of claims 1 to 3, wherein Z is a single bond, -CONH-, - NHCO- or -CO-.

5. The aromatic compound or a pharmaceutically acceptable salt thereof according to any one of claims 1 to 4, wherein R³ is
(1) pyrrolidinyl group which is optionally substituted by (a) oxo group, (b) hydroxymethyl group, (c) alkyl group, (d) amino group which is optionally substituted by one or two alkyl group(s), or (e) carbamoyl group which is optionally substituted by one or two alkyl group(s),
(2) piperidinyl group which is optionally substituted by alkyl group, alkanoyl group, cyano group, amino group which is optionally substituted by one or two alkyl group(s) or oxo group,
(3) piperadinyl group which is optionally substituted by alkyl group,
(4) morpholinyl group which is optionally substituted by alkyl group, or
(5) tetrahydropyridyl group which is optionally substituted by alkyl group.

6. The aromatic compound or a pharmaceutically acceptable salt thereof according to any one of claims 1 to 5, wherein X is -NH-, Y is -CH₂-, -CH(CH₃)-or -C(CH₃)₂-, and ring B is benzene which is substituted by one or two substituents selected from the group consisting of halogen atom, alkyl group and haloalkyl group.
